# EUROPEAN PATENT SPECIFICATION

(11) **EP 2 158 211 B1**
(45) Date of publication and mention of the grant of the patent: **10.08.2016**
(21) Application number: 08758934.7
(22) Date of filing: 02.06.2008
(51) Int. Cl.: C07K 14/015, C12N 15/62, C12N 15/86, C12N 5/10, A61K 38/16, A61K 48/00, C12N 15/864

(54) **MUTATED STRUCTURAL PROTEIN OF A PARVOVIRUS**
MUTIERTES STRUKTURPROTEIN EINES PARVOVIRUS
PROTÉINE STRUCTURALE MUTÉE D'UN PARVOVIRUS

(30) Priority: 31.05.2007 US 932410 P
(43) Date of publication of application: 03.03.2010
(73) Proprietor: Medigene AG, 82152 Planegg/Martinsried (DE)
(72) Inventor: LUX, Kerstin, 81379 München (DE); BÜNING, Hildegard, 50933 Köln (DE); PERABO, Luca, 50924 KöIn (DE); NIELAND, John, DK-8000 Aarhus (DK); BOUCAS, Jorge, 50733 Köln (DE); HALLEK, Michael, 50935 Köln (DE); RITTER, Mirko, 82152 Planegg (DE); HÖRER, Markus, 82152 Planegg (DE)
(74) Representative: Bösl, Raphael Konrad
(86) International application number: PCT/EP2008/004365
(87) International publication number: WO 2008/145400

(56) References cited:
- WO-A-03/054197
- WO-A-2005/017101
- WU P ET AL: "MUTATIONAL ANALYSIS OF THE ADENO-ASSOCIATED VIRUS TYPE 2 (AAV2) CAPSID GENE AND CONSTRUCTION OF AAV2 VECTORS WITH ALTERED TROPISM" JOURNAL OF VIROLOGY, THE AMERICAN SOCIETY FOR MICROBIOLOGY, US, vol. 74, no. 18, 1 September 2000 (2000-09-01), pages 8635-8647, XP001028219 ISSN: 0022-538X cited in the application
- GRIFMAN M ET AL: "Incorporation of tumor-targeting peptides into recombinant adeno-associated virus capsids" MOLECULAR THERAPY, ACADEMIC PRESS, SAN DIEGO, CA, US, vol. 3, no. 6, 1 June 2001 (2001-06-01), pages 964-975, XP002235294 ISSN: 1525-0016 cited in the application
- RABINOWITZ J E ET AL: "Insertional Mutagenesis of AAV2 Capsid and the Production of Recombinant Virus" VIROLOGY, ACADEMIC PRESS,ORLANDO, US, vol. 265, no. 2, 20 December 1999 (1999-12-20), pages 274-285, XP004439668 ISSN: 0042-6822
- SHI WENFANG ET AL: "RGD inclusion in VP3 provides adeno-associated virus type 2 (AAV2)-based vectors with a heparan sulfate-independent cell entry mechanism." MOLECULAR THERAPY : THE JOURNAL OF THE AMERICAN SOCIETY OF GENE THERAPY APR 2003, vol. 7, no. 4, April 2003 (2003-04), pages 515-525, XP002503474 ISSN: 1525-0016
- PERABO L ET AL: "In Vitro Selection of Viral Vectors with Modified Tropism: The Adeno-associated Virus Display" MOLECULAR THERAPY, ACADEMIC PRESS, SAN DIEGO, CA, US, vol. 8, no. 1, 1 July 2003 (2003-07-01), pages 151-157, XP003000206 ISSN: 1525-0016 cited in the application
- MUELLER O J ET AL: "Random peptide libraries displayed on adenoassociated virus to select for targeted gene therapy vectors" NATURE BIOTECHNOLOGY, NATURE PUBLISHING GROUP, NEW YORK, NY, US, vol. 21, no. 9, 1 September 2003 (2003-09-01), pages 1040-1046, XP002297275 ISSN: 1087-0156 cited in the application

## Description

The present invention is related to a multimeric structure comprising a structural protein of a parvovirus with an amino acid insertion at the insertion site I-453, a library comprising the protein, a nucleic acid encoding the protein, a vector, virus or cell comprising the nucleic acid, a medicament comprising the protein, nucleic acid or multimeric structure as well as methods and uses involving the protein, nucleic acid or multimeric structure.

Monoclonal antibody therapies have been one of the most successful therapy forms of new drug developments over the last couple of years in therapeutic fields such as oncology, autoimmune and inflammatory diseases. In monoclonal antibody therapies patients are injected with a specific monoclonal antibody that recognizes the antigen involved in the disease. Antibodies recognize their antigen with the variable domain of the antibody which is also referred to as the idiotype of the antibody.

However, monoclonal antibody therapies also have certain drawbacks. It can be observed that, if the concentration of a specific antibody with one particular idiotype is too high, the patient's immune system develops an antibody response against the idiotype of the therapeutic monoclonal antibody and thereby limits its efficacy. This kind of antibody that recognizes an antibody's idiotype is referred to as an anti-idiotypic antibody. In addition, antibodies to monoclonal therapeutic antibodies directed against other parts of the monoclonals often limit efficacy of a passive antibody therapy. Therefore, many of the monoclonal antibody drugs need to be used in combination with the traditional immunosuppression regiments, increasing the overall treatment costs. Furthermore, active suppression of the patient's immune system is detrimental especially; if an intact immune system is required to control the stage of disease such as for oncological indications.

As being a passive vaccination against the target antigen the monoclonal antibody has to be injected frequently depending on the half life of the antibody within the serum of the patient. Therefore, such treatments are expensive and inconvenient for the patients.

An alternative for such monoclonal antibody therapies already exists exemplified by a number of clinical developments using anti-idiotypic antibodies as drugs. Such anti-idiotypic antibody therapies are based on the fact (see above) that the patient's immune system can induce an antibody response against the idiotype of an antibody. If one uses a monoclonal antibody expressing a functional imitation of a target epitope (paratope or mimotope) as an idiotype, the patient's immune system will generate a polyclonal antibody response wherein a subset of these antibodies is able to cross-react with the target epitope in the patient. Such antibody expressing a paratope is referred to as an anti-idiotypic antibody (based on Jerne's network model of idiotypic relationships (Jerne, 1974, Jerne et al., 1982). Thus, selective immunization with an anti-idiotypic antibody can induce a specific immune response directed against the original antigen (Varela and Coutinho, 1991, Jefferis, 1993, Chatterjee et al., 1994).

Therefore, a vaccination with such an anti-idiotypic antibody actively induces a polyclonal antibody response. As a consequence such anti-idiotypic antibody vaccines have a number of advantages over a passive immunization by a standard monoclonal antibody. There is no antibody response towards the anti-idiotypic antibody that limits its efficacy as exactly this immune response is used as the therapeutic principle. Therefore, it is also not necessary to combine the antibody treatment with an immunosuppression regimen. And further, due to the fact that the anti-idiotypic treatment is an active immunization, the drug only has to be injected from time to time to boost the antibody response generated by the patient himself maintaining a continuous titer of specific antibodies. Additionally, anti-idiotypic antibodies induce a polyclonal antibody response against the target antigen that hampers the potential mechanism for resistance to the treatment of e.g. in tumor cells.

However, anti-idiotypic antibody therapies face major disadvantages. The titers of the induced polyclonal antibody response obtained by the vaccination with anti-idiotypic antibodies are often not high enough to establish a beneficial treatment. This is due to the lack of a strong antigen as a vaccine, since antibodies per definition are not very immunogenic. Furthermore, it is difficult to generate specific anti-idiotypic vaccines because of this lack of immunogenicity and technical difficulties to identify anti-idiotypic antibodies.

A series of publications describes that an antigen placed in the context of an ordered surface of a viral particle - here a papilloma virus particle - can induce a B cell response that even can abrogate B cell tolerance to such antigen by direct crosslinking the respective B-cell receptor. Bovine papilloma virus-like particles (VLPs) conjugated to an Aβ peptide through biotin were used to generate an immune response against the self antigen Aβ (Li et al., 2004). Further, this group used bovine papilloma virus-like particles having the murine chemokine receptor mCCR5 inserted into an immunodominant site of the viral L1 protein to immunize mice leading to sera with high anti-CCR5 antibody titers despite the fact that CCR5 is a self-antigen. Further, a macaque L1-CCR5 fusion protein was used to immunize pig tail macaques. 4 of the 5 test animals produced CCR5 specific antibodies. In a further approach TNF-α was joined to VLPs by way of a biotin-streptavidin interaction (Chackerian et al., 2001). These VLPs were successful in generating an auto-antibody response in mice, whereas these antibodies bound native TNF-α. (US 6,719,978).

Therefore, papilloma VLPs have been shown to be a suitable backbone for the presentation of antigens to the immune system in order to generate strong B cell responses, probably because of their dense, ordered and closely packed array of vaccination epitopes. Due do their exceptionally strong B cell induction papilloma VLPs can be especially useful to overcome B cell tolerance to self antigens.

However, linkage of epitopes via biotin is a complicated process step that is difficult to perform under exactly controlled conditions as required by regulatory authorities for an approved drug. Further, the use of a bovine papilloma virus-backbone in humans may generate a dominant immune response against the viral backbone that the generated B-cell response against the inserted epitope is to weak to generate a sufficient priming of B-cells, which is especially important if tolerance has to be broken. Additionally, papilloma viruses are very difficult to manufacture in tissue culture and usually have to get isolated from warts. Therefore, for applications were viruses are necessary that encode a viral genome, papilloma viruses are unsuitable. One such application is the generation of viral libraries of capsid variants that can be used to screen a capsid mutant with certain properties like displaying an epitope matching to a monoclonal antibody of choice or an insert capable of binding to a cellular receptor of choice.

For Adeno-associated virus of type 2 (AAV-2) it was described in the past that the insertion of ligand peptides into structural proteins results in capsids that are able to display the ligand on the surface of the capsid and mediate transduction through the interaction of the ligand with its receptor thereby redirecting viral tropism by genetic capsid modifications (Girod et al., 1999, Grifman et al., 2001, Nicklin et al., 2001, Shi et al., 2001, Wu et al., 2000), which is referred to as hereinafter retargeting. In particular, it has been demonstrated that the insertion of an integrin binding Arg-Gly-Asp (RGD) motif at the insertion site I-587 of the AAV capsid proteins VP-1 enabled AAV particles to transduce cells via α_{V}β₁ integrins (Aumailley et al., 1990, Girod et al., 1999). Successful targeting of gene vectors such as AAV-2 is important to increase the efficiency and safety of gene therapy, since it would allow to restrict the gene transfer into the desired tissue, minimize the risk associated with the transfer of potentially dangerous genes into other cell types and increase the concentration of the therapeutic gene product delivered to the ill tissue (Kay et al., 2001, Pfeifer and Verma, 2001). Although other potential insertion sites have been described for AAV-2, I-587 has by far been used most successfully and can be regarded as the best site for capsid modifications.

Further, AAV-2 libraries displaying random peptide inserts at the position I-587 have been reported that were successfully screened for targeting mutants (Perabo et al., 2003, Perabo, 2003, Waterkamp et al., 2006).

A further advantage of parvoviruses in this context is that due to the high structural conservation of parvoviruses knowledge obtained for e.g. AAV-2 can easily be transferred to other parvoviruses. Whenever repeated administration of the product is necessary the switch between different parvovirus backbones displaying the same peptide/epitope can circumvent (neutralizing) antibodies that have been raised in an earlier application.

However, an insertion within I-587 - depending on its use - may have certain disadvantages. The insertion of ligand peptides into this site has been reported to ablate binding of heparin sulphate proteoglycan (HSPG), which is AAV-2's primary receptor, in some but not in all mutants (Perabo et al., 2006b). This phenomenon is likely due to the fact that an insertion interferes with at least two of the five positively charged amino acids of the recently identified HSPG binding motif (Kern et al., 2003, Opie et al., 2003), namely R₅₈₅ and R₅₈₈. Inserted peptides containing a net negative charge are prone to confer an HSPG nonbinding phenotype, while positive charges facilitate the interaction with HSPG (Perabo et al., 2006b). Conclusively, the interference of the HSPG binding site with the insertion site I-587 limits its universal applicability.

Generally speaking one can expect a certain interference of an inserted peptide/ligand with its surrounding amino acids of the capsid backbone. Such interference determines which kind of insert is acceptable for the viral capsid at a specific site. If another site is used for insertions, one can expect that the backbone context is different and different peptides can successfully be integrated. Therefore, the "ideal" peptide (e.g. for targeting the virus) may interfere at one site but may perfectly fit at another site.

In case of vaccination purposes HSPG binding might be necessary or at least useful for the capsids to enter predendritic cells, whose activation is needed to exert a T_{H1}-response. Consequently, the insertion of a B-cell epitope into I-587 may ablate HSPG binding and therefore would not trigger a T_{H1}-response. Additionally, viruses with an intact HSPG binding motif can still be efficiently purified using common heparin affinity chromatography.

Further, the combination of I-587 with a further insertion site would be ideal to increase the density of B-cell epitopes on the surface of the capsid or to display two different epitopes expressed from a single cap gene - in contrast to mosaic capsids generated by co-expressing more than one different cap gene, which is disadvantageous from a regulatory stand point.

Taken together these facts and considerations suggest that AAV and other parvoviruses are suitable backbones for vaccination purposes and/or retargeting approaches in the gene therapy context, but an additional insertion site with equal or improved properties is needed. Therefore, the underlying problem of the present invention is the identification of a further insertion site being an alternative or even superior to I-587, in which peptides can be inserted alone or in combination with insertions into e.g. I-587, which peptides are displayed on the surface of a capsid and which peptides are at least bound by a respective antibody and for the use of retargeting viral vectors can mediate transduction of target cells.

It has now been surprisingly found that the position after amino acid G₄₅₃ of AAV-2 is especially suitable for such insertions.

Accordingly, the major object of the present invention is a multimeric structure comprising a parvovirus structural protein which comprises an amino acid insertion, wherein the amino acid insertion is directly C-terminal of amino acid G₄₅₃ in the sequence of AAV-2 or the corresponding amino acid of any other parvovirus, and wherein the structural protein with the insertion is capable of particle formation, and wherein the insertion (a) has a length of 4 to 30 amino acids, and/or (b) is an epitope.

Insertions near, but not exactly at this site have previously been suggested but were only of no or limited success. The insertion of the 14-amino-acid peptide L14 after amino acid R₄₄₇ (I-447) (Girod et al., 1999) led to intact capsids as the conformation-sensitive antibody A20 still reacted with it. Further, an L14-specific antibody specifically recognized the insert in an ELISA. The mutant capsid was further able to specifically bind to cells expressing the L14-specific integrin receptor. However, successful transduction did not occur for the insertion in I-447 in such cells - in contrast to a mutant capsid with the same insertion at I-587. These data show that in principle it is possible to insert peptides at I-447 as capsids are still formed and the inserted peptide is displayed on the surface of the protein, but at least for the L14 peptide such insertion does not lead to a successful transduction suggesting that I-447 is not an ideal candidate for insertions in general.

Also Wu et al. (Wu et al., 2000) report the insertion of a hemagglutinin (HA) peptide at the position I-447. Indeed, the mutated structural protein shows intermediate capsid formation and transduction (table 5, page 8643) but clearly this insertion site is inferior to I-587.

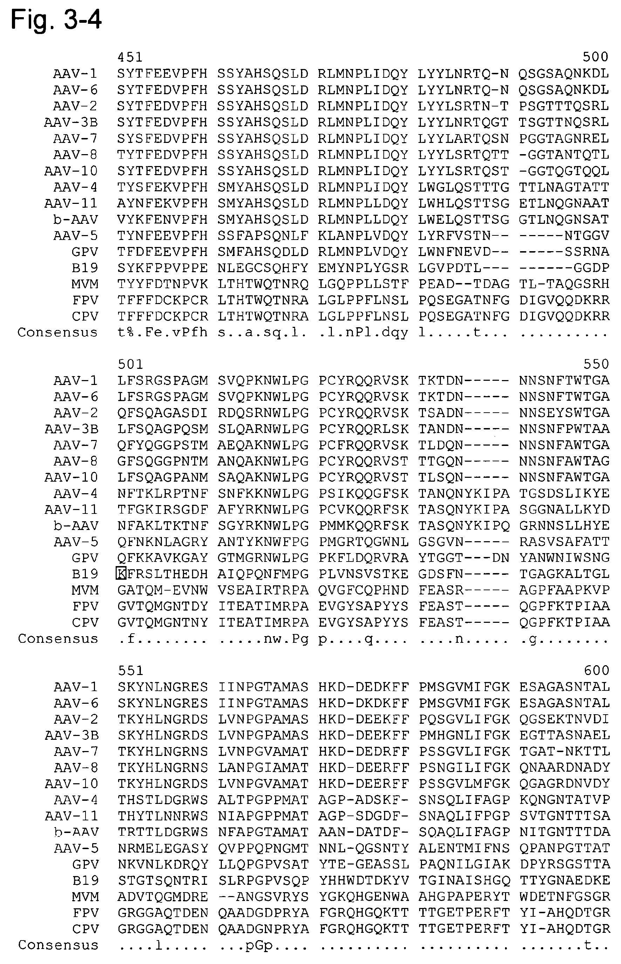

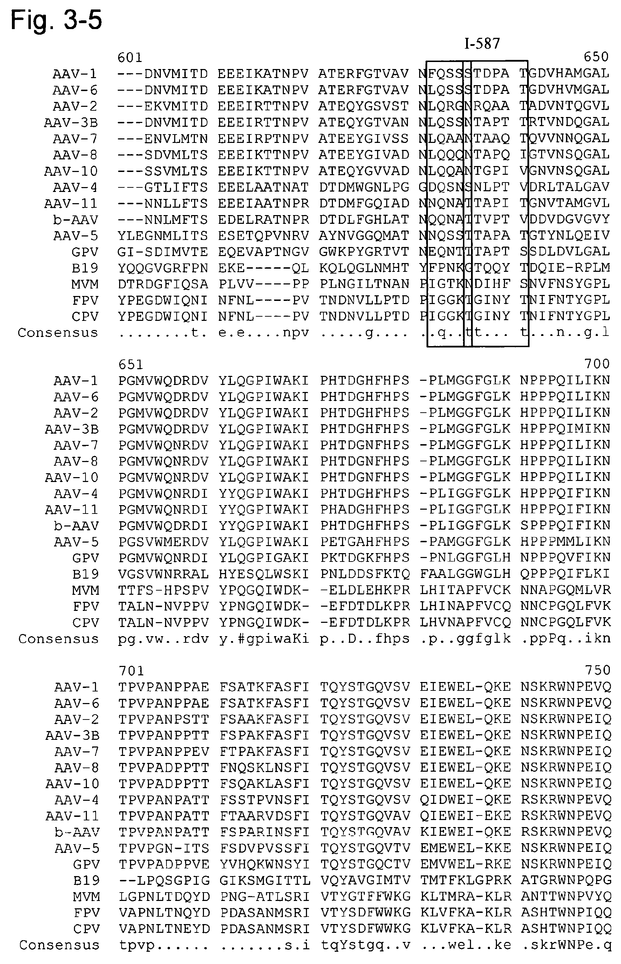

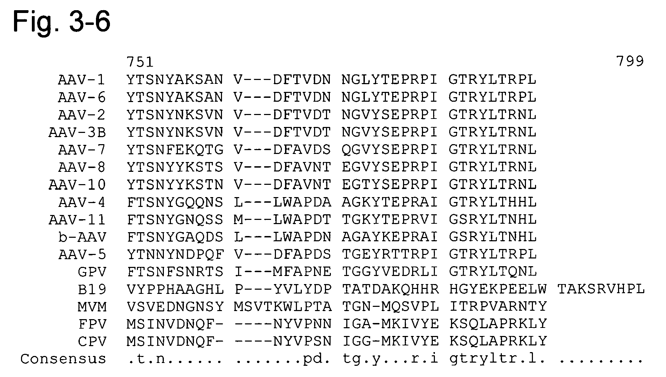

Further, Grifman et al. inserted a Myc epitope between T₄₄₈ and N₄₄₉ (referred to as by the authors 449Myc, see Fig. 3B; and herein I-448) (Grifman et al., 2001). The Myc epitope was accessible to an anti-Myc antibody and was therefore present on the surface of the capsid. Whereas successful retargeting was again reported for the insertion after N₅₈₇ (here the insertion of an NGR motif that mediates binding to CD13), no data on the retargeting using the I-449 site was presented indicating that retargeting was again not successful despite the fact that this site can be used to display inserts on the surface of a capsid.

In another study the insertion of an RGD4C peptide inserted after amino acid R₄₅₉ severely diminished transducing titers, whereas the insertion of the same peptide at positions A₁₃₉, Q₅₈₄ and R₅₈₈ were well tolerated (Shi and Bartlett, 2003).

Due to the near β-barrel structures that might get affected by insertions at I-453, one would not have expected that I-453 actually is a superior insertion site or at least an alternative insertion site compared to I-587.

Surprisingly, in the context of the present invention insertions directly C-terminally of G₄₅₃ of AAV-2 were found to be superior or at least a valid alternative to I-587.

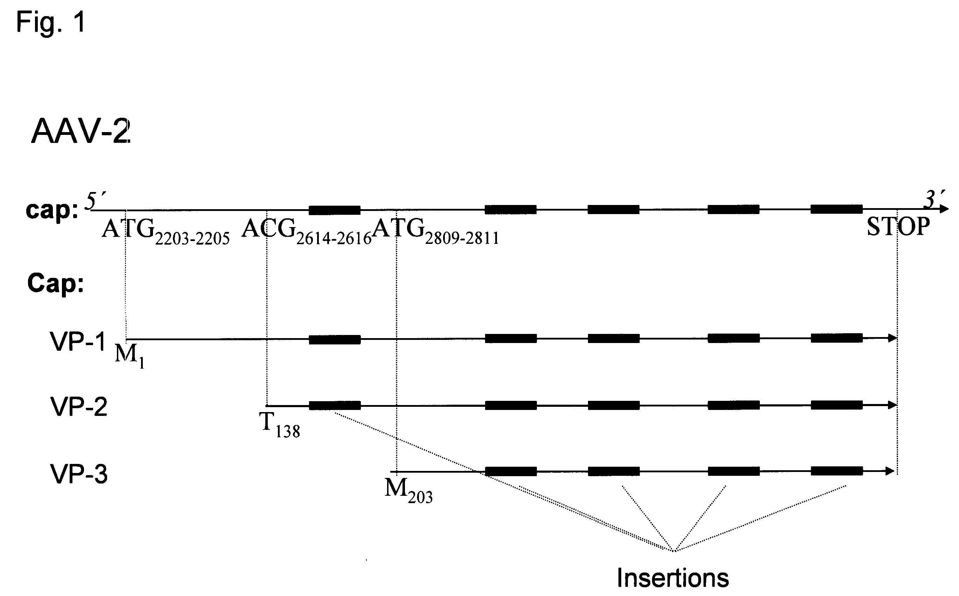

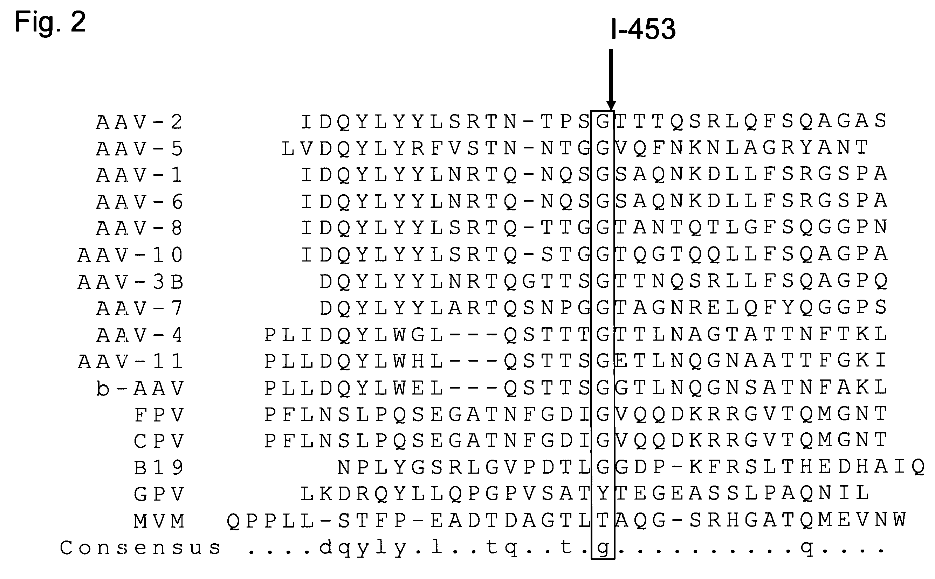

As further detailed below peptide sequences that have been successfully inserted after G₄₅₃ and that were displayed on the capsid surface, were recognized by peptide-specific antibodies and - in case of a targeting mutant - mediated viral transduction. When aligning amino acid sequences of various parvoviruses it has surprisingly been found that G₄₅₃ of AAV-2 is conserved among all adeno-associated viruses included in the alignment and some more distantly related parvoviruses such as FPV, CPV and B19 (see Figure 2). Previously published alignments of this region do not reveal this conserved amino acid (Grifman et al., 2001) Fig. 1, A and B, Loop III, (Girod et al., 1999) Fig. 1c). Accordingly, the invention is not limited to AAV-2 but is also applicable to other parvoviruses as defined below. Additional parvovirus sequences can easily be aligned to the provided alignment (Figure 2).

According to the present invention, the amino acid insertion is directly C-terminal of amino acid G₄₅₃ in the sequence of AAV-2 or the corresponding amino acid of any other parvovirus. Also disclosed herein are insertions that can be made at the herein defined insertion site I-453, being an insertion located directly N- or C-terminally, preferably C-terminally of one amino acid in the sequence of 5 amino acids N- or C-terminal of the corresponding amino acid to AAV-2's G₄₅₃, preferably 3, more preferably 1, especially directly N- or C-terminal, in particular C-terminal, of the corresponding amino acid to AAV-2's G₄₅₃. This means that the insertion site I-453 referred to in the description corresponds to the amino acids listed in Table 1. The arrow (▼ ) indicates the position of the insertion sites according to the claims.

**Table 1: I-453**

| **Parvovirus** | **Amino acid no.** | **Amino acid seq.** | **SEQ ID NO:** |
|---|---|---|---|
| AAV-2 | G₄₅₃ | NTPSG▼TTTQS | SEQ ID NO: 1 |
| AAV-5 | G₄₄₆ | NNTGG▼VQFNK | SEQ ID NO: 2 |
| AAV-1 | G₄₅₄ | QNQSG▼SAQNK | SEQ ID NO: 3 |
| AAV-6 | G₄₅₄ | QNQSG▼SAQNK | SEQ ID NO: 4 |
| AAV-8 | G₄₅₆ | QTTGG▼TANTQ | SEQ ID NO: 5 |
| AAV-10 | G₄₅₆ | QSTGG▼TQGTQ | SEQ ID NO: 6 |
| AAV-3b | G₄₅₄ | GTTSG▼TTNQS | SEQ ID NO: 7 |
| AAV-7 | G₄₅₆ | SNPGG▼TAGNR | SEQ ID NO: 8 |
| AAV-4 | G₄₄₅ | STTTG▼TTLNA | SEQ ID NO: 9 |
| AAV-11 | G₄₄₄ | STTSG▼ETLNQ | SEQ ID NO: 10 |
| b-AAV | G₄₄₇ | STTSG▼GTLNQ | SEO ID NO: 11 |
| FPV | G₃₀₇ | FGDIG▼VQQDK | SEQ ID NO: 12 |
| CPV | G₂₇₁ | FGDIG▼VQQDK | SEQ ID NO: 13 |
| B19 | G₂₆₈ | PDTLG▼GDPKF | SEQ ID NO: 14 |
| GPV | Y₃₂₃ | VSATY▼TEGEA | SEQ ID NO: 15 |
| MVM | T₃₀₉ | AGTLT▼AQGSR | SEQ ID NO: 16 |

| | | | |
|---|---|---|---|
| ▼ indicates the insertion site within I-453 according to the present invention for each structural protein listed. | | | |

As the previously known insertion site I-587, I-453 lies within the C-terminal region of the CAP proteins that is present in VP-1, VP-2 and VP-3. Consequently, an insertion of a coding DNA sequence in frame into the cap gene at the corresponding site of I-453 leads to a respective amino acid insertion into VP-1, VP-2 and VP-3 (see Figure 1).

The following definitions explain how the defined terms are to be interpreted in the context of the products, methods and uses of the present invention:
A "structural protein" means a protein that is part of the capsid of the virus. For parvoviruses the structural proteins are generally referred to as VP-1, VP-2 and/or VP-3, encoded by the cap gene. The amino acid sequences of structural proteins of parvoviruses are well known in the art. They are conserved within the parvoviruses. Amino acid positions provided herein that are not further specified refer to the AAV-2 sequence of the major coat protein VP-1 as published by Ruffing et al. (Ruffing et al., 1994); Genpept Accession No. 2906023).

A "mutated structural protein" means a structural protein that has at least one mutation in comparison to the respective structural protein of the wild-type virus.

A "parvovirus" means a member of the family of Parvoviridae containing several genera divided between two subfamilies Parvovirinae (Parvovirus, Erythrovirus, Dependovirus, Amdovirus and Bocavirus) and Densovirinae (Densovirus, Iteravirus, Brevidensovirus, Pefudensovirus and Contravirus) (Fields: Virology, fourth edition 2001, Volume 2, chapters 69 (especially Table 1) and 70, Lippincott Williams Wilkins, Philadelphia; http://virus.stanford.edu/parvo/parvovirus.html, http://www.ncbi.nlm.nih.gov/ICTVdb/Ictv/fs_parvo.htm#SubFamily1). Preferred parvoviruses are members of the genus Parvovirus such as AAV-1, AAV-2, AAV-3b, AAV-4, AAV-5, AAV-6, AAV-7, AAV-8, AAV-9, AAV-10, AAV-11, AAV-12, bovine AAV (b-AAV), canine AAV (CAAV), canine parvovirus (CPV), mouse parvovirus, minute virus of mice (MVM), B19, H1, avian AAV (AAAV), feline panleukopenia virus (FPV) and goose parvovirus (GPV). More preferred parvoviruses are those that have a conserved G aligning to G₄₅₃ of AAV-2, which are AAV-1, AAV-2, AAV-3b, AAV-4, AAV-5, AAV-6, AAV-7, AAV-8, AAV-10, AAV-11, FPV, CPV and B19 (see Figure 2). Most preferred are AAV-2, AAV-1 and AAV-6.

An "epitope" is the part of a macromolecule that is recognized by the immune system, specifically by antibodies, B cells, or cytotoxic T cells. Although epitopes are usually thought to be derived from nonself proteins, sequences derived from the host that can be recognized are also classified as epitopes. Epitopes have a length of at least 4 amino acids, preferably 4 to 30 amino acids, more preferably 5 to 20 amino acids, especially 5 to 15 amino acids. Epitopes can be linear or three-dimensional formed typically by amino acids that are distant from each other in the primary protein structure but become closely related in a secondary and/or tertiary structure. Epitopes that are specifically recognized by B cells are referred to as B-cell epitopes.

A "tolerogen" is a self-antigen that is - in its natural environment - accessible to the humoral immune system. It may be either secreted or otherwise released from a living cell or associated to the outer surface of or integrated into the cellular membrane. Generally speaking tolerogens do - under normal circumstances in contrast to e.g. autoimmune diseases - not evoke a specific immune response due to tolerance against the antigen which results from a previous exposure to the same antigen. Tolerance can occur due to central tolerance or peripheral tolerance. Central tolerance refers to tolerogens which corresponding antigens have been exposed to T cells in the thymus leading to elimination of the specific T cells. Peripheral tolerance occurs when antigens / epitopes / mimotopes / paratopes are presented to T cells without appropriate additional stimuli, commonly provided by inflammation leading to anergy. Still, it has been observed that tolerogens can induce to some extent regulatory B-cell responses (Vogel et al., 2004).

In one preferred embodiment this invention relates to tolerogens due to peripheral tolerance, preferably tolerogens derived from tumor antigens/epitopes/mimotopes/paratopes. Tolerogens encompassed by this invention include peptides, nucleic acids, carbohydrates, and lipids, preferably peptides.

Preferred tolerogens are antigens on the surface of a cell, especially tumor cells, e.g. receptors, especially growth factor receptors (preferably EGFR), tumor antigens (preferably Her2/NEU, Melan A, high molecular weight melanoma associated antigen (HMW MAA), CA125), viral receptors (CCR5), CD20, acetylcholine receptors, interleukin receptors (IL-13 receptor). Further preferred tolerogens can be blood proteins (preferably CETP), interleukins (preferably IL-6, IL-9, IL-13, IL-17), cytokines, TNF-family members (preferably TNF-α), immunoglobulins (preferably IgE), complement factors, misfolded proteins (preferably β-ayloid) and growth factors (preferably VEGF).

A "tolerogen-derived epitope" of a specific tolerogen in the context of the products, methods and uses of the present invention refers to a B-cell epitope that
i) is identical to a B-cell epitope of the tolerogen,
ii) a derivative (e.g. a mutant) of a B-cell epitope of the tolerogen that crossreacts with an antibody that binds the B-cell epitope of the tolerogen,
iii) a mimotope of a B-cell epitope of the tolerogen, and/or
iv) a paratope of a B-cell epitope of the tolerogen.

The length of a tolerogen-derived epitope is typically 4-30, preferably 5-20 and most preferably 5-15 amino acids.

The derivative of a B-cell epitope of a tolerogen may be generated by one or more amino acid substitutions, preferably one or more conservative amino acid substitutions, i.e. substitutions that take place within a family of amino acids that are related in their side chains and chemical properties. Examples of such families are amino acids with basic side chains, with acidic side chains, with non-polar aliphatic side chains, with non-polar aromatic side chains, with uncharged polar side chains, with small side chains, with large side chains etc. Further, derivatives may be obtained by one or more single amino acid deletion(s) and/or insertion(s).

"Crossreaction" or "crossreact" of B-cell epitopes with a specific monoclonal antibody means according to this invention that the its affinity (K_{D}; see below) of the epitopes with the antibody are within two magnitudes, preferably within one magnitude when comparing the B-cell epitope to its derivative.

Tolerogen-derived epitopes within the multimeric structure comprising parvovirus mutated structural proteins according to this invention are identical, resemble or mimic antigen stretches of a tolerogen that are - in their natural environment - accessible to the immune system, e.g. epitopes of membrane protein located in the extracellular part, serum proteins, immunoglobulins, plaque proteins. Such antigen stretches are preferably located on the surface of such protein within the body of a mammal, preferably a human.

A "mimotope" is a non-linear structural epitope composed of several amino acids derived from different regions of the linear sequence of the structural protein located in close neighborhood due to the overall tertiary structure of the capsid that is specifically bound by an antibody, or a linear epitope mimicking a discontiuous epitope of the structural protein.

A "paratope" is the antigen binding site that is specifically bound by an antibody.

The mimotope or paratope in the context of the present invention might consist of (parts of) the inserted peptide sequence alone or might be composed of inserted peptide and parvovirus core particle amino acid residues.

An "insertion" of (an) amino acid(s) is generally speaking an insertion of at least one heterologous amino acid into the sequence of - for this invention - a parvovirus structural protein. 'Heterologous' in this context means heterologous as compared to the virus, from which the parvovirus structural protein is derived from. The inserted amino acids can simply be inserted between two given amino acids of the parvovirus structural protein. An insertion of amino acids can also go along with a deletion of given amino acids of the parvovirus sturctural protein at the site of insertion, leading to a complete substitution (e.g. 10 given amino acids are substituted by 10 or more inserted amino acids) or partial substitution (e.g. 10 given amino acids are substituted by 8 inserted amino acids) of amino acids of the parvovirus structural protein.

The term "binder" refers to a molecule that specifically binds to its respective binding partner. Commonly used binders are antibodies, especially monoclonal antibodies, antibody derivatives such as single chain antibodies or antibody fragments. In principle all classes of antibodies can be used, preferred are IgG antibodies. Fragments or multimers of antibodies can equally be used. Commonly used fragments are single chain antibodies, Fab- or (Fab)₂-fragments. Examples of other suitable binders are protein scaffolds such as anticalins or lipocalins (Nygren and Skerra, 2004), receptors or parts thereof (e.g. soluble T-cell receptors), ankyrine, microbodies or aptamers.

The term "specifically binds" means that two molecules A and B, preferably proteins, bind to each other thereby generating complex AB with an affinity (K_{D} = k_{off}/ kₒₙ) of at least K_{D} = 1x10⁻⁵ mol/l, preferably 1x10⁻⁷ mol/l, more preferably 1x10⁻⁸ mol/l, especially 1x10⁻⁹ mol/l.

"Heterologous" in the context of the present invention means a peptide sequence, e.g. an epitope that is not present on the parvovirus wild-type viral capsid and/or structural protein.

The term "antigen" in the context of the products, methods and uses of the present invention refers to any target antigen against which an immune reaction should be induced. Such target antigens are usually antigens that are susceptible to the humoral immune response. They are usually proteins that may be posttranslationally modified, as for example glycosylated proteins. Preferred antigens are serum proteins, proteins that can be found at least under certain conditions (e.g. in a disease state) in the blood (e.g. CETP, IL-6, IL-17, TNF-α), and membrane proteins, especially receptor proteins (e.g. CD20, acetylcholine receptors, IL13R, EGFR). Especially preferred antigens are IgE, tumor-antigens (e.g. Melan A, high molecular weight melanoma associated antigen (HMW MAA), CA125, IL13R, Her2/NEU, L1 cell adhesion molecule), VEGF, EGFR, CD20, CETP (cholesterol ester transfer protein), TNF-family members (e.g. TNF-α), interleukins (IL-9, IL-6, IL-13, IL-17), or misfolded proteins leading to a protein aggregation and, therefore, causing conformational diseases (for an overview see Uversky et al., 2006, e.g. β-amyloid). Excluded from the above definition of "antigen" are parvovirus antigens, i.e. antigens inherent the unmutated parvovirus itself, e.g. derived from B19 (Klenerman et al., 2002).

By inserting amino acids at I-453 the inserted amino acids are located on the surface of the capsid formed by the structural proteins. The display of the amino acids on the surface enables the insertion to exert their vaccination or targeting function.

Insertions at position I-453 did not interfere with particle formation of the capsid proteins. The multimeric structure of the present invention shows advantageous characteristics, in particular if they are to be used for vaccination purposes, as it is believed that ordered/virus-like particles are superior to exert a strong and insertion-specific immune response. However, as was shown for human papilloma virus-like particles also capsid subunits like capsomeres were able to be used for vaccination purposes. As a vaccination with a particle containing a genome is a gene therapy it is preferred to use inactivated (e.g. by gamma or UV-irradiation) genome-containing AAV particles, or virus-like particles of the respective parvovirus. Such virus-like particles (or in brief particles) are capsid-like structures that are composed of the structural proteins of the respective parvovirus, e.g. VP-1, VP-2 and/or VP-3, or parts thereof such as N- or C-terminal truncated structural proteins but do essentially not contain a viral genome. VP-2 alone has been shown to assemble into virus-like particles and can be expressed in various expression systems such as bacteria e.g. *E. coli*, yeasts, e.g. *Saccharomyces cerevisiae, hansenula polymorpha, Pichia pastoris,* in insect cells, e.g. the baculovirus expression system (SF9, SF+ or High Five cells), or in mammalian cells (such as CHO, HeLa, 293, BHK, or PerC6).

As far as the context of gene therapy is concerned, where viral vectors are constructed, the capability of forming particles such as capsids is an highly preferred feature, as intact particles are in general needed to package a viral genome carrying the transgene.

In a preferred embodiment the parvovirus is an adeno-associated virus, preferably selected from the group consisting of AAV-1, AAV-2, AAV-3b, AAV-4, AAV-5, AAV-6, AAV-7, AAV-8, AAV-9, AAV-10, AAV-11, AAV-12 and b-AAV, especially selected from the group consisting of AAV-1, AAV-2, AAV-5 and AAV-8. In a further preferred embodiment the parvovirus is selected from the group consisting of bovine AAV (b-AAV), canine AAV (CAAV), canine parvovirus (CPV), mouse parvovirus, minute virus of mice (MVM), B19, H1, avian AAV (AAAV), feline panleukopenia virus (FPV) and goose parvovirus (GPV), more preferably FPV, CPP, B19, GPV and MVM, especially FPV, CPV and B19.

The claimed multimeric structue contains a protein having an amino acid insertion that has a length of about 4 to about 30 amino acids, preferably about 5 to about 20, most preferably about 5 to about 15 amino acids. Typically, the size of a B-cell epitope is at least 5 amino acids (US 2004/0228798A1). Same is true for a number of known amino acid sequences suitable as targeting sequences. Larger inserts are likely to interfere with each other. It is also encompassed by this invention that one insert comprises more than one functional amino acid sequence (e.g. epitopes and/or targeting sequences) in a row. For example the insert can comprise 2, 3, 4, 5 or 6 identical inserts in a row or 2, 3, 4, 5 or 6 different functional amino acid sequences such as different epitopes.

The nature of the amino acid insertion is preferably an epitope, especially a B-cell epitope. Epitopes are generally preferred if the structural proteins are modified to be suitable for vaccination purposes. The epitopes can be known B-cell epitopes that have been identified as linear epitopes, paratopes or sequence that form mimotopes. Therefore, B-cell epitopes can be both linear and structural. However, it is especially preferred to use linear epitopes that are no mimotopes.

Further, the epitopes can be sequences that have been identified by phage display or by AAV display, where a library of amino acid sequences is displayed on the surface of phages or AAV and such phages/AAVs are identified that specifically bind to a specific binder, especially an antibody of choice. The inserted amino acid sequence can be sequenced and transferred into I-453. In a preferred embodiment the amino acid sequence is directly identified from an AAV-library, where the library of amino acid sequences had been inserted into I-453, for example in analogy to the AAV-2 libraries described in (Perabo et al., 2003, Lieber, 2003, Muller et al., 2003) (WO 03/054197). In this context the advantage is used that the amino acid insert is identified in the same surface context as it is used for vaccination later on. Therefore, the conformation is not changed compared to settings where an epitope is transferred from an original context (e.g. from an antigen or from a phage) to the site I-453.

In a preferred embodiment the B-cell epitope is heterologous to the parvovirus.

In an especially preferred embodiment the inserted B-cell epitope is a tolerogen-derived epitope. As described above it is especially difficult to break tolerance and mutimeric structures according to this invention are especially suitable for this purpose. Preferred tolerogen-derived epitopes are derived from IgE, CETP, CCR5, HER2/Neu, TNF-α, IL-17, IL-6 or β-amyloid, preferably human IgE and human β-amyloid, exemplified by the following preferred epitopes:

**IgE epitopes:**

| | |
|---|---|
| VNLTWSRASG ("Kricek") | (SEQ ID NO: 50) |
| EFCINHRGYWVCGD ("Rudolf") | (SEQ ID NO: 55) |
| EDGQVMDVDLS ("Flex") | (SEQ ID NO: 85) |
| EKQRNGTLT ("Bind-2") | (SEQ ID NO: 86) |
| TYQCRVTHPHLPRALMR ("3DEpi1") | (SEQ ID NO: 87) |
| RHSTTQPRKTKGSG ("3DEpi2") | (SEQ ID NO: 88) |
| DSNPRGVSAYLSR (3DEpi3) | (SEQ ID NO: 89) |
| TITCLWDLAPSK ("3DEpi4") | (SEQ ID NO: 90) |
| KTKGSGFFVF ("C4E") | (SEQ ID NO: 91) |
| THPHLPRALMRS ("Wang-CS") | (SEQ ID NO: 92) |
| GETYQCRVTHPHLPRALMRSTTK ("Wang") | (SEQ ID NO: 93) |
| LPRALMRS ("C21") | (SEQ ID NO: 94) |
| INHRGYWV ("C4M") | (SEQ ID NO. 95) |

**CETP epitopes:**

| | |
|---|---|
| CDAGSVRTNAPD | (SEQ ID NO: 60) |
| AKAVSNLTESRSESLQS ("CETP TP10") | (SEQ ID NO: 96) |
| SLTGDEFKKVLET ("CETP TP11") | (SEQ ID NO: 97) |
| REAVAYRFEED ("CETPTP12") | (SEQ ID NO: 98) |
| INPEIITLDG ("CETP TP13") | (SEQ ID NO: 99) |
| DISVTGAPVITATYL ("CETP TP18") | (SEQ ID NO: 100) |
| DISVTGAPVITA ("CETP TP20A") | (SEQ ID NO: 101) |
| PKTVSNLTESSSESVQS ("hTP10") | (SEQ ID NO: 102) |
| SLMGDEFKAVLET ("hTP11") | (SEQ ID NO: 103) |
| QHSVAYTFEED ("hTP12") | (SEQ ID NO: 104) |
| INPEIITRDG ("hTP13") | (SEQ ID NO: 105) |
| DISLTGDPVITASYL ("hTP18") | (SEQ ID NO: 106) |
| DISLTGDPVITA ("hTP20") | (SEQ ID NO: 107) |
| DQSIDFEIDSA ("hRitsch-1") | (SEQ ID NO: 108) |
| KNVSEDLPLPTFSPTLLGDS ("hRitsch-2") | (SEQ ID NO: 109) |
| KNVSEDLPLPT ("hRitsch-3") | (SEQ ID NO: 110) |
| CDSGRVRTDAPD ("hCETP-intern") | (SEQ ID NO: 111) |
| FPEHLLVDFLQSLS ("hCTP C-Term") | (SEQ ID NO: 112) |

### β-amyloid epitope:

### DAEFRHDSG (SEQ ID NO: 65)

**CCR5 epitopes:**

| | |
|---|---|
| HYAAAQWDFGNTMCQL (Chackerian, 1999) | (SEQ ID NO: 113) |
| AAQWDFGNTMCQ (Barassi et al., 2005) | (SEQ ID NO: 114) |
| RSQKEGLHYT (Misumi et al., 2006) | (SEQ ID NO: 115) |

**TNF-α epitopes:**

| | |
|---|---|
| SSRTPSDKPVAHWANPQAE ("TNF-α V1") | (SEQ ID NO: 116) |
| SRTPSDKPVAHWANP ("TNF-α V2") | (SEQ ID NO: 117) |
| SSRTPSDKP ("TNF-α V3") | (SEQ ID NO: 118) |

**IL-17 epitopes:**

| | |
|---|---|
| NADGNVDYHMNSVP ("IL-17 V1") | (SEQ ID NO: 119) |
| DGNVDYHMNSV ("IL-17 V2") | (SEQ ID NO: 120) |

**IL-6 epitopes:**

| | |
|---|---|
| RSFKEFLQSSLRALRQ ("IL-6 V1") | (SEQ ID NO: 121) |
| FKEFLQSSLRA ("IL-6 V2") | (SEQ ID NO: 122) |

**HER2/Neu epitope:**

| | |
|---|---|
| QMWAPQWGPD (Riemer et al. 2007) | (SEQ ID NO: 123). |

As described earlier it is one embodiment to modify structural proteins in order to retarget the parvovirus to a different cell or tissue. Therefore, in another preferred embodiment of the present invention the amino acid insertion is a sequence that brings about an increase in the transducing activity of the mutated parvovirus. Increase in the transducing activity according to this invention means preferably that the ratio of genomic particles divided by transducing particles (GenP/tP) as determined in Example 6.1 is lowered for a cell line of choice compared to the respective unmutated parvovirus. An increase in this context preferably refers to a decrease of GenP/tP of at least about 25 %, more preferably at least about 100 %, still more preferably at least about 300 %, most preferably at least about 1000 %.

Such increase in the transducing activity is usually accomplished by an amino acid insertion that mediates binding of the structural protein in the form of a particle to a cell membrane receptor. These inserted targeting sequences can be known ligands or parts thereof for a given receptor. Further, the targeting sequences can be sequences that have been identified by phage display or by AAV display, where a library of amino acid sequences is displayed on the surface of phages or AAV and such phages/AAVs are identified that specifically bind to a receptor of choice. The inserted amino acid sequence can be sequenced and transferred into I-453. In a preferred embodiment the amino acid sequence is directly identified from an AAV-library, where the library of amino acid sequences had been inserted into I-453, for example in analogy to the AAV-2 libraries described in (Perabo et al., 2003, Lieber, 2003, Muller et al., 2003), (WO 03/054197). In this context the advantage is used that the amino acid insert is identified in the same surface context as it is used later on. Therefore, the conformation is not changed compared to settings where a targeting sequence is transferred from an original context (e.g. as part of ligand or from a phage) to the site I-453.

In an especially preferred embodiment the inserted targeting sequence does contain an RGD motif, especially it is the sequence ACDCRGDCFCA (SEQ ID NO: 84), herein referred to as RGD-4C peptide. The RGD motif in general and especially the RGD-4C peptide mediate the binding to the integrins, especially α_{V}β₃ and a_{V}β₅. Consequently, these structural proteins can be used to target cells expressing these integrins.

In a further preferred embodiment the insertion brings about an alteration in a chromatographic property of the structural protein. It is preferred to insert a known tag that can be used for binding the structural protein or a particle composed of the structural protein to a ligand. Such tags are well known in the art. Examples are given in Table 2.

**Table 2: Tags and corresponding ligands**

| **Tag** | **Ligand** |
|---|---|
| HIS | Nickel |
| GST | Glutathione |
| Protein A | IgG |
| Biotin or Strep | Streptavidin |
| Calmodulin-binding peptide | Calmodulin |
| Fc-Peptide of IgG | Protein A |
| Flag | FLAG- or 3xFLAG peptide |
| HA (hemagglutinin) | HA peptide |

Depending on their use the particles of this invention may have to be purified to high purity. Otherwise unmodified structural proteins can be modified by insertion to alter their chromatographic properties as it has been described in WO 01/05991. In case of AAV-2 the HSPG binding capabilities due to the loop structure around I-587 remain unchanged if a tag is inserted into I-453. Furthermore, modified structural proteins that comprise for example a targeting insert and/or an epitope at a site different to I-453 can be further modified to display a tag that enables simple and scalable purification to high purity.

The insertion according to this invention may have an N- and/or C-terminal linker. A linker according to this invention is a further stretch of at least one amino acid N-and/or C-terminal of the inserted epitope, targeting sequence or tag, preferably of 2-12 amino acids. Preferred amino acids for the linker are amino acids selected from the group consisting of Ala, Gly, Ser, Pro, and Cys, especially 3 Ala upstream and 2 downstream of the B-cell epitope, 5 Ala upstream and 5 downstream of the B-cell epitope, or 3-5 Gly upstream and 3-5 Gly downstream of the B-cell epitope.

In a further preferred embodiment the insertion comprises linker sequences which enable a circularization of the inserted peptide sequences in order to better display the insertion. Accordingly spacer sequences are selected to form Zinc-fingers (Zn-finger), well known in the art. Preferred Zn-finger motifs are CXXC or C₂H₂:. Preferred Zn-finger motifs are C₂H₂, C₄, and C₂HC including but not limited to motifs CX₂CXₙC₂, CX₂CX₁₀₋₃₀CX₂C, CX₅HX₁₀₋₃₀CX₂C, CX₂CX₁₀₋₃₀CX₄H (Laity et al., 2001, Gamsjaeger et al., 2007).

An example of a preferred Zn-finger linker is:

X₍₃₋₅₎CXXCX₍₀₋₅₎(NNK)ₙ X_{(O-5)}CXXCX₍₃₋₅₎

(X=Gly or Ala, C=Cys; with each N being any nucleotide and K standing for G or T). Thus the random NNK sequence protrudes from the capsid surface.

In an especially preferred embodiment the linker comprises at least one Cys N-terminal and at least one Cys C-terminal of the insertion. Such cysteins are capable of forming a disulfide bond to generate a loop that stabilizes the insertion and thereby facilitates its binding to its antibody, receptor or ligand.

It is a further embodiment of the present invention that the parvovirus structural protein comprises one or more further mutation(s) at a site different from I-453. This/These further mutation(s) is/are independently selected from the group consisting of a point mutation, an internal deletion, a terminal deletion, an insertion and a substitution. In general, the purpose of such further mutation may be selected from the same group of purposes as explained above for the insertion into I-453, namely to generate a vaccine, to target a vector to a different cell/tissue and/or change the chromatographic properties in order to purify the particles to high purity. Accordingly, an additional insertion may again be an epitope, preferably a B-cell epitope or CTL epitope (as further specified below), especially a tolerogen-derived epitope, a targeting sequence that enhances the transduction activity, preferably a ligand to a receptor of choice, or a tag. For further characterization of these further insertions reference is made to the above sections that described the features for the I-453 insertion that equally apply for a second insertion.

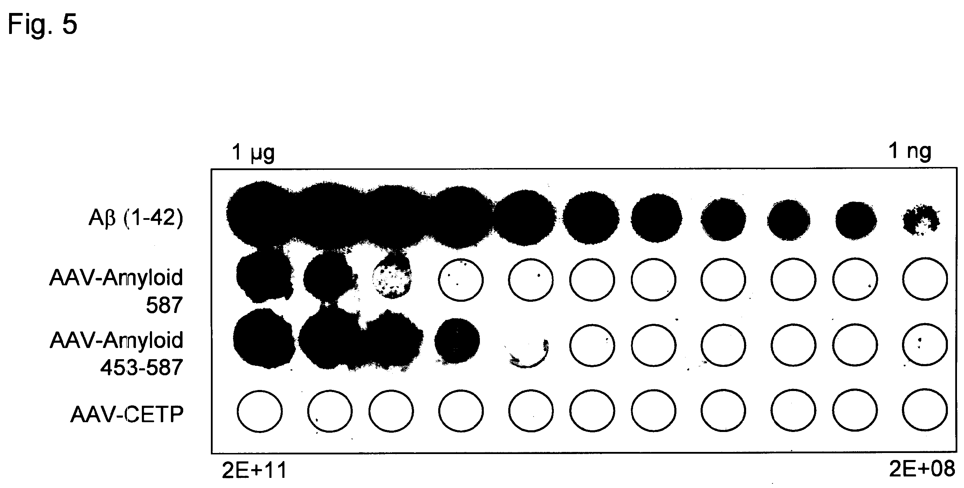

This further peptide can be identical to the insertion in I-453. This is preferred if it is key to have a large number of identical peptides being optimally presented on the surface of a particle, especially in cases where direct B-cell receptor (BCR) crosslinking is required for T-cell independent priming of B-cells and breaking of tolerance against self-antigens. A higher density of B-cell epitopes increases the likelihood of optimal peptide-specific BCR crosslinking which requires a defined distance between BCRs (e.g. about 5-10 nm), and therefore, respective B-cell epitopes being presented on a parvovirus capsid. Furthermore, as shown in this invention (Figure 5), modifications of parvovirus capsids at two or more different sites at a time can lead to a conformational cross-talk within the capsid structure leading to an improved presentation of the inserted peptide sequence. Moreover, a larger number of inserted B-cell epitopes decreases the probability for undesired immune reactions against the parvovirus backbone due to i) masking of natural parvovirus B-cell epi-/mimotopes and/or ii) slight structural capsid changes rendering these natural B-cell epi-/mimotopes less immunogenic.

Consequently, in this case it is especially preferred that the inserted peptide is a B-cell epitope, even more preferred a tolerogen-derived epitope. Parvovirus structural proteins displaying a high number of epitopes on the surface of a virus-like particle/capsid can efficiently be used to generate T-cell independent B-cell responses and even break tolerance. As shown in examples 5.4 and 6.2, identical epitopes, respectively B-cell epitopes can be inserted in two different insertion sites, here I-453 and I-587, and are accessible to soluble ligand, in this case α_{V}β₃, or β-amyloid, respectively. Therefore, it is an especially preferred embodiment of this invention that an identical peptide is inserted at I-453 and I-587and that this peptide is a B-cell epitope, most preferred a tolerogen-derived epitope. Another preferred double insertion variant is a variant with insertions at I-453 and I-261.

However, the further peptide can be a different one compared to the peptide inserted into I-453.

Therefore, it is a further embodiment of the present invention that an insertion at position I-453 is combined with at least one further amino acid insertion at one or more additional site. Additional suitable insertion sites identified by using AAV-2 are well known in the art and are exemplarily listed in Table 3. However, it should be understood that the further amino acid insertion at one or more additional site(s) is/are not limited to those listed in the following.

**Table 3: Further insertion sites**

| **Insertion site** | **corresp. amino acid / sequence of AAV-2** | | **SEQ ID NO:** | **References** |
|---|---|---|---|---|
| I-1 | M₁ | M₁ AADGY | SEQ ID NO: 17 | (Wu et al., 2000) |
| I-34 | P₃₄ | PPPKP₃₄ AERHK | SEQ ID NO: 18 | (Wu et al., 2000) |
| I-138 | T₁₃₈ | EPVKT₁₃₈ APGKK | SEQ ID NO: 19 | (Wu et al., 2000, Warrington et al., 2004, Lux et al., 2005) |
| I-139 | A₁₃₉ | PVKTA₁₃₉ PGKKR | SEQ ID NO: 20 | (Shi et al., 2001, Shi and Bartlett, 2003, Arnold et al., 2006) |
| I-161 | K₁₆₁ | SGTGK₁₆₁ AGQQP | SEQ ID NO: 21 | (Shi et al., 2001, Arnold et al., 2006) |
| I-261 | S₂₆₁ | YKQIS₂₆₁ SQSGA | SEQ ID NO: 22 | (Girod et al., 1999) |
| I-266 | A₂₆₆ | SQSGA₂₆₆ SNDNH | SEQ ID NO: 23 | (Wu et al., 2000) |
| I-381 | N₃₈₁ | YLTLN₃₈₁ NGSQA | SEQ ID NO: 24 | (Girod et al., 1999) |
| I-447 | R₄₄₇ | YYLSR₄₄₇ TNTPS | SEQ ID NO: 25 | (Girod et al., 1999, Wu et al., 2000) |
| I-448 | T₄₄₈ | YLSRT₄₄₈ NTPSG | SEQ ID NO: 26 | (Grifman et al., 2001) |
| I-459 | R₄₅₉ | TTQSR₄₅₉ LQFSQ | SEQ ID NO: 27 | (Shi et al., 2001, Arnold et al., 2006) |
| I-471 | R₄₇₁ | ASDIR₄₇₁ DQSRN | SEQ ID NO: 28 | (Asokan and Samulski, 2006, Moskalenko et al., 2000) |
| I-520 | G₅₂₀ | LVNPG₅₂₀ PAMAS | SEQ ID NO: 29 | (Shi et al., 2006) |
| I-534 | F534 | EEKFF₅₃₄ PQSGV | SEQ ID NO: 30 | (Girod et al., 1999) |
| I-570 | P₅₇₀ | RTTNP₅₇₀ VATEQ | SEQ ID NO: 124 | own data |
| I-573 | T₅₇₃ | NPVATₛ₇₃EQYGS | SEQ ID NO: 31 | (Girod et al., 1999) |
| I-584 | Q₅₈₄ | STNLQ₅₈₄ RGNRQ | SEQ ID NO: 32 | (Shi et al., 2001, Shi and Bartlett, 2003, Shi et al., 2006) |
| I-587 | N₅₈₇ | LQRGN₅₈₇ RQAAT | SEQ ID NO: 33 | (Girod et al., 1999, Shi et al., 2001, Grifman et al., 2001, Ried et al., 2002, Nicklin et al., 2001, Work et al., 2004, White et al., 2004, Arnold et al., 2006, Maheshri et al., 2006, Work et al., 2006) |
| I-588 | R₅₈₈ | QRGNR₅₈₈ QAATA | SEQ ID NO: 34 | (Shi and Bartlett, 2003, Muller et al., 2003, Waterkamp et al., 2006) |
| I-591 | A₅₉₁ | NRQAA₅₉₁ TADVN | SEQ ID NO: 35 | (Wu et al., 2000) |
| I-657 | P₆₅₇ | VPANP₆₅₇ STTFS | SEQ ID NO: 36 | |
| I-664 | A₆₆₄ | TFSAA₆₆₄ KFASF | SEQ ID NO: 37 | (Wu et al., 2000) |
| I-713 | T₇₁₃ | NVDFT₇₁₃ VDTNG | SEQ ID NO: 38 | |
| I-716 | T₇₁₆ | FTVDT₇₁₆ NGVYS | SEQ ID NO: 39 | (Maheshri et al., 2006) |

I-570 is especially suitable as an insertion site that goes along with a deletion of given amino acids of the parvovirus structural protein at the site of insertion, leading to a complete substitution. In this case the amino acids RTTNPVATEQ can be substituted by a targeting peptide or epi- or mimotope.

Insertions have successfully also been made into AAV-serotypes other than AAV-2 (Table 4).

**Table 4: Insertions into AAV-serotypes other than AAV2**

| **AAV serotype** | **Sequence** | **SEQ ID NO :** | **Ins. site/amino acid relative to AAV2** | | **References** |
|---|---|---|---|---|---|
| AAV1 | FQSSS₅₈₈ TDPAT | SEO ID NO: 125 | I-587 | N₅₈₇ | own data |
| AAV1 | SSSTD₅₉₀ PATGD | SEQ ID NO: 40 | I-589 | Q₅₈₉ | (Arnold et al., 2006, Stachler and Bartlett, 2006) |
| AAV3 | NNLQS₅₈₆-SNTAP | SEQ ID NO: 41 | I-585 | K₅₈₅ | (Arnold et al., 2006) |
| AAV4 | GGDQS₅₈₄-NSNLP | SEQ ID NO: 42 | I-585 | | (Arnold et al., 2006) |
| AAV5 | TNNQS₅₇₅-STTAP | SEQ ID NO: 43 | I-585 | | (Arnold et al., 2006) |

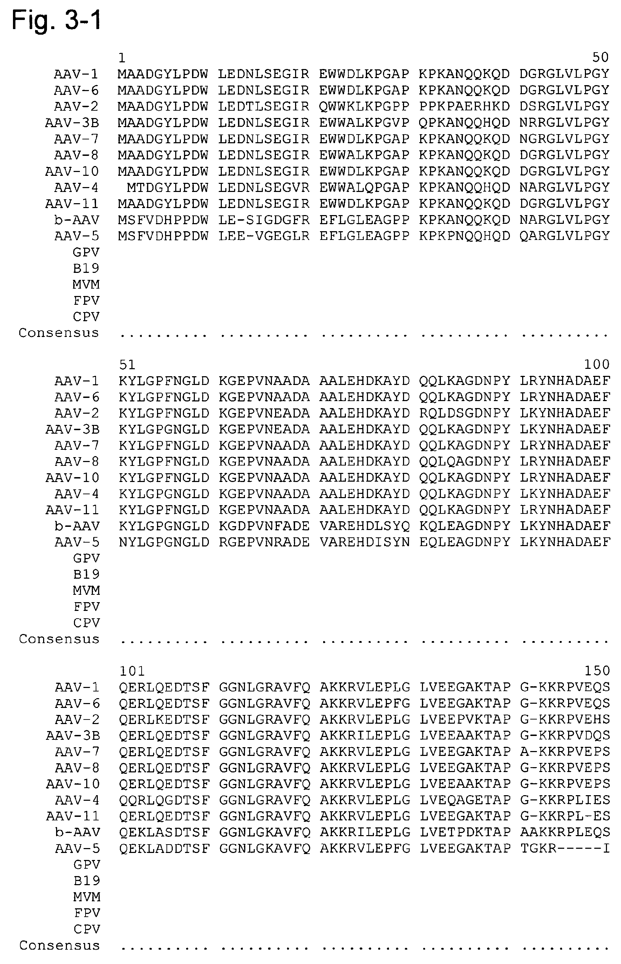

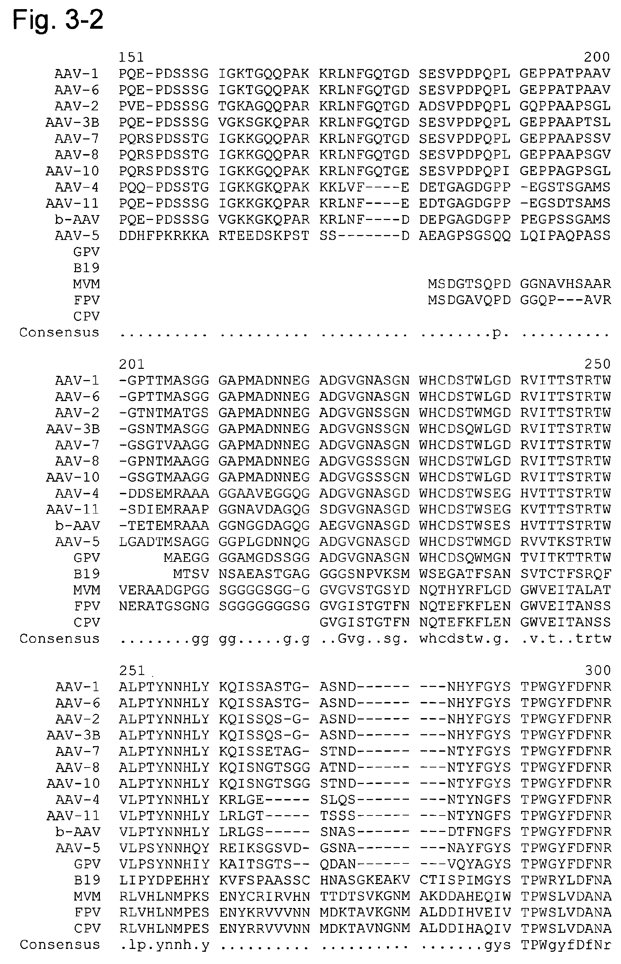

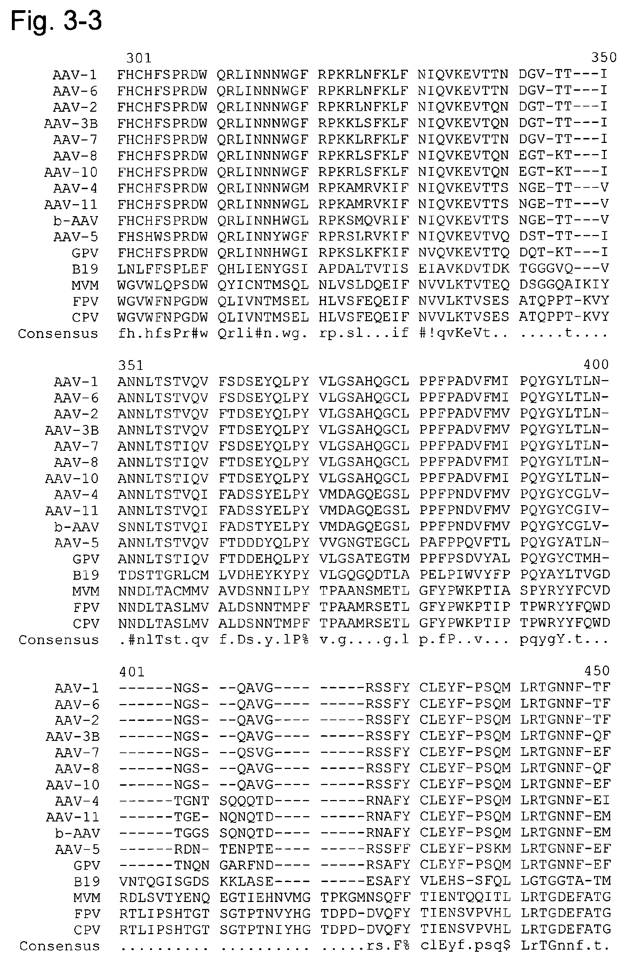

The used nomenclature I-### within this invention refers to the insertion site with ### naming the amino acid number relative to the VP-1 protein of AAV2, however meaning that the insertion may be located directly N- or C-terminal, preferably C-terminal of one amino acid in the sequence of 5 amino acids N- or C-terminal of the given amino acid, preferably 3, more preferably 2, especially 1 amino acid(s) N- or C-terminal of the given amino acid. For parvoviruses other than AAV2 the corresponding further insertion sites can be identified by performing an amino acid alignment or by comparison of the capsid structures, if available. Such alignment has been performed for the parvoviruses AAV1, AAV6, AAV2, AAV3b, AAV7, AAV8, AAV10, AAV4, AAV11, b-AAV, AAV5, GPV, B19, MVM, FPV and CPV (see Figure 3).

Most of the work on targeting parvoviruses was done using AAV2. However, due to the high conservation of at least large stretches and the large member of closely related family members it is easy to identify corresponding sites of AAV2 within other parvoviruses, e.g. by using alignments as shown in Figure 3.

An insertion into the corresponding position of the coding nucleic acid of one of these sites of the cap gene leads to an insertion into VP-1, VP-2 and/or VP-3, as the cap proteins are encoded by overlapping reading frames of the same gene with staggered start codons. Therefore for AAV2, according to this nomenclature insertions between amino acids 1 and 138 are only inserted into VP-1, insertions between 138 and 203 are inserted into VP-1 and VP-2, and insertions between 203 and the C-terminus are inserted into VP-1, VP-2 and VP-3, which is of course also the case for the insertion site I-453. A schematic organization of the cap gene of AAV2 is provided in Figure 1. Therefore, the present invention encompasses structural genes of parvoviruses with corresponding insertions in the VP-1, VP-2 and/or VP-3 proteins.

More preferred additional insertion sites are I-138, I-261, I-570, I-575, I-584, I-587, I-588 and I-590.

The most preferred further insertion site is I-587, as various insertions have been made in the amino acid stretch around N₅₈₇ (LQRGN₅₈₇ RQAAT) of AAV2. Within this stretch insertions of various peptides were made C-terminal of amino acids Q₅₈₄, N₅₈₇, R₅₈₈ and A₅₉₁ in AAV2 (Table 3) and C-terminal of amino acids of other AAV-serotypes corresponding to R₅₈₅ and Q₅₈₉ of AAV2 (Table 4).

Amino acid 138 is the N-terminus of VP-2. Preferred embodiments are VP-2 structural proteins with an additional N-terminal fusion to one of the amino acids within the stretch T₁₃₈ APGKKR. In order to achieve an N-terminal fusion to VP-2 only, one could use an expression construct with the coding sequence for VP-2 with the respective insert comprising its own start codon. This construct would be co-transfected with a vector construct where the start codon for VP-2 was eliminated.

Further, preferably the further inserted nucleic acid sequence may be inserted at any site corresponding to the first amino-terminal amino acids 1 to 50 of VP-1.

Within this invention an AAV2 structural protein was generated that contained an insertion of an β-amyloid tolerogen-derived epitope both at I-453 and I-587. Surprisingly, it was shown that compared to a structural protein containing the same insert only at I-587 the respective particles were much better recognized by a β-amyloid-specific antibody (see example 5).

Additionally encompassed by this invention are point mutations such as substitutions or internal deletions, where at least one amino acid is deleted or replaced by a different amino acid that decreases binding of the structural protein and/or respective particles composed of the structural proteins to primary or secondary cellular receptors for the respective virus. This detargeting of the virus from its natural host cell is important especially if systemic versus local or loco-regional administration of the particles is intended, as uptake of the particles by the natural host cells limits the effective dose of the particles. In case of AAV2 and AAV6 HSPG is reported to be the primary receptor for viral uptake in a large number of cells, especially liver cells. For AAV2 HSPG-binding activity is dependent on a group of 5 basic amino acids, R₄₈₄, R₄₈₇, R₅₈₅, R₅₈₈ and K₅₃₂ (Kern et al., 2003). Recently it was reported that the lysine-to-glutamate amino acid substitution K₅₃₁E leads to the suppression of AAV6's ability to bind heparin or HSPG ((Wu et al., 2006)).

Accordingly, preferred point mutations are those that reduce the transducing activity of the particle for a given target cell mediated by the natural receptor by at least 50%, preferably at least 80%, especially at least 95%, in case of HSPG as primary receptor the binding of the particles to HSPG. Transducing ability can be determined as described in example 6.1 as the GenP/tP ratio (see also above).

Consequently, further mutations preferred for HSPG-binding particles are those mutations that deplete or replace a basic amino acid such as R, K or H, preferably R or K which is involved in HSPG binding of the respective virus, by a non-basic amino acid such as A, D, G, Q, S and T, preferably A or an amino acid that is present at the corresponding position of a different but highly conserved AAV serotype lacking such basic amino acid at this position. Consequently preferred amino acid substitutions are R₄₈₄A, R₄₈₇A, R₄₈₇G, K₅₃₂A, K₅₃₂D, R₅₈₅A, R₅₈₅S, R₅₈₅Q, R₅₈₈A or R₅₈₈T, especially R₅₈₅A and/or R₅₈₈A for AAV2, and K₅₃₁A or K₅₃₁ E for AAV6.

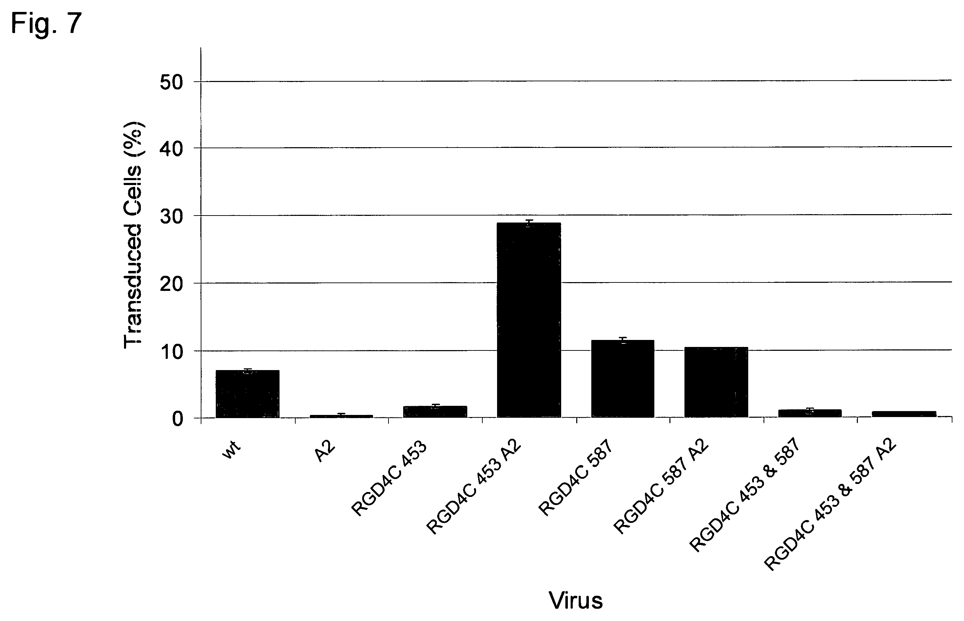

One especially preferred embodiment of the invention are such mutimeric structures comprising structural protein mutants of AAV2 that additionally contain the two point mutations R₅₈₅A and R₅₈₈A as these two point mutations are sufficient to ablate HSPG binding activity to a large extent. These point mutations enable an efficient detargeting from HSPG-expressing cells which - for targeting purposes - increases specificity of the respective mutant virus for its new target cell. Furthermore, these point mutations seem to lead to a structural change rendering the RGD4C 453 A2 capsid mutant transducing (Figure 7).

It is also an embodiment of the present invention that the parvovirus mutated structural protein comprises at least one further mutation that reduces the ability to induce a B-cell response against a parvovirus-specific epitope and/or mimotope, thereby reducing the natural antigenicity of the respective particle. Administration of particles, either as vaccines or as vectors, is usually hampered by existing antibodies directed against certain epitopes of the particle/capsid. In case of AAV2, the majority of the human population has an AAV2 positive serum status. In case of non-human parvoviruses the patient will generate a strong humoral immune response against the particle backbone that may neutralize the vector or may dominate an intended immune response against an inserted epitope. Point mutations as well as insertions have been described to modify the natural antigenicity of particles to evade a preexisting immune response (e.g. (Huttner et al., 2003); WO 01/05990). Perabo et al. were able to identify point mutations that increased AAV2's immune-escaping ability by up to 5.5-fold higher N50 values (amount of human serum needed to halve the number of transduced cells) in comparison to AAV2 with wild-type structural proteins (Perabo et al., 2006a), namely a point mutation at positions R₄₅₉ and N₅₅₁. Therefore, a further preferred embodiment is a mutimeric structure comprising a mutated structural protein that further comprises a point mutation that reduces the ability to induce a B-cell response against an AAV-specific epitope and/or mimotope, preferably for AAV2 a point mutation at position R₄₅₉ and/or N₅₅₁.

A further mutation can be also used to compose more complex mimotopes or to enhancing the correct display of an inserted amino acid sequence. Such further mutations can either be identified by using structure prediction software or by inserting random point mutations into the respective cap gene e.g. by error prone PCR and then selecting for matured display of the inserted amino acid sequence.

In a further preferred embodiment the further mutation might be adequate to introduce at least one cytotoxic T-cell epitope (CTL epitope). For both infectious diseases and cancer it is most useful to combine both humoral and cellular immune responses to fight these diseases. The multimeric structures according to this invention are in principle capable of pseudo-infecting cells. Accordingly these structures - like viruses - are able to enter cells, are processed to peptides, the peptides are loaded onto MHC class I and II molecules and finally presented to CD8- or CD4-positive T cells. The T-cells become stimulated after specific recognition of such processed peptide presented by MHC class I or II molecules. As a consequence of such stimulation CD8 cells may differentiate into cytotoxic T cells and then cause a cellular immune response. CD4 cells may develop into T helper cells which stimulate B cells to provide a humoral immune response or CD8-positive T cells to provide a cytotoxic immune response, which may themselves induce lysis of infected cells and other cells carrying and presenting the same peptide. Suitable CTL epitopes are known in the art for various cancer antigens or viral antigens, or they can be predicted from given antigen sequences using for example the peptide prediction program by Parker under http://www-bimas.cit.nih.gov/molbio/hla_bind/ (Parker et al., 1994). Proposed CTL epitopes can be validated according to the methods as exemplified for HPV-epitopes in US 6,838,084, examples 2-8 (herein incorporated by reference). As processing of CTL epitopes occurs within the cell it is not necessary that such CTL epitopes are located on the surface or are present in a specific conformation.

A further embodiment of the present invention is a library of structural proteins, wherein the library comprises a set of different parvoviral structural proteins inserted at I-453 as described above. AAV2 libraries have been previously described for the insertion site I-587(Perabo et al., 2003, Lieber, 2003, Muller et al., 2003). The same technology can equally be applied to the newly identified insertion site I-453. The advantages described for the insertion site I-453 for insertions versus I-587 and other sites in general equally apply to a library generated for the insertion site I-453. E.g. using I-453 based libraries may result in the selection of other peptides (as for example with I-587 based libraries) since neighboring residues may have an influence on the exposure and functionality of the peptides inserted into the structural protein. In addition, the sites (I-587 and I-453) are located on different loops of the AAV capsid. Thus a different mechanism of cell interaction can be assumed. Furthermore, AAV particles derived from I-453 libraries can be purified with heparin affinity chromatography, as the heparin binding site overlapping with I-587 is still intact. Further, a library with inserted sets of amino acid insertions in both I-453 and a further insertion site can be used to enhance the multiplicity of the library and select structural proteins with higher binding affinities.

In a preferred embodiment genotype and phenotype of virion particles of the library are coupled. This means that the genomic mutant of the virion is identical to the phenotypic mutant of the same virion or, in other words, that each structurally modified virus codes for its structural protein mutant.

In contrast to a bacterial transformation, where only one bacteriophage is taken up by one bacterial cell, using transfection methods for eukaryotic cells many DNA copies (up to 1x10⁶) can be taken up per cell (Dean et al., 2005). Therefore, in the case of an AAV library one cell can replicate thousands of AAV genomes at the same time where each may express a different mutated structural protein with a different peptide sequence inserted into VP1, VP2, and/or VP3 of AAV. At least some of these structural proteins can assemble a complete viral capsid (consisting of 5 VP1, 5 VP2 and 50 VP3 proteins) encapsidating essentially only one of the thousands of AAV genomes present in the cell. In case of a geno-/phenotypically coupled library at least 10%, preferably more than 25%, especially more than 50% of the resulting AAV particles have an encapsulated genome which codes for at least 25%, preferably more than 50%, especially more than 80% of the 60 VP proteins of which its capsid is composed. As a consequence, if an uncoupled library was used for a first screening against a target antibody or target receptor, the chance that screened particles contained the genome coding for this specific amino acid sequence might be very low.

In general, geno-/phenotypically coupled virion particles/libraries are obtained when introducing essentially one single copy of the virus genome into each virion production cell entering the cell nucleus. This cell will only produce capsid protein variants encoded of exactly the introduced genome which is replicated and afterwards packaged into the mutant virion particle. Different experimental settings can ensure this:
To obtain a geno-/phenotypically coupled library of parvovirus virions a library of parvovirus virions is produced by transfecting a plasmid library into production cells under suitable conditions whereas a low copy number of viral genomes equal to or less than 100 genomes per cell is used, preferably equal to or less than 10 genomes, more preferably equal to or less than one genome per cell, resulting in geno-/phenotypically coupled virions/library. The overall transfection efficacy will be finally decisive for the ideal number of virus genomes per cell to be transfected.

The required amount of virus plasmid can be quantified, if e.g. autonomous replicating plasmids with similar size as the virus genome encoding a reporter gene such as GFP are used as a model system. Autonomous replicating plasmids are e.g. systems comprising SV40 origin of replication and large T antigen or the EBV (ebstein barr virus) P1 origin and EBNA. Increasing amounts of the self-replicating reporter gene plasmid are cotransfected with carrier DNA such as empty plasmid DNA (e.g. pUC derivates) keeping the amount of total DNA constant. In theory, each cell transfected with the reporter gene plasmid will, due to its self-replication, express sufficient amounts of reporter protein to be detected. At some ratio of reporter gene vector to carrier DNA, a further increase of reporter gene plasmid will lead to a corresponding increase in the number of transfected cells. By this means, the ideal amount of self-replicating reporter gene plasmid can be determined, reflecting the ideal amount of vector genomes.

Similarly, another read-out system for detection of successfully transfected cells are methods such as in-situ PCR to detect the transfected plasmid genome on a single cell level.

Alternatively, the geno-/phenotypically coupled library of parvovirus virions can be produced by transducing a (non- or partially coupled) virion library into production cells under suitable conditions at a ratio of genomes per cell of 5 to 5,000, preferably 10 to 1,000, more preferably 50 to 300, especially approximately 100, and selecting transduction conditions to be independent from infection pathways, particularly through unspecific uptake through pinocytosis and/or phagocytosis, resulting in geno-/phenotypically coupled virions/library.

Especially relevant for the screening of structural proteins having epitope inserts is the method used for infecting cells after binding virions have been separated from non-binding virions. It is known that a peptide insertion into the I-587 site of AAV2 frequently destroys (depending on the sequence of the inserted peptide) the heparin binding motif required for efficient infection of HSPG-receptor containing cells such as HeLa or 293 cells. It has now been found that an insertion in I-453 optionally in combination with I-587 can alter the transducing activity of respective virions. Therefore, simple infection methods could bias the screening method and lead only to mutants that still can enter HeLa cells specifically through the respective receptor, in case of AAV2 through heparan sulfate proteoglycan (HSPG). Therefore, an unspecific uptake of the virus particle by the production cell may be advantageous. Such unspecific uptake can be achieved by seeding production cells on immobilized parvovirus virions. For this method, the virions are directly coated to a support such as a tissue culture plate. Alternatively, first a capsid-specific antibody (in case of AAV2 for example A20) is coated to the support and second the capsids are bound to the coated antibodies. The advantage in the latter case is that the antibody/virus particle complex, respectively the virus particle itself is more efficiently detached from the support and thereby internalized by the cell. Importantly, introduction of foreign peptide sequences into I-453 of AAV2 does not destroy the affinity of A20 to the respective mutant particle as the epitopes of A20 are hardly if at all affected by the peptide insertion. The cells, e.g. HeLa cells, are finally seeded on the bound capsids. It is expected that this procedure leads to an uptake of the virus, e.g. AAV, by the cell independent of the natural infectious pathway, presumably by pinocytosis and/or phagocytosis.

Alternatively or additionally, the selection step can be carried out on cells expressing a specific receptor for a binder of choice which is used for selecting the wanted parvoviral variant. E.g. cells can be used which express the FcγRI which is specific for any binder comprising an Fc-part of an antibody. For this example, such FcγRI expressing cells can be transduced with a library pool of parvoviruses. First, a negative selection can be performed to avoid unspecific selection of parvoviral candidates which by themselves are able to transduce cells independently from an interaction of a binder with the FcγRI. Therefore, FcγRI-expressing cells are incubated with the library pool. The supernatant (pool of parvoviruses which is not able to transduce the cells) is collected and subsequently incubated with the binder of choice (e.g. selection antibody) to perform the positive selection. In the positive selection parvoviruses decorated with the binder will be able to transduce FcγRI expressing cells through the coupling of the binder to the FcγRI on the surface of the cells. The transduced cells can subsequently be used to amplify the particles in the presence of the binder, assuming that the intracellular trafficking is not impaired within the cells.

In a further preferred embodiment a geno-/phenotypically coupled library of parvovirus virions can be obtained by a method where selected virions are specifically taken up by production cells. In this case the library of parvovirus virions is produced by transducing the library into production cells under suitable conditions at a ratio of genomes per cell of 10 to 10,000, preferably 50 to 5,000, more preferably 100 to 3,000, especially approximately 1,000, wherein transduction conditions are selected to be dependent on infection pathways, particularly through specific receptor binding, resulting in geno-/phenotypically coupled virions/library. In order to achieve such receptor-specific uptake the virions of the library are preferably not immobilized but added to the cells in suspension, whereas both cells and virions can be in suspension or cells are immobilized and virions are added in suspension. Therefore, the transfection of the cells is basically dependent on the virus's infection pathway.

Dependence on infection pathways means that virions are taken up by the cells e.g. through receptor-specific uptake, e.g. for AAV2 heparin sulfate proteoglycan (HSPG)-specific uptake (e.g. for virion libraries where natural infection pathways are not blocked or destroyed by the inserted random peptide sequences). To keep biodiversity of the library during the coupling step (either by transfection of virus genomes or by cell transduction with virion particles by either means, uptake or infection), always a at least 10-fold, preferably 100-fold, especially 500-fold excess of genomic particles compared to the multiplicity of parvoviral mutants should be transduced in order to ensure that each virus variant is amplified. To further ensure that each virus is coupled in the resulting library an at least 2-fold, preferably at least 5-fold excess of cells is to be used compared to total number of genomic particles.

Geno-/phenotype coupling is desired as the genetic information of the packed DNA can easily be used to obtain the sequence of those particles having high affinity or avidity to the respective antigen binder. It is an object of the invention to use for the identification of a parvovirus mutated structural protein such geno-/phenotypically coupled libraries with a coupling of at least 5%, preferably of at least 25% and more preferably of at least 50%, especially at least 90%.

In a preferred embodiment the library of the present invention has a multiplicity of parvoviral mutants of greater than 10³, preferably greater than 10⁵, more preferably greater than 10⁶, especially greater than 10⁷ Multiplicity means according to this invention the number of different virions or viral genomes within the library. In principal it is advantageous to use a library of high multiplicity as the likelihood to identify an optimal clone increases with the multiplicity of the library. The multiplicity of the library is generated by insertion of a nucleic acid insert into the coding region of the gene encoding a parvoviral structural protein leading to an amino acids insertion into a position within the parvoviral structural protein.

One embodiment of the present invention is a multimeric structure comprising a parvovirus structural protein of the present invention. A multimeric structure according to this invention is a structure of at least 5, preferably at least 10, more preferably at least 30, most preferably at least 60 structural proteins. They can form regular particles such as capsomeres, virus-like particles (empty viral shells) or capsids. Alternatively, they also can form non-regular aggregates. As explained above the formation of particles capable of packaging a viral genome is a highly preferred feature, particularly if the structural proteins of this invention shall be used as viral vectors. In case the structural proteins are intended for use as vaccines such particle formation may not be necessary to exert a sufficient immune response and capsomeres or aggregates may be sufficient. Still, it is believed that particle formation is also beneficial for the display of the inserted epitopes, especially if direct cross linking of B-cell epitopes is necessary for breaking tolerance.

One embodiment of the present invention is a nucleic acid encoding a structural protein as described above. The nucleic acid is preferably a vector comprising the claimed nucleic. Nucleic acids, especially vectors are necessary to recombinantly express the structural proteins of this invention.

A further embodiment is a library of vectors, wherein the library comprises a set of different vectors described above. In a preferred embodiment the library has a multiplicity of parvoviral mutants of greater than 10³, preferably greater than 10⁵, more preferably greater than 10⁶, especially greater than 10⁷. Multiplicity means according to this invention the number of different virions or viral genomes within the library. In principal it is advantageous to use a library of high multiplicity as the likelihood to identify a suitable or even ideal clone increases with the multiplicity of the library.

Another embodiment of the present invention is a virus, preferably a parvovirus as further characterized above, comprising a nucleic acid as characterized above or a vector as characterized above.

Another embodiment of the present invention is an isolated cell comprising a nucleic acid as characterized above or a vector as characterized above.

A further embodiment of the mutated structural proteins according to this invention is their use for gene therapy. The gene therapy vector is formulated to contain common salts, buffer and excipients. The gene therapy vector according to this invention can be administered by common routes of administration such as intra-venously or local or loco-regional.

Also described is a process for the preparation of a structural protein of a parvovirus, the method comprising the steps of:
a) expressing a nucleic acid according to this invention under suitable conditions, and
b) isolating the expressed structural protein of step a).

A further embodiments of the present invention is a method for altering the tropism of a parvovirus, the method comprising the steps of: a) co-expressing parvoviral helper and vector functions, wherein the helper function expresses a parvoviral structural protein according to this invention under conditions that enable parvovirus formation, and b) isolating the parvovirus

A further embodiment of the present invention is a method for displaying an epitope on the surface of a parvovirus, the method comprising the steps of: a) expressing the nucleic acid according to this invention under suitable conditions, and b) isolating the expressed structural protein of step a).

Also described is a method for vaccinating a mammal, the method comprising the vaccination of a mammal, preferably a human, with a structural protein, preferably a particle according to this invention. As disclosed above the structural protein is formulated to contain common salts, buffer, excipients and/or adjuvants. Preferred adjuvants are listed below. The vaccines according to this invention can be administered by common routes of administration as described below. Such vaccination is preferably used for breaking immune tolerance, but also for treating infectious diseases, the method comprising the vaccination of a mammal, preferably a human, with a structural protein according to the invention.

Further described is a method for transducing cells *in vitro* or *in vivo,* the method comprising the steps of: a) co-expressing parvoviral helper and vector functions, wherein the helper function expresses a parvoviral structural protein according to this invention under conditions that enable parvovirus formation, b) isolating the parvovirus, and c) transducing cells with said parvovirus.

A method for producing a library of nucleic acids comprising a multiplicity of expressible nucleic acids according to this invention is given, comprising the steps of: a) providing a set of nucleic acids encoding each a parvoviral structural protein, b) inserting a library of inserts in frame into a plurality of nucleic acids at a position corresponding to that defined herein.

A further embodiment of the present invention is a medicament comprising at least one parvovirus structural protein according to this invention and/or a nucleic acid according to this invention, preferably at least one multimeric structure according to this invention. Preferably such medicament is used as a vaccine or as a gene transfer vector. The parvovirus structural protein according to this invention, the nucleic acid according to this invention, and the multimeric structure according to this invention may be defined as detailed above.

A further embodiment of the present invention is the use of at least one parvovirus structural protein according to this invention and/or a nucleic acid according to this invention, preferably at least one multimeric structure according to this invention for the manufacture of a vaccine or for use as a gene transfer vector.

As described earlier one preferred utility of the mutated structural proteins according to this invention is their use as a vaccine. Vaccine in the context of this invention means that an immune response, preferably a humoral immune response is generated after administration of the mutated structural protein. The vaccine is formulated to contain common salts, buffer, excipients and/or adjuvants.

The medicament of the present invention may further encompass pharmaceutically acceptable carriers and/or excipients. The pharmaceutically acceptable carriers and/or excipients useful in this invention are conventional and may include buffers, stabilizers, diluents, preservatives, and solubilizers. Remington's Pharmaceutical Sciences, by E. W. Martin, Mack Publishing Co., Easton, PA, 15th Edition (1975), describes compositions and formulations suitable for pharmaceutical delivery of the (poly)peptides herein disclosed. In general, the nature of the carrier or excipients will depend on the particular mode of administration being employed. For instance, parenteral formulations usually comprise injectable fluids that include pharmaceutically and physiologically acceptable fluids such as water, physiological saline, balanced salt solutions, aqueous dextrose, glycerol or the like as a vehicle. For solid compositions (e. g. powder, pill, tablet, or capsule forms), conventional non-toxic solid carriers can include, for example, pharmaceutical grades of mannitol, lactose, starch, or magnesium stearate. In addition to biologically neutral carriers, pharmaceutical compositions to be administered can contain minor amounts of non-toxic auxiliary substances, such as wetting or emulsifying agents, preservatives, and pH buffering agents and the like, for example sodium acetate or sorbitan monolaurate.

In a preferred embodiment the medicament further comprises an immunostimulatory substance such as an adjuvant. The adjuvant can be selected based on the method of administration and may include mineral oil-based adjuvants such as Freund's complete and incomplete adjuvant, Montanide incomplete Seppic adjuvant such as ISA, oil in water emulsion adjuvants such as the Ribi adjuvant system, syntax adjuvant formulation containing muramyl dipeptide, or aluminum salt adjuvants. Preferably, the adjuvant is a mineral oil-based adjuvant, especially ISA206 (SEPPIC, Paris, France), most preferably ISA51 (SEPPIC, Paris, France). In another preferred embodiment the parvovirus mutated structural protein is co-formulated with at least one suitable adjuvant such as CpG, Imidazoquinolines, MPL, MDP, MALP; flagellin, LPS, LTA, or cholera toxin or derivative thereof, HSP60, HSP70, HSP90, saponins, QS21, ISCOMs, CFA, SAF, MF59, adamantanes, aluminum hydroxide, aluminum phosphate or a cytokine.

In a more preferred embodiment the immunostimulatory substance is selected from the group comprising polycationic polymers, especially polycationic peptides such as polyarginine, immunostimulatory deoxynucleotides (ODNs), peptides containing at least two LysLeuLys motifs, especially KLKLLLLLKLK (SEQ ID NO: 126), neuroactive compounds, especially human growth hormone, alumn, adjuvants or combinations thereof. Preferably, the combination is either a polycationic polymer and immunostimulatory deoxynucleotides or of a peptide containing at least two LysLeuLys motifs and immunostimulatory deoxynucleotides. In a still more preferred embodiment the polycationic polymer is a polycationic peptide.

In an even more preferred embodiment of the invention the immunostimulatory substance is at least one immunostimulatory nucleic acid. Immunostimulatory nucleic acids are e.g. neutral or artificial CpG containing nucleic acids, short stretches of nucleic acids derived from non-vertebrates or in form of short oligonucleotides (ODNs) containing non-methylated cytosine-guanine dinucleotides (CpG) in a defined base context (e.g. as described in WO 96/02555). Alternatively, also nucleic acids based on inosine and cytidine as e.g. described in WO 01/93903, or deoxynucleic acids containing deoxy-inosine and/or deoxyuridine residues (described in WO 01/93905 and WO 02/095027) may preferably be used as immunostimulatory nucleic acids in the present invention. Preferably, mixtures of different immunostimulatory nucleic acids are used in the present invention. Additionally, the aforementioned polycationic compounds may be combined with any of the immunostimulatory nucleic acids as aforementioned. Preferably, such combinations are according to the ones described in WO 01/93905, WO 02/32451, WO 01/54720, WO 01/93903, WO 02/13857 and WO 02/095027 and the Australian patent application A 1924/2001.

In a further embodiment the parvovirus mutated structural protein of this invention is used for the manufacture of a vaccine for preventing or treating an autoimmune disease (e.g. diabetes type 1), a tumor disease (examples are: melanoma: e.g. HMW MAA, glioblastome multiforme: e.g. CA125, anti-IL13R, colon cancer: e.g. CA125 or anti-EGF(R), breast cancer: e.g. HER2/NEU, ovarian cancer: e.g. L1 adhesion molecule, B-cell lymphoma: e.g. CD20), an allergic disease (asthma, allergies such as allergic rhinitis, e.g. IgE), a metabolic disease (e.g. high cholesterol, intervention into the cholesterol metabolism, obesity, hypertension, e.g. CETP), an inflammatory disease (rheumatoid arthritis, Crohn's disease, psoriasis, e.g. IL-6, IL-17, TNF-α), a neurological disease (e.g. Alzheimer, e.g. β-amyloid) or to be used in ophthalmology.

Examples for autoimmune disease that are especially suitable for this invention are listed in Table 5.

**Table 5: Autoimmune diseases and suitable antibody targets/antigens**

| **Disease** | **antibody target/antigen** |
|---|---|
| Myasthenia gravis | Acetylcholine receptors |
| Graves's disease | Thyroid-stimulating hormone receptor |
| Thyroiditis | Thyroid |
| Insulin-resistant diabetes | Insulin receptor |
| Asthma | Beta-2 adrenergic receptors |
| Juvenile insulin-dependent diabetes | Pancreatic islet cells |
| Pernicious anemia | Gastric parietal cells |
| Addison's disease | Adrenal cells |
| Idiopathic hypoparathyroidism | Parathyroid cells |
| Spontaneous infertility | Sperm |
| Premature ovarian failure | Interstitial cells, corpus luteum cells |
| Pemphigus | Intercellular substance of skin |
| Primary biliary cirrhosis | Mitochondria |
| Autoimmune hemolytic anemia | Erythrocytes |
| Idiopathic thrombocytopenic purpura | Platelets |
| Idiopathic neutropenia | Neutrophils |
| Vitiligo | Melanocytes |
| Osteosclerosis and Meniere's disease | Type-II collagen |
| Chronic active hepatitis | Nuclei of hepatocytes |
| Goodpasture's syndrome | Basement membranes |
| Rheumatoid arthritis | Gamma globulin, virus-related antigens, IL-6, IL-17, TNF-α |
| Sjogren's syndrome | Nuclei and centromeres |
| Systemic lupus erythematosus | Nuclei, DNA, RNA, erythrocytes, etc. |
| Scleroderma | Nuclei and centromeres |
| Polymyositis | Nuclei, RNA |

Preferred autoimmune diseases are asthma, Juvenile insulin-dependent diabetes (diabetes type 1) and rheumatoid arthritis. Therefore, preferred antigens are the corresponding antigens of Beta-2 adrenergic receptors, pancreatic islet cells, Gamma globulin E, virus-related antigens, IL-6, IL-17 and TNF-α.

Examples for tumor diseases disease that are especially suitable for this invention are listed in Table 6.

**Table 6: Tumor diseases and suitable antibody targets/antigens**

| **Disease** | **antibody target/antigen** |
|---|---|
| Melanoma | HMW MAA (= high molecular weight melanoma associated antigen), BAGE, GAGE, MAGE-3, Melan A, MART-1, NY ESO, gp 100, tyrosinase |
| Colon cancer | CA125, EGFR |
| Gliobastome multiforme (GBM) | CA125, IL13R |
| Breast cancer | Her2/NEU |
| Ovarian cancer | L1 cell adhesion molecule |
| various cancers (e.g. for colon cancer, small lung cell carcinoma) | VEGF |
| B-cell lymphoma, e.g. Non-Hodgkin Lymphoma | CD20 |

Examples for allergic diseases are asthma, especially atopic asthma, and all types of allergies. The preferred target antigens for vaccination against allergic diseases are IgE, IL-9, and IL-13, especially IgE.

An example for a metabolic disease is a disorder in the cholesterol metabolism (e.g. atherosclerosis), a preferred target antigen is CETP.

Examples for inflammatory diseases that are especially suitable for this invention are listed in Table 7.

**Table 7: Inflammatory diseases and suitable antibody targets/antigens**

| **Disease** |
|---|
| COPD (chronic obstructive pulmonary disease) |
| OA (osteoarthritis) |
| Rheumatoid arthritis |
| Polymyalgia rheumatica |
| Gouty arthritis, Gout, Pseudogout |
| Atherosclerosis |
| Crohn's disease (inflammatory bowel disease) |
| Shoulder tendinitis, Bursitis |
| Colitis |
| Multiple Sclerosis |
| Systemic Lupus Erythematosus |
| Psoriasis |
| Juvenile diabetes |
| Type I diabetes mellitus (insulin-resistant diabetes) |
| Hypothyroidism |
| Chronic fatigue syndrome |
| Kawasaki's disease |
| Cardiavascular disease |
| Pericarditis |
| Lymph adenopathy |
| Raynaud's phenomenon |
| Sarcoidosis |
| Sjogren's syndrome |
| Spondyloarthropathies |
| Vasculitides |
| Scleroderma |
| Goodpasture's syndrome |
| Wegener's granulomatosis |
| temporal = Giant cell arteritis |
| Celiac disease |
| Addison's disease |
| Autoimmune hepatitis |
| Grave's disease |
| Graft-vs-host disease |

Preferred target antigens are TNF-α, CD20, IL-6 and IL-17.

Examples for diseases in ophthalmology are age-related macular degeneration (AMD) and diabetic retinopathy, a preferred target in these indications is VEGF.

Other preferred diseases are Alzheimer disease with the target antigen β-amyloid.

The parvovirus mutated structural protein according to this invention can be especially useful for manufacture of a medicament for breaking immune tolerance.

In the context of the uses of the invention, the features of the parvovirus mutated structural protein are as defined above.

In a preferred embodiment the disease is not an infectious disease, meaning a disease caused by a virus, a bacterium, a fungus or a eukaryotic parasite.

In a further embodiment parvovirus mutated structural protein is not used to make a vector that is used in gene therapy.

A preferred embodiment of the instant invention is a structural protein of a parvovirus as further defined above comprising an anti-idiotypic epi-/mimotope of an anti-IgE antibody, and/or an IgE epi-/mimotope. Preferred vaccines are the following:

### Vaccines for the treatment of asthma and allergic diseases

Atopic asthma and allergic rhinitis are caused by adverse immune responses, typified by IgE, against otherwise harmless environmental proteins, allergens. In sensitized individuals, allergen-specific IgE becomes localized in tissues by binding to the high-affinity receptor for IgE, FcεRI, expressed by mast cells in various tissues and basophils as well as eosinophils in the blood. Subsequent encounters with the allergen result in cross-linking of IgE/FcεRI, which triggers effector cell degranulation and the release of both preformed mediators (histamine, proteolytic enzymes, and proteoglycans) and *de novo* synthesized mediators (prostaglandin D₂, leukotrienes, and cytokines). Together, these mediators are responsible for the clinical manifestations of allergic reactions, including hay fever, asthma, and eczema, as well as life-threatening anaphylactic reactions. Standard therapy includes inhaled corticosteroids (ICS), Beclomethasone Dipropionate (BDP), long-acting β-agonists (LABA) and leukotriene receptor antagonists (LTRAs).

The receptor-binding region of human IgE was previously mapped to the N-terminal region of the CH3 domain (Helm et al., 1988, Helm et al., 1989). Site-directed mutagenesis studies to identify the amino acid residues directly involved in the interaction have been conducted on both IgE (Presta et al., 1994) and FcεRI (Cook et al., 1997). In addition, the crystal structure of the human IgE-FcεRIα complex was recently solved by Garman and colleagues (Garman et al., 2000). The amino acid regions that are involved in receptor binding are localized in three loops and spread over most of the Cε3 domain (Pro-364, Arg-365, Arg-408, Ser-411, Lys-415, Glu-452, Arg-465, and Met-469). Binding is mediated primarily by electrostatic interaction.

Anti-IgE therapy is based on antibodies which bind the receptor-binding target domain Cε3 region of IgE, thereby preventing the binding of IgE to the FcεRI receptor and, therefore, preventing sensitization of mast cells and basophils. However, even if 99% of free IgE was neutralized by the anti-IgE antibody, the therapy still would fail because the few remaining IgE molecules would be sufficient to sensitize the respective cells. Therapeutic efficacy is provided through additional actions: FcεRI expression is regulated by the level of free IgE, in a way that reduced levels of free IgE lead to lowered densities of FcεRI on basophils and mast cells and lowered sensitivities. And, anti-IgE may lead to down-regulation of IgE production by eliminating or down-regulating IgE-expressing B cells, perhaps by cross-linking membrane-bound IgE and causing apoptosis, anergy or most likely also by complement-mediated and cell-mediated cytolysis. The latter mechanism was, however, not found in clinical trials performed with Omalizumab. For this monoclonal antibody, reduction of IgE production from B-cells (plasma cells) mediated by lowered IgE levels was only observed in animal and *in-vitro* experiments.

Most of the therapeutic monoclonal antibodies in development can only bind and neutralize free IgE or IgE associated with B-cells. In contrast, FcεRI-bound IgE is not accessible for these anti-IgE antibodies. Anti-IgE antibodies directed against regions of the IgE molecule outside of the receptor binding region (such as the variable, antigen-binding domain of IgE referred to as the IgE idiotype), can bind to an IgE molecule while it is bound to its receptor. This results in cross-linking of receptor-bound IgE, causing an anaphylactic shock in animals treated systemically with such antibodies. Importantly, except for defense mechanisms against parasite infections, IgE seems to play no role in normal physiology and IgE-deficient people are healthy with no apparent sign of pathology (Levy and Chen, 1970).

Omalizumab (XOLAIR^{®}) is a humanized monoclonal anti-IgE antibody for passive immunization, and the first available/approved anti-IgE therapy on the market. A total of 7 phase III clinical trials were performed with this monoclonal anti-IgE antibody, which bind to the Cε3 region of IgE (for a review refer to (Bousquet et al., 2005)) without cross linking the FcεRI receptor. Omalizumab significantly reduced the rate of asthma exacerbations by 38% and the rate of total emergency visits by 47%. The efficacy of Omalizumab was unaffected by patient age, gender, baseline serum IgE or by 2- or 4-weekly dosing schedule, although benefit in absolute terms appeared to be greatest in patients with more severe asthma, defined by a lower value of percentage predicted forced expiratory volume in 1 s (FEV₁) at baseline.

As outlined before, one disadvantage of passive immunization with a monoclonal antibody is the requirement of infusions every 2-4 weeks with relatively high antibody doses making such therapies expensive. Therefore, alternative approaches are needed for the treatment of allergic diseases such as atopic allergies or asthma.

According to the present invention this problem is solved by a structural protein of a parvovirus comprising an anti-idiotypic epi-/mimotope of an anti-IgE antibody, and/or an IgE epi-/mimotope inserted at the insertion site I-453. Such structural proteins are preferably capable of forming virus-like particles. They harbor anti-idiotypic epi-/mimotopes of an anti-IgE antibody and/or IgE epi-/mimotopes on the surface of the capsid shell. Therefore the anti-idiotypic epi-/mimotopes of an anti-IgE antibody, respectively the IgE epi-/mimotopes are accessible to the humoral immune system. Such structural protein can be used as vaccines in patients in order to induce specifically an immune response against IgE, meaning antibodies that cross-react with IgE (anti-IgE antibodies), thereby preventing binding of IgE to its high affinity receptor FcεRI.

Especially preferred embodiments of the invention are structural proteins of parvoviruses, especially AAV, that contain IgE epitopes or mimotopes, preferably previously known epitopes or mimotopes inserted at insertion site I-453 that can be used as vaccines. In a preferred embodiment the B-cell epitope is a human epitope. Preferably it is inserted into I-453 and at least one further insertion site, preferably I-261, I-534, I-570, I-573 or I-587, especially into I-453 and I-587, preferably of AAV1, AAV2 or AAV-6.

For a lot of the publicly available therapeutic antibodies which can be used as target antibody for AAV selection, the epitopes are not known. To be able to compare the epitopes of the target antibodies and the antibodies induced in e.g. mice after vaccination, epitope mapping can be performed. For example, epitopes recognized by anti mouse or anti human IgE antibodies can be identified from arrays using overlapping peptide scans from the respective IgE spotted on nylon membranes. Preferred antibodies are those with a binding pattern similar to that of Omalizumab, which can be used for selection of mimotopes from the AAV capsid library. Epitopes recognized by antibodies induced in e.g. mice after vaccination can be identified from arrays spotted on glass slides. Cross-reactivity of anti human IgE antibodies or antibodies induced in mice after vaccination with the constant chain regions of other Ig's can be monitored in Westernblot experiments.

Especially preferred embodiments of the invention are structural proteins of parvoviruses, especially AAV, that contain IgE epitopes or mimotopes, preferably previously known epitopes or mimotopes.
Preferred IgE epitopes or mimotopes can first be identified as described by Rudolf, Stadler, Vogel and colleagues (Rudolf et al., 1998, Rudolf et al., 2000, Stadler et al., 1999, Vogel et al., 2000): one can develop so-called mimotope immunization vaccines based on peptide phage display libraries screened for particles recognizing BSW17, a mouse monoclonal anti-human IgE antibody. Peptide sequences best recognized by BSW17 are the preferred epitopes/mimotopes
EFCINHRGYWVCGD ("Rudolf" (Rudolf et al., 2000), (SEQ ID NO: 55) with G, W and V (underlined) being conserved among all sequences identified (the cysteine residues (in bold) mediate a circular form of the peptide via disulfide bridging), C4M' (Rudolf et al., 2000), and Kricek (Kricek et al., 1999).

Second, in the course of this invention previously unknown epitopes that are especially suitable for vaccination purposes against allergic diseases like asthma have been identified which are preferred IgE epitopes which are preferred IgE epitopes: Bind2, Flex, 3DEpi1, 3DEpi2, 3DEpi3, 3DEpi4, C4E, Wang-CS, Wang and C21.

The present invention further relates to novel IgE B-cell epitopes Bind2, Flex, 3DEpi1, 3DEpi2, 3DEpi3, 3DEpi4, C4E, Wang-CS, Wang, and C21 and/or to a functionally active variant thereof. A functionally active variant of these epitopes means a B-cell epitope which generates in a rabbit vaccination experiment according to example 8.6 a B-cell response in this case measurable as titer of specific antibodies binding to human IgE.

Such functionally active variants can either be single peptides or mixtures of single peptides consisting of peptide sequences of up to 40 amino acids, preferably up to 25 amino acids, more preferably 15 amino acids, especially 9 amino acids of the given sequence, or a fusion of such functionally active variant to a carrier. Such carrier is meant to be any molecule except for the naturally occurring IgE protein or part thereof (larger than the functionally active variant), preferably a parvoviral particle, but also a different virus- or bacteriophage particle, a polymer (e.g. LPH) or a fusion protein, capable of generating a B cell response (as defined above) against such functionally active variant. Such fusion to a carrier can i.e. be obtained by chemically linking the variant to the carrier or by genetically making fusion proteins or insertion variants.

These and similar sequences or parts therefore including or excluding the cysteine residues and flanking sequences can be introduced into positions I-453 and others of AAV VP. The corresponding AAV particles can be manufactured (initially as genome-containing infectious AAV), purified and characterized. Although AAV capsids have a different conformational structure than phages, the chance that a similar structure of the mimotope sequence EFCINHRGYWVCGD (SEQ ID NO: 55) is present on both, phages and AAV, is high due to the cysteine residues building up a loop structure of the peptide sequence. For linear epitopes such as Kricek (VNLTWSRASG, SEQ ID NO: 50), interchangeability should also be possible. If these AAV particles bind BSW17 (the anti-IgE antibody used for phage display), they can be used as an anti-IgE vaccine that can be used with and without co-formulation in a suitable adjuvant (as described herein).

Especially preferred embodiments of the invention are structural proteins of parvoviruses that contain IgE epi-/mimotopes that, once injected into an immunocompetent mammal, induce anti-IgE specific antibodies with therapeutic efficacy without cross-linking properties. Cross-linking properties means that in an immunocompetent mammal the generated anti-IgE antibodies are binding IgE molecules in a way that IgE/FcεRI binding is still possible. By such way, and if one antibody binds several IgE molecules at a time, the high-affinity FcεRI receptor is cross linked on effector cells leading to its degranulation. This would induce a systemic anaphylactic shock. On the other hand, the structural proteins of parvoviruses should be able to directly crosslink the respective B-cell receptor (binding the IgE epi-/mimotopes or the anti-idiotype epi-/mimotope of an anti-IgE antibody) to activate the corresponding B-cells and to induce anti-IgE antibody production independent of a T-cell response.

### Vaccines for the treatment of Alzheimer's disease

In general, misfolded proteins leading to a protein aggregation and, therefore, causing conformational diseases, are good candidate targets for an active immunization approach with AAV vaccines. Ideally, B-cell epitopes represented by misfolded proteins or protein aggregates only are chosen for presentation on AAV particles (for an overview, please refer to Uversky et al., 2006, especially table 1-1).

Especially preferred embodiments of the invention are structural proteins of parvoviruses, especially AAV, that contain β-amyloid epitopes or mimotopes at insertion site I-453, preferably known epitopes or mimotopes, that can be used for the treatment of Alzheimer disease. In the context of the present invention a B-cell epitope of β-amyloid was inserted into a parvovirus capsid and displayed on the surface of the capsid. In a preferred embodiment the B-cell epitope is a human epitope. Preferably it is inserted into I-453 and at least one further insertion site, preferably I-261, I-534, I-570, I-573 or I-587, especially into I-453 and I-587 of preferably AAV1, AAV2 or AAV6. In an especially preferred embodiment the B-cell epitope has the sequence DAEFRHDSG (SEQ ID NO: 65).

### Vaccines for the treatment of atherosclerosis

Atherosclerosis is a disease affecting arterial blood vessels. It is a chronic inflammatory response in the walls of arteries, in large part due to the accumulation of macrophage white blood cells and promoted by low density (especially small particle) lipoproteins (plasma proteins that carry cholesterol and triglycerides) without adequate removal of fats and cholesterol from the macrophages by functional high density lipoproteins (HDL). It is commonly referred to as a "hardening" or "furring" of the arteries. It is caused by the formation of multiple plaques within the arteries. There is a strong inverse relationship between the plasma concentration of cholesterol in HDLs (HDL-C) and the development of coronary heart disease (CHD). Plasma concentration of HDL-C is a powerful predictor of CHD. Although 33% of patients with CHD have low plasma levels of HDL-C as their primary lipid abnormality, there is currently no effective therapy for increasing the plasma concentration of HDL-C. Diet and moderate exercise are ineffective, statins afford only a modest 5% to 7% increase in HDL-C, and niacin has side effects and compliance profiles that limit its use.

One therapeutic approach that has been suggested for increasing plasma HDL-C concentrations is the inhibition of cholesteryl ester transfer protein (CETP) activity. CETP is a 74-kDa plasma glycoprotein that facilitates transfer of neutral lipids and phospholipids between lipoproteins and contributes to the regulation of plasma concentration of HDL-C. CETP functions in the plasma to lower the concentration of HDL-C by moving cholesteryl esters from HDLs to VLDLs and LDLs (Rittershaus et al., 2000).

Accordingly it is a further embodiment of the invention to provide structural proteins of parvoviruses, especially AAV, that contain CETP epitopes or mimotopes at insertion site I-453 that can be used as a vaccine for the treatment of atherosclerosis. Preferably the epitope or mimotope is inserted into I-453 and at least one further insertion site, preferably I-261, I-534, I-570, I-573 or I-587, especially into I-453 and I-587, preferably of AAV1, AAV2 or AAV-6. Suitable epitopes or mimotopes are the human CETP derived peptides hTP10, hTP11, hTP12, hTP13, hTP18 and hTP20, hRitsch-1, hRitsch-2, hRitsch-3, hCETP-intern and hCETP C-Term.

The present invention further relates to novel CETP B-cell epitopes hTP10, hTP11, hTP12, hTP13, hTP18, hTP20, hRitsch-1, hRitsch-2, hRitsch-3, hCETP-intern and hCETP C-Term and/or to a functionally active variant thereof.

### Vaccines for the treatment of tumor diseases

Antibody therapies such as HERCEPTIN^{®}, AVASTIN^{®}, ERBITUX^{®}, OMNITARG^{®}, RITUXAN^{®}, CAMPATH^{®}, ZEVALIN^{®}, MYLOTARG^{®}, BEXXAR^{®} or Panitumumab play an increasing role in fighting various types of tumor diseases. These antibodies specifically bind epitopes of factors causing uncontrolled cellular growth, such as growth factor receptors or growth factors. Accordingly, it is a further embodiment of this invention to provide structural proteins of parvoviruses, especially AAV, that contain epitopes of such factors causing uncontrolled cellular growth.

HER2/neu (also known as ErbB-2, ERBB2) is a protein giving higher aggressiveness in breast cancers. It is a member of the ErbB protein family, more commonly known as the epidermal growth factor receptor family. HER2/neu has also been designated as CD340. HER2/neu is notable for its role in the pathogenesis of breast cancer and as a target of treatment. It is a cell membrane surface-bound receptor tyrosine kinase and is normally involved in the signal transduction pathways leading to cell growth and differentiation. Approximately 25-35 percent of breast cancers have amplification of the HER2/neu gene or over expression of its protein product. Overexpression also occurs in other cancer such as ovarian cancer and stomach cancer. Clinically, HER2/neu is important as the target of the monoclonal antibody trastuzumab (marketed as HERCEPTIN^{®}).

As for an active vaccination approach, the epitope sequence QMWAPQWGPD (SEQ ID NO: 123) presented in a circular way has been shown to induce polyclonal antibodies with therapeutic effectiveness. Therefore, a Her2/NEU-AAV vaccine can be generated by insertion of this peptide into AAV using suitable adaptor sequences (Riemer et al., 2007).

Accordingly, especially preferred embodiments of the invention are structural proteins of parvoviruses, especially AAV, that contain epitopes or mimotopes of tumor antigens, preferably HER2/neu, especially the epitope described by Riemer, at I-453, preferably known epitopes or mimotopes. In the context of the present invention a B-cell epitope of HER2/neu can be inserted into a parvovirus capsid and displayed on the surface of the capsid. In a preferred embodiment the B-cell epitope is a human epitope. Preferably it is inserted into I-453 and at least one further insertion site, preferably I-261, I-534, I-570, I-573 or I-587, especially into I-453 and I-587, preferably of AAV1, AAV2 or AAV-6.

### Vaccines for the treatment of autoimmune diseases and chronic inflammatory diseases

Autoimmune diseases as well as inflammatory diseases arise from an overactive immune response of the body against substances and tissues normally present in the body. In other words, the body attacks its own cells.

Rheumatoid arthritis (RA) is an autoimmune disease which causes chronic inflammation of the joints, the tissue around the joints, as well as other organs in the body affecting 0.5-1.0 % of the population in the industrialized world. It commonly leads to significant disability and consequently to a significant reduction of quality of life. If not treated appropriately, RA leads to a reduction of life expectancy (Smolen and Steiner, 2003).

Psoriasis is a chronic inflammatory disease of the skin characterized by overgrowth of epidermal cells, angiogenesis, infiltration of immune cells, and increased production of cytokines. Similar activation of immune cells and increased production of cytokines is associated with autoimmune diseases and (chronic) inflammatory diseases as further listed below.

In order to limit or control such disease causing/related immune responses it has become an established therapeutic modality to neutralize cytokines involved in the pathogenesis of autoimmune and inflammatory diseases. Antibodies (infliximab, adalimumab) and a soluble receptor construct neutralizing the action of TNF-α (etanercept) have been established in the treatment of RA and other disease. Now there is evidence implicating several novel cytokines, including IL-32 and IL-17, in the pathogenesis of RA. In addition we assess the development of existing targets as they move towards clinical evaluation, particularly IL-1, IL-6, IL-15, IL-18 and the IL-12 superfamily (Asquith et al., 2007).

Accordingly, preferred embodiments of the invention are structural proteins of parvoviruses, especially AAV, that contain epitopes or mimotopes of cytokines, preferably of TNF-α, IL-6 and/or IL-17, preferably known epitopes or mimotopes, inserted at insertion site I-453 that can be used as vaccines for the treatment of autoimmune diseases and/or chronic inflammatory diseases, preferably rheumatoid arthritis and/or Crohn's disease. In a preferred embodiment the B-cell epitope is a human epitope. In a preferred embodiment the B-cell epitope is a human epitope or mimotope. Preferably it is inserted into I-453 and at least one further insertion site, preferably I-261, I-534, I-570, I-573 or I-587, especially into I-453 and I-587, preferably of AAV1, AAV2 or AAV-6.

Preferred B-cell epitopes are the human epitopes:
TNF-α V1, TNF-α V2, TNF-α V3, IL-17 V1, IL-17 V2, IL-6 V1, IL-6 V2

The present invention further relates to novel cytokine B-cell epitopes TNF-α V1, TNF-α V2, TNF-α V3, IL-17 V1, IL-17 V2, IL-6 V1 and IL-6 V2 and/or to a functionally active variant thereof.

### Vaccines for the treatment of infectious diseases

Blocking of viral infection by induction of auto-antibodies against the cellular receptor of the virus is a suggested mechanism of a preventive or therapeutic vaccination against viruses, preferably for viruses where classical vaccination attemps have failed like HIV using CCR5 as the target receptor (Chackerian, 1999).

Accordingly, preferred embodiments of the invention are structural proteins of parvoviruses, especially AAV, that contain epitopes or mimotopes of viral receptors, preferably of CCR5, preferably known epitopes or mimotopes, inserted at insertion site I-453 that can be used as vaccines for the treatment of such viral infection and associated diseases, preferably HIV infection/AIDS. In a preferred embodiment the B-cell epitope is a human epitope. In a preferred embodiment the B-cell epitope is a human epitope or mimotope. Preferably it is inserted into I-453 and at least one further insertion site, preferably I-261, I-534, I-570, I-573 or I-587, especially into I-453 and I-587, preferably of AAV1, AAV2 or AAV-6.

Preferred B-cell epitopes are HYAAAQWDFGNTMCQL (SEQ ID NO: 113), YAAQWDFGNTMCQ (SEQ ID NO: 114), RSQKEGLHYT (SEQ ID NO: 115) or a functionally active variant thereof

Accordingly, a further aspect of the present invention relates to a medicament of of the invention for the treatment and/or prevention of
a) an allergic disease and/or asthma whereas at insertion site I-453, and preferably at at least one further insertion site, more preferably at insertion site I-261, I-534, I-570, I-573 or I-587, especially I-453 and I-587, preferably of AAV1, AAV2 or AAV-6, the structural protein of a parvovirus comprises an anti-idiotypic epi-/mimotope of an anti-IgE antibody, and/or an IgE epi-/mimotope, particularly a mimotope of sequence of EFCINHRGYWVCGD (SEQ ID NO: 55), wish the first G, W and V being conserved and cysteine residues C mediating a circular form of the peptide via disulfide bridging, VNLTWSRASG (SEQ ID NO: 50) or INHRGYWV (SEQ ID NO: 95) or particularly an epitope selected from the group consisting of EKQRNGTLT (SEQ ID NO: 86), EDGQVMDVDLS (SEQ ID NO: 85), TYQCRVTHPHLPRALMR (SEQ ID NO: 87), RHSTTQPRKTKGSG (SEQ ID NO: 88), DSNPRGVSAYLSR (SEQ ID NO: 89), TITCLWDLAPSK (SEQ ID NO: 90), KTKGSGFFVF (SEQ ID NO: 91), THPHLPRALMRS (SEQ ID NO: 92), GETYQCRVTHPHLPRALMRSTTK (SEQ ID NO: 93, LPRALMRS (SEQ ID NO: 94) and a functionally active variant thereof;
b) Alzheimer's disease whereas at insertion site I-453, and preferably at at least one further insertion site, more preferably at insertion site I-261, I-534, I-570, I-573 or I-587, especially I-453 and I-587, preferably of AAV1, AAV2 or AAV-6, the structural protein of a parvovirus comprises a β-amyloid epitope or mimotope, particularly comprising or having the sequence DAEFRHDSG (SEQ ID NO: 65) or a functionally active variant thereof;
c) atherosclerosis whereas at insertion site I-453, and preferably at at least one further insertion site, more preferably at insertion site I-261, I-534, I-570, I-573 or I-587, especially I-453 and I-587, preferably of AAV1, AAV2 or AAV-6, the structural protein of a parvovirus comprises a CETP epitope or mimotope, particularly an epitope selected from the group consisting of PKTVSNLTESSSESVQS (SEQ ID NO: 102), SLMGDEFKAVLET (SEQ ID NO:103), QHSVAYTFEED (SEQ ID NO: 104), INPEIITRDG (SEQ ID NO: 105), DISLTGDPVITASYL (SEQ ID NO: 106), DISLTGDPVITA (SEQ ID NO: 107), DQSIDFEIDSA (SEQ ID NO: 108), KNVSEDLPLPTFSPTLLGDS (SEQ ID NO: 109), KNVSEDLPLPT (SEQ ID NO: 110), CDSGRVRTDAPD (SEQ ID NO: 111), FPEHLLVDFLQSLS (SEQ ID NO: 112) and a functionally active variant thereof;
d) a tumor disease whereas at insertion site I-453, and preferably at at least one further insertion site, more preferably at insertion site I-261, I-534, I-570, I-573 or I-587, especially I-453 and I-587, preferably of AAV1, AAV2 or AAV-6, the structural protein of a parvovirus comprises a tumor antigen, particularly a HER2/neu epitope or mimotope, especially the epitope QMWAPQWGPD (SEQ ID NO: 123) or a functionally active variant thereof; or
e) an autoimmune disease and/or a chronic inflammatory disease, preferably rheumatoid arthritis and/or Crohn's disease, whereas at insertion site I-453, and preferably at at least one further insertion site, more preferably at insertion site I-261, I-534, I-570, I-573 or I-587, especially I-453 and I-587, preferably of AAV1, AAV2 or AAV-6, the structural protein of a parvovirus comprises an epitope or mimotope of a cytokine, preferably of TNF-α, IL-6 and/or IL-17, especially an epitope selected from the group consisting of SSRTPSDKPVAHVVANPQAE (SEQ ID NO: 116), SRTPSDKPVAHVVANP (SEQ ID NO: 117), SSRTPSDKP (SEQ ID NO: 118), NADGNVDYHMNSVP (SEQ ID NO: 119), DGNVDYHMNSV (SEQ ID NO: 120), RSFKEFLQSSLRALRQ (SEQ ID NO: 121), FKEFLQSSLRA (SEQ ID NO: 122) or or a functionally active variant thereof.
f) an infectious disease, preferably HIV infection, whereas at insertion site I-453, and preferably at at least one further insertion site, more preferably at insertion site I-261, I-534, I-570, I-573 or I-587, especially I-453 and I-587, preferably of AAV1, AAV2 or AAV-6, the structural protein of a parvovirus comprises an epitope or mimotope of a viral receptor, preferably of CCR5, especially an epitope selected from the group consisting of HYAAAQWDFGNTMCQL (SEQ ID NO: 113), YAAQWDFGNTMCQ (SEQ ID NO: 114), RSQKEGLHYT (SEQ ID NO: 115) or a functionally active variant thereof.

### Figures

**Figure 1****: Schematic organization of the cap gene of AAV2**
**Figure 2****: Amino acid sequence alignment of various parvoviruses with a boxed insertion site I-453.**
For references for the aligned parvoviruses see Figure 3. Further parvoviruses can be found at *http:llwww.ncbi.nlm.nih.gov*/*ICNdb*/*Ictvlfs_parvo.htm#SubFamily1.* The corresponding amino acids to G₄₅₃ are boxed.
**Figure 3****: Amino acid sequence alignment of parvoviruses** (AAV1, AAV6, AAV2, AAV3b, AAV7, AAV8, AAV10, AAV4, AAV11, b-AAV, AAV5, GPV, B19, MVM, FPV and CPV)
Alignment was made using Multalin version 5.4.1, (Corpet, 1988). Symbol comparison table: blosum62, Gap weight: 12, Gap length weight: 2, Consensus levels: high=90% low=50%. Consensus symbols: ! is anyone of IV; $ is anyone of LM; % is anyone of FY; # is anyone of NDQEBZ. The amino acids corresponding to N₅₈₇ of AAV2 and the preferred insertion range for I-587 are boxed.

| ***Name*** | ***Length*** | ***Check*** | ***Weight*** | ***Seq. GP-No.*** |
|---|---|---|---|---|
| AAV1 | 799 | 4900 | 0.26 | 9632548 |
| AAV6 | 799 | 5176 | 0.26 | 2766607 |
| AAV2 | 799 | 2359 | 0.50 | 2906023 |
| AAV3b | 799 | 3639 | 0.50 | 2766610 |
| AAV7 | 799 | 132 | 0.50 | 22652859 |
| AAV8 | 799 | 3007 | 0.37 | 22652862 |
| AAV10 | 799 | 4671 | 0.37 | 48728343 |
| AAV4 | 799 | 7292 | 0.74 | 2337940 |
| AAV11 | 799 | 2546 | 0.74 | 48728346 |
| b-AAV | 799 | 5299 | 0.79 | 48696559 |
| AAV5 | 799 | 5950 | 1.34 | 91134730 |
| GPV | 799 | 3208 | 1.92 | 9628653 |
| B19 | 799 | 1920 | 2.45 | 4092542 |
| MVM | 799 | 332 | 2.05 | 2982110 |
| FPV | 799 | 7156 | 1.61 | 494031 |
| CPV | 799 | 7674 | 1.61 | 494746 |
| *consensus* | *799* | *6436* | *0.00* | |

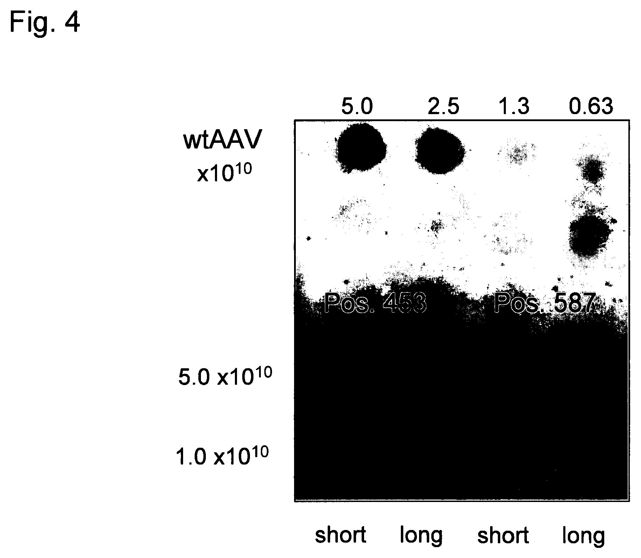

**Figure 4****: Interaction of an anti**-**CETP antibody with the respective CETP epitope inserted into the AAV2 capsid at position I-453 and I-587**
5.0 x10¹⁰ and 1.0 x10¹⁰ capsids of the variants AAV-CETP-453-short and AAV-CETP-453-long (for further details see example 1.2) were spotted onto a nitrocellulose membrane. In addition, 5.0 x10¹⁰ capsids of the variants AAV-CETP-587-short and AAV-CETP-587-long were spotted onto the same membrane. As negative control wtAAV was spotted ranging from 5.0 x10¹⁰ to 6.3 x10⁹ capsids per dot. The membrane was incubated with a polyclonal anti-CETP antibody directed against the CETP epitope inserted into the AAV capsid. Binding of the anti-CETP antibody to the spotted AAV variants was detected with an anti-rabbit IgG HRP (horse radish peroxidase) conjugate.
**Figure 5****: Detection of a β-amyloid epitope displayed by AAV2 at I-587 or I-453/I-587 by β-amyloid-specific antibody**
Serial dilutions (2x10¹¹ - 2x10⁸ capsids) of purified AAV particles displaying a β-amyloid epitope at I-587, I-453 and I-587, a CETP epitope at I-587 (as negative control) and 1 µg to 1 ng of the β-amyloid peptide (aa 1 - 42, BIOSOURCE, as positive control) were dotted on a membrane. The β-amyloid epitope was detected using an anti-β-amyloid mAb 6 x10¹⁰ (CHEMICON) and as secondary antibody a peroxidase-labeled anti-mouse IgG antibody (CALTAG). Signals were detected by chemiluminescence.

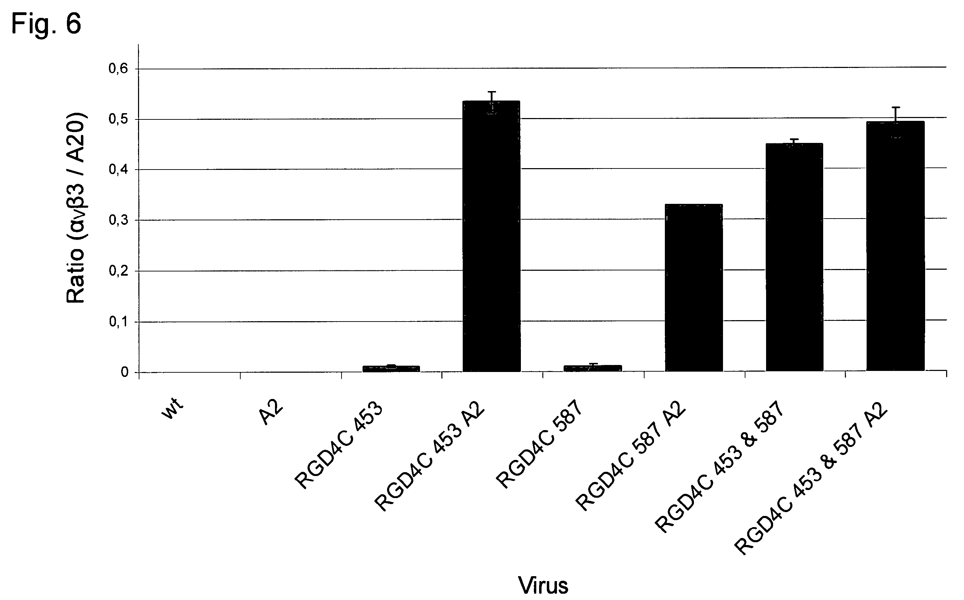

**Figure 6****: Normalization of AAV2 particles detected by monoclonal antibody A20**
ELISA plate was coated with A20 (75 ng/well, PROGEN). After blocking (PBS, 1% Milk Powder, 1% Tween) 1.00 x10¹⁰ particles were given per well. For detection of a functional RGD purified α_{V}β₃ integrin (100 ng/well, CHEMICON) was added to the plate and detected with anti-integrin α_{V} antibody (C-terminus/intracellular, Dil. 1:1000, CHEMICON). For quantification of viral particles in each well biotinylated A20 (250 ng/well, PROGEN) was used. The ratio "anti-integrin α_{V}": "A20-biot" was used for normalization of the amount of α_{V}β₃-binding to total particles.
**Figure 7****: HSPG independent transduction**
Chinese Hamster Ovarian cells with HSPG KO phenotype were transduced with 1,000 genomic particles per cell of the indicated mutant. Percentage of transduced cells was measured using flow cytometry.

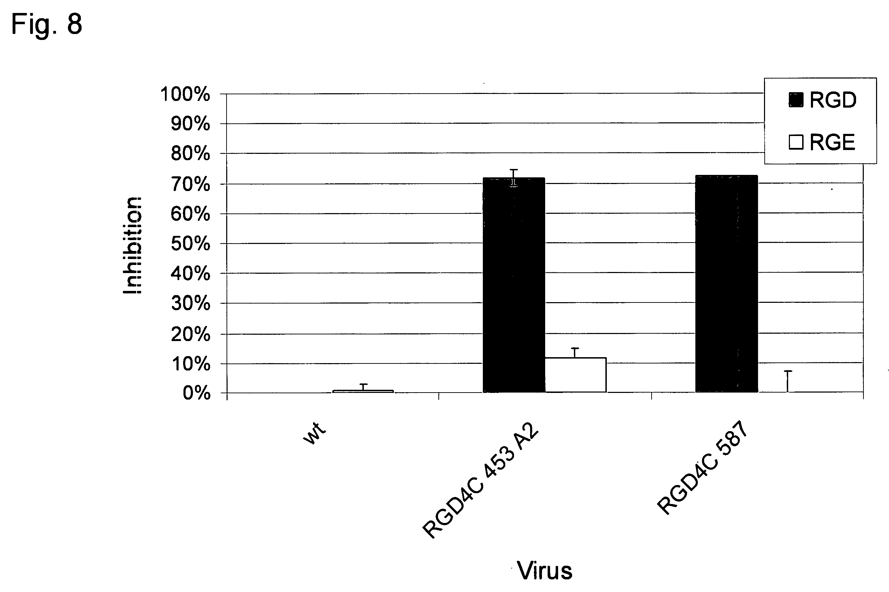

**Figure 8****: Competition of transduction with soluble peptide**
Transduction of CHO (HSPG KO) cells was performed with indicated virions 24 h after seeding the cells. Medium was removed. 1/2 vol. of medium was given to the well containing competition peptide (600 µM) or, as in the case of the controls, just medium. After 15 min of incubation at RT 1/2 medium containing virus was given to the cells. MOI = 1.000 genomic particles per cell. 48 h after transduction GFP expression was measured by flow cytometry.

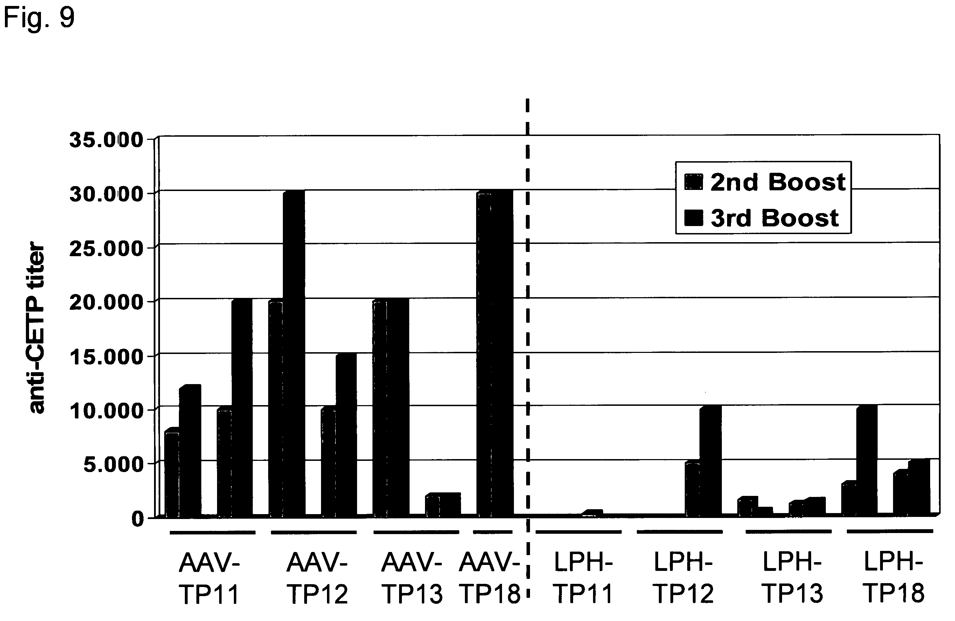

**Figure 9****: Induction of auto-antibodies by AAV-based vaccines vs. peptide based vaccines**
Rabbits (n=2) were immunized with the AAV-based CETP vaccines AAV-TP11, AAV-TP12, AAV-TP13 or AAV-TP18 s.c. in the presence of an adjuvant. AAV-based CETP vaccines were compared with the corresponding peptide vaccines containing the same epitope coupled to LPH (Limulus polyphemus hemocyanine). The titer of CETP auto-antibodies in the immune sera was measured after the 2^{nd} (gray) and 3^{rd} (black) boost immunization.

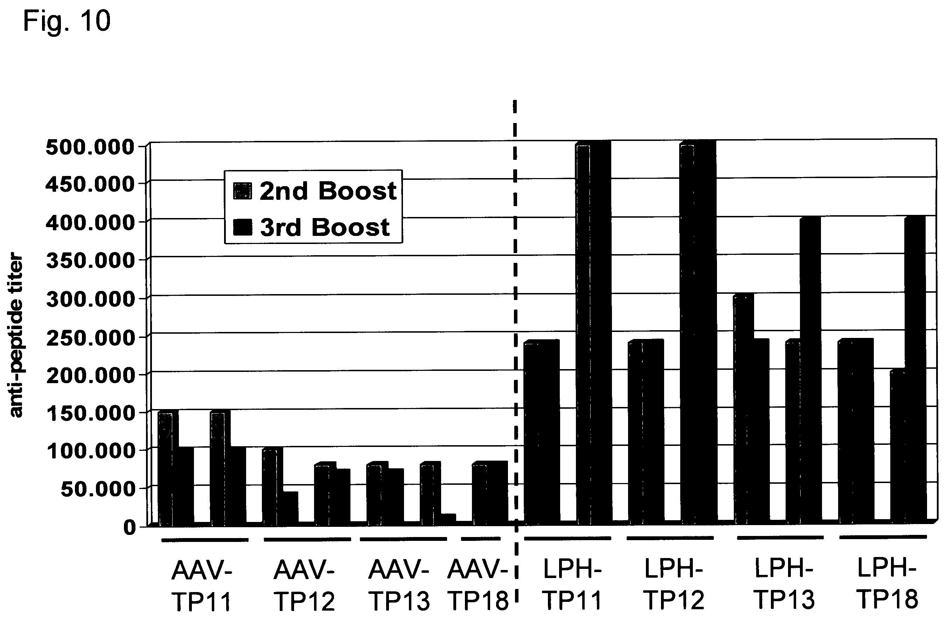

**Figure 10****: Induction of auto-antibodies by AAV-based vaccines vs. peptide based vaccines**
Rabbits (n=2) were immunized with the AAV-based CETP vaccines AAV-TP11, AAV-TP12, AAV-TP13, or AAV-TP18 s.c. in the presence of an adjuvant. AAV-based CETP vaccines were compared with the corresponding peptide vaccines containing the same epitope coupled to LPH (Limulus polyphemus hemocyanine). The titer of auto-antibodies directed against the epitope (linear peptide) in the immune sera was measured after the 2^{nd} (gray) and 3^{rd} (black) boost immunization.

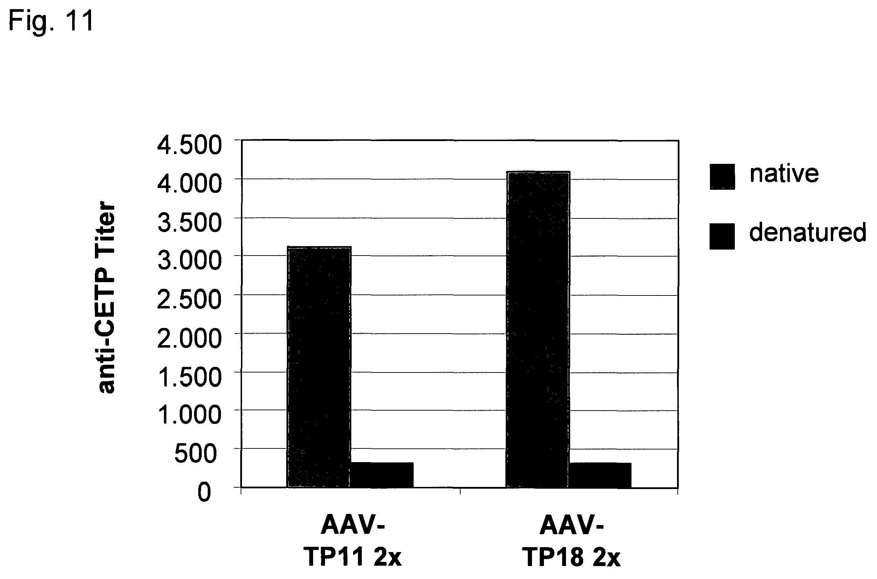

**Figure 11****: Induction of auto-antibodies by native and heat-denatured AAV-based vaccines**
Rabbits (n=4) were immunized with native (gray) or heat-denatured (black) AAV-based CETP vaccines AAV-TP11 2x or AAV-TP18 2x s.c. in the presence of an adjuvant. The titer of CETP auto-antibodies in the immune sera was measured after the 1^{st} boost immunization.

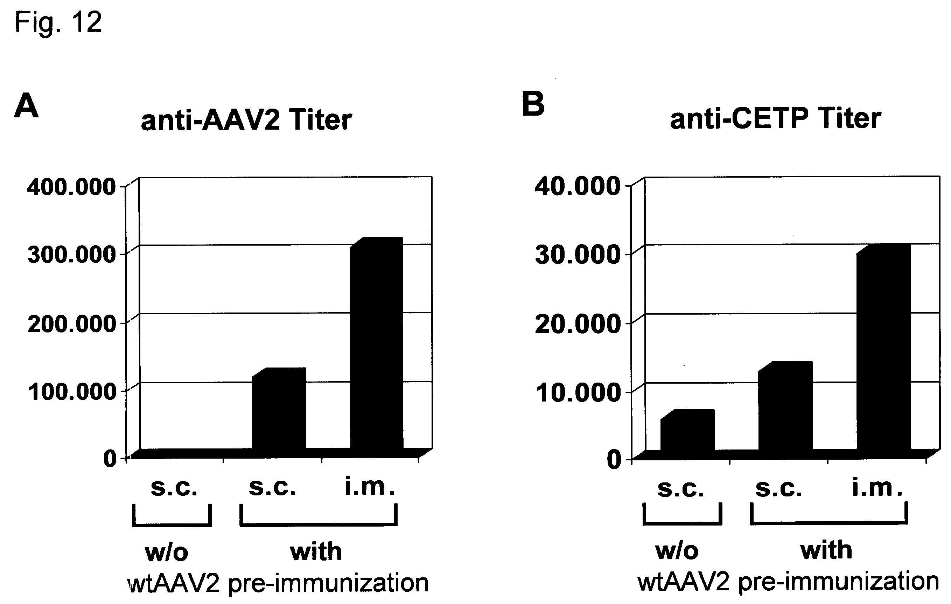

**Figure 12****: Evaluation of the impact of anti-AAV2 antibodies on immunization with AAV2-based vaccines**
(A) To evaluate the impact of anti-AAV2 antibodies on the immunization success of AAV2-based vaccines, rabbits (n=3) were pre-immunized by two applications of 4.5 µg wtAA2 (s.c. or i.m.). Serum was analyzed two weeks after 2^{nd} application for the level of anti-AAV2 antibodies. A control group (n=2) was not pre-immunized with wtAAV2.
(B) Following pre-immunization with wtAAV2 rabbits were vaccinated with the AAV2-based vaccine AAV-TP18 (7.2 µg per application). The vaccine was administered s.c. or i.m. in the presence of an adjuvant. Sera were analyzed two weeks after the 1^{st} boost vaccination for the level of CETP auto-antibodies. Results were compared to vaccination (s.c.) of animals without wtAAV2 pre-immunization.

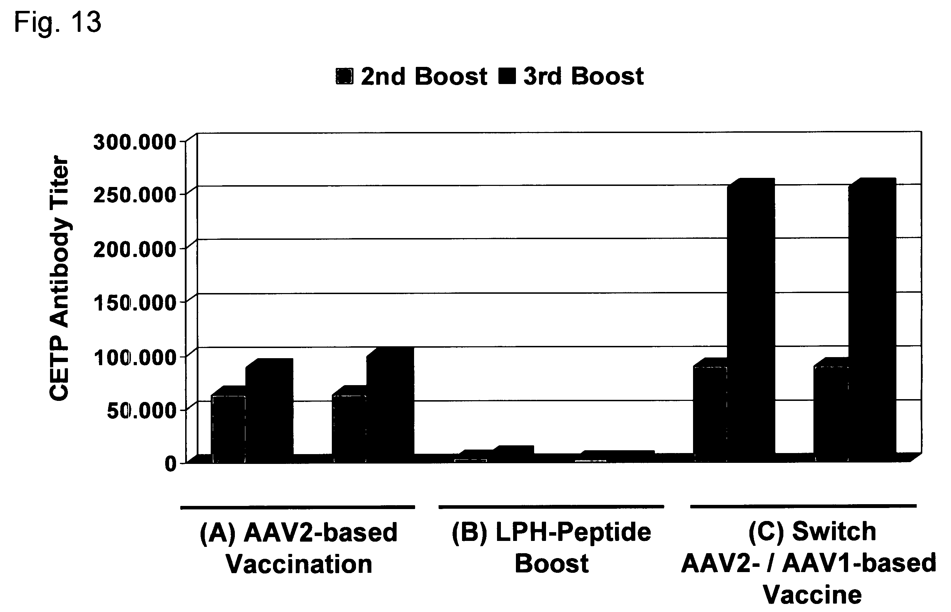

**Figure 13****: Evaluation of different prime / boost regimens for AAV-based vaccines**
Three different prime/boost regimens were evaluated. Group A received one prime and three boost applications of AAV2-CETin-2x (AAV2-based vaccination). Group B received one prime and one boost immunization with AAV2-CETin-2x followed by two boost immunizations with the LPH-coupled CETP-intern peptide (LPH-peptide boost). Group C received one prime and one boost immunization with AAV2-CETln-2x followed by two boost immunizations with AAV1-CETin (switch AAV2-/AAV1-based vaccine). Immune sera were analyzed for anti-CETP-reactivity (CETP auto-antibody titer) two weeks after the 2^{nd} (gray) and 3^{rd} boost (black) immunization.

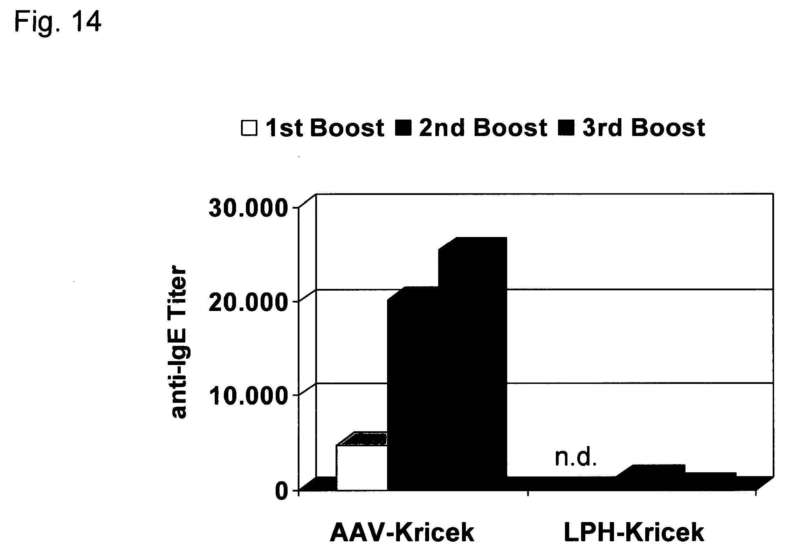

**Figure 14****: Vaccination against human IgE**
Rabbits (n=2) were immunized with AAV2 particles carrying a human IgE epitope ("Kricek") at position I-587. In a control group rabbits were immunized with the same IgE epitope coupled to LPH (LPH-Kricek). Immune sera were analyzed for anti-IgE reactivity two weeks after the 1^{st} (white), 2^{nd} (gray) and 3^{rd} (black) boost immunization. n. d.: not determined.

### Examples

The following examples exemplify the invention for AAV, especially for AAV2. Due to the general similarities within the structures of the adeno-associated viruses and other parvoviruses the invention can be easily transferred to other parvoviruses.

### 1. Generation of modified AAV variants by insertion of epi- or mimotope sequences at position I-453 of the AAV capsid by genetic manipulation

The approach described below is used for the insertion of epi- or mimotopes into the AAV capsid at position I-453 using a defined cloning strategy. This strategy includes the generation of a Notl and Ascl restriction site within the cap gene by site-directed mutagenesis that allows the insertion of DNA fragments encoding epi- or mimotope at position I-453 of AAV cap flanked by a short or long alanine adaptor sequence.

### 1.1. Creation of singular Notl and Ascl restriction sites in vector pCI-VP2

The vector pCI-VP2 was created by PCR amplification of the AAV2 VP-2 gene mutating the minor ACG start codon into an ATG and cloning of the respective PCR product into the polylinker sequence of pCI (PROMEGA). The Notl site at nucleotide 18 of pCI-VP2 (nucleotide 1099 of pCI) was destroyed by site directed mutagenesis using the primers
mutashe-3 5'-GAG TCG ACC CGG GCA GCC GCT TCG AGC-3'
   (SED ID NO: 44)
   and
mutashe-4 5'-GCT CGA AGC GGC TGC CCG GGT CGA CTC-3'
   (SEQ ID NO: 45)
together with the QUICKCHANGE II SITE-DIRECTED MUTAGENESIS KIT (STRATAGENE) according to the instructions of the manufacturer. The resulting vector was referred to as pCI-VP2-ΔNot18. To introduce a Notl and Ascl restriction site that allows for the cloning of epitope or mimotope sequences at position I-453 of the AAV capsid, the vector pCI-VP2-ΔNot18 was modified by site directed mutagenesis using the primers
mutashe-5 5'-CA AAC ACT CCA AGT GGA GGG CGC GCC GCT ACC
   ACC ACG CAG TC-3' (SEQ ID NO: 46)
   and
mutashe-6 5'-GA CTG CGT GGT GGT AGC GGC GCG CCC TCC ACT
   TGG AGT GTT TG-3' (SEQ ID NO: 47)
to introduce the Ascl site first as well as the primers
mutashe-7 5'-CA AAC ACT CCA AGT GGA GCG GCC GCA GGG CGC
   GCC GCT AC-3' (SEQ ID NO: 48)
   and
mutashe-8 5'-GT AGC GGC GCG CCC TGC GGC CGC TCC ACT TGG
   AGT GTT TG-3' (SEQ ID NO: 49)
to introduce the Notl site subsequently.
Site specific mutagenesis was performed using the QUIKCHANGE II SITE-DIRECTED MUTAGENESIS KIT (STRATAGENE) according to the instructions of the manufacturer. The resulting vector is referred to as pCIVP2-I453-Notl-Ascl.

### 1.2. Cloning of epitope or mimotope sequences into pCIVP2-1453-Notl-Ascl

For cloning of epi- or mimotope sequences into pCIVP2-I453-Notl-Ascl, forward and reverse oligonucleotides were designed that encode the respective epi- or mimotope sequences with a short or long alanine adaptor sequence and contain a 5'-site extension. The 5'-site extension of the oligonucleotides was designed so that annealing of the forward and reverse oligonucleotides results in a dsDNA with 5'-site and 3'-site overhangs compatible with overhangs generated by Notl and Asci restriction of the plasmid pCIVP2-I453-Notl-Ascl. The sequences of the oligonucleotides and the respective epi- or mimotope sequences including the alanine adaptors are summarized in Table 8. Each of the inserted epi- or mimotope sequences is flanked by a short or long adaptor according to the following scheme (Xₙ represents the mimotope or epitope sequence):
short Ala adaptor: (A)₃-Xₙ-R-(A)₂ (A short)
long Ala adaptor: (A)₅-Xₙ-(A)₂-R-(A)₂ (A long)
long Gly adaptor: (A)₂-(G)₅-Xₙ-(G)₅-R-(A)₂ (G long)

**Table 8: Oligonucleotides used for cloning of epi- or mimotope sequences at position I-453**

| **Name / Peptide Seq.** | **Type** | **Forward Oligonucleotide** | **Reverse Oligonucleotide** | **Adaptor** |
|---|---|---|---|---|
| **Kricek** VNLTWSRASG SEQ ID NO: 50 | Epitope | 5'-ggccgcagtgaacctgac ctggagcagagcctccggc-3' SEQ ID NO: 51 | 5'-cgcggccggaggctctgct ccaggtcaggttcactgc-3' SEQ ID NO: 52 | A short |
| | | 5'-ggccgcagccgcagtgaa cctgacctggagcagagcctcc ggcgcggca-3' SEQ ID NO: 53 | 5'-cgcgtgccgcgccggag gctctgctccaggtcaggttcact gcggctgc-3' SEQ ID NO: 54 | A long |
| **Rudolf** EFCINHRGYWVCGD SEQ ID NO: 55 | Mimotope | 5'-ggccgcagaattctgcata aaccacaggggatactgggtgt gcggagac-3' SEQ ID NO: 56 | 5'- cgcggtctccgcacaccc agtatcccctgtggtttatgcaga attctgc -3' SEQ ID NO: 57 | A short |
| | | 5'-ggccgcagccgcagaattc tgcataaaccacaggggatact gggtgtgcggagacgcggca- 3' SEQ ID NO: 58 | 5'-cgcgtgccgcgtctccgca cacccagtatcccctgtggtttat gcagaattctgcggctgc-3' SEQ ID NO: 59 | A long |
| **CETP-intern** CDAGSVRTNAPD SEQ ID NO: 60 | Epitope | 5'-ggccgcatgcgacgctgg cagtgtgcgcaccaatgcacca gac -3' SEQ ID NO: 61 | 5'-cgcggtctggtgcattggtg cgcacactgccagcgtcgca tgc -3' SEQ ID NO: 62 | A short |
| | | 5'-ggccgcagccgcatgcga cgctggcagtgtgcgcaccaat gcaccagacgcggca -3' SEQ ID NO: 63 | 5'-cgcgtgccgcgtctggtgc attggtgcgcacactgccagc gtcgcatgcggctgc -3' SEQ ID NO: 64 | A long |
| **β-amyloid** DAEFRHDSG SEO ID NO: 65 | Epitope | 5'-ggccggcggaggcggtgg ggacgccgaattcagacacga cagcggcggaggcggtggag gg-3' SEQ ID NO: 66 | 5'-cgcgccctccaccgcctcc gccgctgtcgtgtctgaattcgg cgtccccaccgcctccgcc-3' SEQ ID NO: 67 | G long |

To anneal the oligonucleotides 50.0 µg of the forward oligonucleotide and 50.0 µg of the reverse oligonucleotide were mixed in a total volume of 200 µl 1x PCR-Buffer (QIAGEN) and incubated for 3 min at 95°C in a thermomixer. After 3 min at 95°C the thermomixer was switched off and the tubes were left in the incubator for an additional 2 h to allow annealing of the oligonucleotides during the cooling down of the incubator. To clone the annealed oligonucleotides into pCIVP2-I453-Notl-Ascl the vector was linearized by restriction with Notl and Ascl and the cloning reaction was performed using the Rapid DNA Ligation Kit (Roche). Briefly, the annealed oligonucleotides were diluted 10-fold in 1x DNA Dilution Buffer and incubated for 5 min at 50°C. 100 ng of these annealed oligonucleotides and 50 ng of the linearized vector pCIVP2-I453-Notl-Ascl were used in the ligation reaction, which was performed according to the instructions of the manufacturer of the Rapid DNA Ligation Kit (Roche). *E. coli* XL1 blue were transformed with an aliquot of the ligation reaction and plated on LB-Amp agar plates. Plasmids were prepared according to standard procedures and were analyzed by sequencing.

### 1.3. Subcloning of epitope or mimotope sequences from pCIVP2 into pUCAV2

For production of recombinant AAV particles carrying a mimo- or epitope insertion at position I-453 the BsiWI/Xmal fragment of pCI-VP2-453-Notl-Ascl encoding a VP-2 fragment containing the epitope or mimotope at position I-453 was sub-cloned into pUCAV2, which was modified as described below.

Cloning of vector pUCAV2 is described in detail in US 6,846,665. Basically, this vector contains the complete AAV genome (Bgl II fragment) derived from pAV2 (Laughlin et al., 1983) cloned into BamHI of pUC19.

pUCAV2 is used for production of the modified AAV particles. Since there are three XmaI sites in pUCAV2 it is not possible to use the XmaI site of pUCAV2 for subcloning of the BsiWI/Xmal fragment of pCI-VP2-453-Notl-Ascl. Therefore, a new Agel site was introduced into pUCAV2 that is compatible with XmaI and is not present in pUCAV2. To introduce the Agel site pUCAV2 was linearized by SnaBI, dephosphorylated and subsequently blunt-end ligated with a short ds oligonucleotide adaptor containing an internal Agel site. The ds oligonucleotide adaptor was generated by annealing of a
sense 5'-GTA GCC CTG GAA ACT AGA ACC GGT GCC TGC GCC-3'
   (SEQ ID NO: 68)
   and
anti-sense 5'-GGG GCA GGC ACC GGT TCT AGT TTC CAG GGC TAC-3'
   (SEQ ID NO: 69)
oligonucleotide containing an Agel restriction site as described above. The annealed oligonucleotides were ligated with the SnaBI linearized, dephosphorylated pUCAV2 using the Rapid DNA Ligation Kit (Roche) as described above. The resulting vector is referred to as pUCAV2-Agel. pUCAV2-Agel was linearized with BsiWI and Agel and ligated with the BsiWI/Xmal fragment of pCI-VP2-453-Notl-Ascl encoding the VP-2 fragment containing the respective epitope or mimotope at position I-453.

### 2. Generation of modified AAV variants by insertion of epitope sequences at position I-587 of the AAV capsid by genetic manipulation

The approach described below is used for the insertion of epi- or mimotopes into the AAV capsid at position I-587 using a defined cloning strategy. This strategy includes the generation of a Notl and Ascl restriction site within the cap gene by site-directed mutagenesis that allows the insertion of DNA fragments encoding epi- or mimotope at position I-587 of AAV cap flanked by a short or long alanine adaptor sequence.

### 2.1. Creation of singular Notl and Ascl restriction sites in vector pCI-VP2 at insertion site I-587

The vector pCI-VP2 was created by PCR amplification of the AAV2 VP-2 gene mutating the minor ACG start codon into an ATG and cloning of the respective PCR product into the polylinker sequence of pCI (PROMEGA). The Notl site at nucleotide 18 of pCI-VP2 (nucleotide 1099 of pCI) was destroyed by site-directed mutagenesis as described above. The resulting vector was referred to as pCI-VP2-ΔNot18. To introduce a *Not*I and *Asc*l restriction site that allows for the cloning of epitope or mimotopes sequences at position I-587 of the AAV capsid, the vector pCI-VP2-ANot18 was modified by site-directed mutagenesis using the primers
pCI-VP2-ΔNot-I587-for 5' -CC AAC CTC CAG AGA GGC AAC GCG GCC
   GCA AGG CGC GCC AAG CAG CTA CCG CAG-3'
   (SEQ ID NO: 70)
   and
pCI-VP2-ΔNot-I587-reV 5'-CTG CGG TAG CTG CTT GGC GCG CC TT GCG
   GCC GCG TTG CCT CTC TGG AGG TTG G-3'.
   (SEQ ID NO: 71)
Site-specific mutagenesis was performed using the QUIKCHANGE II SITE-DIRECTED MUTAGENESIS KIT (STRATAGENE) according to the instructions of the manufacturer. The resulting vector is referred to as pCIVP2-I587-NotI-AscI.

### 2.2. Cloning of epitope sequences into pCIVP2-I587-NotI-AscI

For cloning of a CETP epitope sequence into pCIVP2-I587-NotI-AscI sense and anti-sense oligonucleotides were designed that encode the CETP epitope with a short or long alanine adaptor sequence and contain 5'-site extensions. The 5'-site extension of the oligonucleotides was designed so that annealing of the sense and anti-sense oligonucleotides results in a dsDNA with 5'-site and 3'-site overhangs compatible with overhangs generated by NotI and AscI restriction of the plasmid pCIVP2-I587-NotI-AscI. The sequences of the oligonucleotides and the encoded CETP epitope sequence including the alanine adaptors are summarized in Table 9. The inserted CETP epitope sequence is flanked by a short or long alanine adaptor according to the following scheme (Xₙ represents the CETP epitope sequence):
short Ala adaptor: (A)₃-Xₙ-(A)₂ (A short)
long Ala adaptor: (A)₅-Xₙ-(A)₅ (A long)
long Gly adaptor: (A)₃-(G)₅-Xₙ-(G)₅-(A)₂ (G long)

**Table 9: Oligonucleotides used for cloning of epitope sequences at position I-587**

| **Name / Peptide Seq.** | **Type** | **sense Oligonucleotide** | **anti-sense Oligonucleotide** | **Adaptor** |
|---|---|---|---|---|
| **CETP-intern** CDAGSVRTNAPD SEQ ID NO: 60 | Epitope | 5' GGCCGCATGCGACG CTGGCAGTGTGCGCACC AATGCACCAGACGCGG 3' SEQ ID NO: 72 | 5' CGCGCCGCGTCTGGT GCATTGGTGCGCACACTG CCAGCGTCGCATGC 3' SEQ ID NO: 73 | A short |
| | | 5' GGCCGCAGCGGCGT GCGACGCTGGCAGTGTG CGCACCAATGCACCAGA CGCGGCGGCGGCGG 3' SEQ ID NO: 74 | 5' CGCGCCGCCGCCGCC GCGTCTGGTGCATTGGTG CGCACACTGCCAGCGTCG CACGCCGCTGC 3' SEQ ID NO: 75 | A long |
| **β-amyloid** DAEFRHDSG SEQ ID NO: 65 | Epitope | 5′ GGCCGCAGGCGGAG GGGGAGGCGACGCCGAG TTCAGACACGACAG CGGCGGCGGAGGGGGAG GCGCGG 3′ SEQ ID NO: 76 | 5′ CGCGCCGCGCCTCCC CCTCCGCCGCCGCTGTCG TGTCTGAACTCGGCGTCG CCTCCCCCTCCGCCTGC 3′ SEQ ID NO: 77 | G long |

The sense and anti-sense oligonucleotides were annealed as described above (1.2). To clone the annealed oligonucleotides into pCIVP2-I587-NotI-AscI the vector was linearized by restriction with NotI and AscI and the cloning reaction was performed using the Rapid DNA Ligation Kit (Roche). Briefly, the annealed oligonucleotides were diluted 10-fold in 1x DNA Dilution Buffer and incubated for 5 min at 50°C. 100 ng of these annealed oligonucleotides and 50 ng of the linearized vector pCIVP2-I587-NotI-AscI were used in the ligation reaction, which was performed according to the instructions of the manufacturer of the Rapid DNA Ligation Kit (Roche). *E. coli* XL1 blue were transformed with an aliquot of the ligation reaction and plated on LB-Amp agar plates. Plasmids were prepared according to standard procedures and were analyzed by sequencing.

### 2.3. Subcloning of epitopes from pCIVP2 into pUCAV2 at position I-587

For production of recombinant AAV particles carrying the CETP epitope insertion at position I-587 the BsiWI/XmaI fragment of pCI-VP2-587-NotI-AscI encoding a VP-2 fragment containing the epitope or mimotope at position I-587 was sub-cloned into pUCAV2, which was modified as described above (1.3). The modified pUCAV2 is referred to as pUCAV2-Agel. pUCAV2-Agel was linearized with BsiWI and Agel and ligated with the BsiWI/XmaI fragment of pCI-VP2-587-NotI-AscI encoding the VP-2 fragment containing the CETP epitope at position I-587.

### 3. Production and purification of AAV variants

### 3.1. AdV helper plasmid

An AdV helper plasmid encoding AdV E2, E4 and VAI-VAII was used for AAV manufacturing in HEK 293-T cells. The helper plasmid pUCAdvE2/E4-VAI-VAII was constructed by subcloning of the BamHI restriction fragment encoding the adenovirus E2 and E4-ORF6 from pAdEasy-1 into the site BamHI site of pUC19. The resulting plasmid is referred to as pUCAdVE2/E4. The VAI-VAII fragment from pAdvantage was amplified by PCR using the primers 
XbaI-VAI-780-3′: 5′-TCT AGA GGG CAC TCT TCC GTG GTC TGG TGG-
3′ SEQ ID NO: 78
 and 
XbaI-VAII-1200-5′ 5′-TCT AGA GCA AAA AAG GGG CTC GTC CCT GTT
TCC-3′ SEQ ID NO: 79,
 cloned into pTOPO and then subcloned into the XbaI site of pUCAdvE2/E4. The resulting plasmid pUCAdvE2/E4-VAI-VAII was evaluated in co-transfection experiments for production of AAV as described below. AAV particle formation was analyzed using the A20 ELISA.

### 3.2. Production of AAV variants by co-transfection of HEK 293-T-cells

For production of AAV particles HEK 293-T cells were co-transfected with the vector plasmid pUCAV2 containing the subcloned epitope (in I-453 and/or I-587) and the helper plasmid pUCAdV (described above).

For co-transfection 7.5x10⁶ 293-T cells were seeded into each 015 cm cell culture plate in a total volume of 17.5 ml medium (DMEM containing 10% FCS, 5 mM L-Gln and ABAM) 24h before transfection and cultivated at 37°C, 5% CO₂ in a humidified atmosphere. For co-transfection of the vector plasmid pUCAV2 containing the epitope (in I-453 or I-587) and pUCAdV a molar ratio of the plasmids of 1:1 was chosen. For Calcium phosphate transfection of one culture plate with 293-T cells using the Calcium phosphate transfection protocol as disclosed in US 2004/0053410, 12.0µg pUCAV2 (containing the epitope in I-453 or I-587) and 24.0 µg pUCAdV were mixed in 875µl 270mM CaCl₂. In brief, 875µl 2x BBS (50mM BES (pH 6.95), 280mM NaCl and 1.5mM Na₂HPO₄) was added to the mixture and the resulting solution was carefully mixed by pipetting. The solution was incubated for 20 min at room temperature and then added drop-wise to the cell culture plate. Cells were incubated at 35°C, 3% CO₂ in a humidified atmosphere for 18h. After 18h at 35°C and 3% CO₂ cells were cultivated for an additional 3d at 37°C, 5% CO₂ in a humidified atmosphere.

293-T cells were harvested with a cell lifter, transferred into 50 ml plastic tubes (Falcon) and centrifuged at 3000g at 4°C for 10min. The cell pellet was resuspended in 1.0 ml lysis buffer (150mM NaCl, 50mM Tris, pH 8.5) and objected to three rounds of freeze and thaw cycles. The lysate was treated with 100 U/ml benzonase (MERCK) at 37°C for 30min. The cell lysate was cleared by two centrifugation steps (3700g, 4°C, 20min) and the AAV-containing supernatant was used for further purification.

The AAV capsid titer of the lysate was determined using a commercially available ELISA (AAV Titration ELISA, PROGEN).

### 3.3. Purification of AAV particles by density gradient centrifugation using iodixanol

AAV particles were purified by iodixanol gradient centrifugation. The virus-containing cell lysate was cleared by centrifugation (3700 g, 4°C, 20 min) and the cleared lysate was transferred to QICKSEAL ultracentrifugation tubes (26x 77mm, BECKMAN). Iodixanol solutions (SIGMA) of different concentrations were layered beneath the virus containing lysate. By this an Iodixanol gradient was created composed of 6.0 ml 60% on the bottom, 5.0 ml 40%, 6.0 ml 25% and 9.0 ml 15% Iodixanol with the virus solution on top. The gradient was spinned in an ultracentrifuge at 416.000g for 1h at 18°C. The 40% phase containing the AAV particles was then extracted with a cannula by puncturing the tube underneath the 40% phase and allowing the solution to drip into a collecting tube until the 25% phase was reached. The AAV capsid titer of the 40% phase was determined using a commercially available ELISA (AAV Titration ELISA, PROGEN).

### 4. AAV variants carrying a CETP Epitope at position I-453 or I-587 of the AAV2 capsid

An epitope (CDAGSVRTNAPD; SEQ ID NO: 60) of rabbit CETP (cholesteryl ester transfer protein) was introduced at position I-453 or I-587 of AAV2 by the cloning approaches described above. In both insertion sites the epitope is flanked by a short or long alanine adaptor. For production of AAV variants HEK 293-T cells were co-transfected with the vector plasmid pUCAV2 containing the subcloned CETP epitope sequence at position I-453 or I-587, and the helper plasmid pUCAdV as described above. AAV variants were purified by Iodixanol gradient centrifugation as described above.

The AAV capsid variants AAV-CETP-453-short, AAV-CETP-453-long, AAV-CETP-587-short and AAV-CETP-587-long were analyzed by dot blot experiments (Figure 4). 5 x10¹⁰ or 1 x10¹⁰ purified AAV particles AAV-CETP-453-short and AAV-CETP-453-long were spotted onto a nitrocellulose membrane using a vacuum device. Likewise, 5 x10¹⁰ purified AAV particles AAV-CETP-587-short and AAV-CETP-587-long were spotted onto the same membrane. As negative control wtAAV was spotted ranging from 5.0x10¹⁰ to 6.3x10⁹ capsids per dot. After blocking of the membrane with blocking buffer (5% milk powder in PBS containing 0.05% Tween-20), the membrane was incubated with a polyclonal anti-CETP serum generated by immunizing rabbits with the CETP epitope coupled to KLH. After washing of the membrane with PBS/0.05% Tween-20, binding of the anti-CETP antibodies to the spotted AAV variants was detected with an anti-rabbit IgG HRP conjugate (CALTAG). After washing, signals were detected by chemiluminescence using the ECL system (AMERSHAM BIOSCIENCE).

The result demonstrate that there is a specific detection of the CETP epitope inserted into the AAV capsid at position I-453 or I-587 by the respective CETP antibody demonstrating that the epitope is displayed on the surface of the AAV particle.

### 5. Double insertion of a β-amyloid epitope at position I-453 and I-587 of the AAV capsid

The cloning approach described below is used for the double insertion of an epi- or mimotope sequence into the AAV capsid at position I-453 and I-587 using a defined cloning strategy.

### 5.1. Insertion of an FseI restriction site into pCIVP2

An FseI restriction site was inserted into the vectors pCIVP2-I587-Not-AscI and pCIVP2-I453-NotI-AscI located between I-453 and I-587 by site-directed mutagenesis using the QUIKCHANGE II SITE-DIRECTED MUTAGENESIS KIT (STRATAGENE) and the oligonucleotides
mutashe-9 5'-GGT GAA TCC GGG **G**CC GGC CAT GGC AAG C-3'
   (SEQ ID NO: 80)
   and
mutashe-10 5'-GCT TGC CAT GGC CGG **C**CC CGG ATT CAC C-3'
   (SEQ ID NO: 81).

### 5.2. Cloning of a β-amyloid epitope at position I-587 of pUCAV2

The β-amyloid epitope DAEFRHDSG (SEQ ID NO: 65) (aa 1 - 9 of human β-amyloid) was cloned into the NotI/AscI restriction site of the vector pCIVP2-I587-NotI-AscI (modified as described in 5.1) using the sense and anti-sense oligonucleotides
β-amyloid-for 5' -GGC CGC AGG CGG AGG GGG AGG CGA CGC CGA GTT
   CAG ACA CGA CAG CGG CGG CGG AGG GGG AGG CGC GG-3'
   (SEQ ID NO: 76)
   and
β-amyloid-rev 5' -CGC GCC GCG CCT CCC CCT CCG CCG CCG CTG TCG
   TGT CTG AAC TCG GCG TCG CCT CCC CCT CCG CCT GC-3'
   (SEQ ID NO: 77)

The oligonucleotides encode the β-amyloid epitope with a glycine adaptor sequence:
(A)₃-(G)₅-DAEFRHDSG-(G)₅-(A)₂ (SEQ ID NO: 82)

Cloning was performed as described above (2.2).

The BsiWI/XmaI fragment of pCI-VP2-587-NotI-AscI encoding a VP-2 fragment containing the β-amyloid epitope at position I-587 was sub-cloned into pUCAV2-Agel as described above (2.3). The resulting vector was referred to as pUCAV2-amyloid-587

### 5.3. Cloning of a β-amyloid epitope at position I-453 of pCIVP2

The β-amyloid epitope (DAEFRHDSG, SEQ ID NO: 65) was cloned into the NotI/AscI restriction site at the insertion site I-453 of the vector pCIVP2-I453-NotI-AscI (modified as described in 5.1) using the sense and anti-sense oligonucleotides
Amyloid 453 for 5' -G GCC GGC GGA GGC GGT GGG GAC GCC GAA TTC
   AGA CAC GAC AGC GGC GGA GGC GGT GGA GGG-3'
   (SEQ ID NO: 66)
Amyloid 453rev 5' -C GCG CCC TCC ACC GCC TCC GCC GCT GTC GTG
   TCT GAA TTC GGC GTC CCC ACC GCC TCC GCC-3'
   (SEQ ID NO: 67)

The oligonucleotides encode the β-amyloid epitope with a glycine adaptor sequence:
(A)₂-(G)₅-DAEFRHDSG-(G)₅-R-(A)₂
   (SEQ ID NO: 83)

Cloning was performed as described above (1.2).

### 5.4. Cloning of a β-amyloid epitope at position I-453 and I-587 of pUCAV2

For production of recombinant AAV particles carrying the β-amyloid epitope at position I-587 and I-453, the vector pUCAV2-amyloid-587 was cut with BsiW/FseI and ligated with the 0.6 kb BsiW/FseI fragment of pCI-VP2-453-NotI-AscI. The BsiW/FseI fragment of pCI-VP2-453-NotI-AscI encodes the VP-2 fragment containing the β-amyloid epitope at position I-453. The resulting vector was referred to as pUCAV2-amyloid-453-587.

### 5.5. Production, purification and evaluation of AAV particles carrying a β-amyloid epitope at I-453 and I-587

For production of recombinant AAV particles carrying the β-amyloid epitope at position I-587 and I-453, 293 cells were transfected with the vector pUCAV2-amyloid-453-587 and the helper plasmid pUCAdV as described above (3.2 and 3.3). The corresponding AAV particles were referred to as AAV-amyloid-453-587.

For production of recombinant AAV particles carrying the β-amyloid epitope at position I-587, 293 cells were transfected with the vector pUCAV2-amyloid-587 and the helper plasmid pUCAdV as described above. The corresponding AAV particles were referred to as AAV-amyloid-587. All AAV particles were purified as described above

To evaluate the expression of the β-amyloid epitope at the surface of the AAV capsid, serial dilutions of purified AAV particles AAV-amyloid-453-587 and AAV-amyloid-587 were dotted on a membrane (Figure 5). As a negative control AAV particles carrying a CETP epitope at position I-587 were dotted. As a positive control a β-amyloid peptide (aa 1 - 42) (BIOSOURCE) was dotted. After blocking of the membrane with blocking buffer (5% milk powder in PBS containing 0.05% Tween-20), the β-amyloid epitope was detected using an anti-β-amyloid mAb 6E10 (CHEMICON) (Figure 5). The anti-β-amyloid mAb was used at a concentration of 1.0 µg/ml in PBS / 1% milk powder / 0.05% Tween-20. Binding of the anti-β-amyloid mAb was detected using a peroxidase labeled anti-mouse IgG antibody (CALTAG). After washing, signals were detected by chemiluminescence using the SUPERSIGNAL WEST PICO CHEMILUMINESCENT SUBSTRATE (PIERCE).

These data demonstrate that the double insertion of the epitope into the insertion sites I-453 and I-587 resulting in higher epitope density at the capsid surface than the singular insertion of the epitope at position I-587 leads to an at least 2 fold higher affinity of the double insertion mutant to the β-amyloid antibody as compared to the I-587-insertion mutant alone.

### 6. Generation of modified AAV2 variants by insertion of a αᵥβ₃ integrin targeting sequence at position I-453 of the AAV capsid by genetic manipulation

### 6.1. Determination of titers

In order to generate a number of insertion mutants that target the αᵥβ₃ and αᵥβ₅ integrins, the targeting peptide RGD-4C with its sequence
ACDCRGDCFCA (SEQ ID NO: 84)
was inserted between G₄₅₃ and T₄₅₄ of AAV2 structural proteins VP-1, VP-2 and VP-3 of AAV2 (mutant named RGD4C 453). This targeting insertion was further combined with the two point mutations R₅₈₅A and R₅₈₈A in order to diminish binding to AAV2's natural receptor HSPG (named RGD4C 453 A2). The identical targeting peptide was inserted into the previously described insertion site I-587 (named RGD4C 587) and the corresponding R₅₈₅A / R₅₈₈A mutant (named RGD4C 587 A2). Further, the identical targeting peptide was inserted into both insertion sites I-453 and I-587 (named RGD4C 453 & 587) and the corresponding R₅₈₅A / R₅₈₈A mutant (named RGD4C 453 & 587 A2).

All mutants were generated by site-direct mutagenesis used to package scGFP and titrated as follows: 293 cells were seeded at 80% confluence and co-transfected by calcium phosphate with a total of 37.5 µg vector plasmid pAAV/EGFP, packaging helper plasmid (coding for Rep and Cap), and adenoviral plasmid pXX6 (Xiao et al., 1998) at a 1:1:1 ratio. 48 h after transfection, cells were harvested and pelleted by low-speed centrifugation. Cells were resuspended in 150 mM NaCl, 50 mM Tris-HCl (pH 8.5), freeze-thawed several times, and treated with Benzonase for 30 min at 37°C. Cell debris was spun down at 3.700 g for 20 min at 4°C. Supernatant was loaded onto a discontinuous iodixanol gradient. 40% phase containing the vector was harvested and titered.

Genomic titers of vector stocks were determined by quantitative PCR (Theiss et al., 2003). For this, viral DNA was isolated from vector stocks according to the DNeasy kit protocol (QIAGEN).

The capsid titers of vector stocks were determined by A20 ELISA as previously described (Girod et al., 1999).

Transducing titers were determined transducing HeLa cells with the respective AAV mutant or wild-type expressing scGFP. Transduced cells were then counted by standard FACS analysis. In brief, 7 x10⁴ HeLa cells were seeded into each well of a 12-well-plate and incubated for 24 h. Serial dilutions of the respective virus were added to the cells and incubated for 48 h at 37°C and 5% CO₂. Cells were trypsinized, transferred into FACS tubes and analyzed by standard conditions by flow cytometry (FACS Becton-Dickinson) in order to calculate the amount of transducing particles per ml.

The amount of capsid per genomic particles in each viral preparation showed, by comparison with wild-type, that all mutants assembled capsids and packaged efficiently viral genomes (Table 10, columns Cap/ml and GenP/ml). Determination of transducing particles (tP) is dependent on all steps of viral transduction including binding, uptake, lysosomal escape and activation of gene expression. All tested mutants except the two double insertions mutants AAV2 RGD4C 453 & 587 and AAV2 RGD4C 453 & 587 A2 had considerably high transducing titers of at least 10⁷ transducing particles per ml. Transducing titers of both double insertion mutants were below the detection limit although a successful cell binding could be determined.

The ratio of capsids per genomic particles provides information about packaging efficiencies and thus allows determining whether a capsid modification interferes with packaging of vector genomes into the preformed AAV capsids. All tested mutants were able to efficiently package viral genomes (Cap/GenP). Consequently, the peptide RGD4C can be successfully inserted into I-453. The tested mutants show capsid formation and efficient packaging of viral genomes.

The ratio of genomic particles per transducing particles (GenP/tP) is an indicator for the ability of a mutant containing a viral genome to successfully transduce a cell and express its reporter gene. Consequently the higher the ratio the lower is the transducing activity of the mutant for the specific cell line.

The insertion of the RGD-4C peptide in either I-453 or I-587 led, compared to wild-type, to a higher GenP/tP ratio. Therefore, the inserted targeting peptide somehow interfered with the transducing activity of HeLa cells. The two double insertion mutants AAV2 RGD4C 453 & 587 and AAV2 RGD4C 453 & 587 A2 were completely unable to transduce HeLa cells. This is surprising as αᵥβ₅, another reported target molecule of the RGD-4C, is known to be present on HeLa cells and further is known to be an AAV2 secondary receptor (Summerford et al., 1999).

As expected, the two point mutations R₅₈₅A and R₅₈₈A (AAV A2 mutant) led to a 3 log reduction of transducing activity of the mutant capsids on HeLa cells. The insertion of one targeting peptide in I-453 or I-587 restored to some extent the A2 mutants' ability to transduce HeLa cells, which can be explained by the fact that αᵥβ₅ is expressed on HeLa cells and that therefore αᵥβ₅ can compensate for the diminished HSPG binding.

**Table 10: Titers of αᵥβ₃ integrin targeting**

| **Virus** | **Cap/ml** | **GenP/ml** | **tP/ml** | **Cap/GenP** | **GenP/tP** |
|---|---|---|---|---|---|
| wtAAV2 | 1,09E+13 | 7,07E+11 | 6,84E+10 | 15 | 10 |
| wtAAV2∓ | 9,50E+12 | 1,25E+12 | 4,34E+10 | 8 | 29 |
| AAV2 A2 | 1,00E+13 | 1,11E+12 | 2,75E+07 | 9 | 40364 |
| AAV2 RGD4C 453 | 1,12E+12 | 4,97E+11 | 1,63E+08 | 2 | 3049 |
| AAV2 RGD4C 453 A2 | 1,54E+12 | 6,12E+11 | 2,55E+08 | 3 | 2400 |
| AAV2 RGD4C 587 | 1,17E+12 | 4,73E+11 | 1,22E+08 | 3 | 3877 |
| AAV2 RGD4C 587 A2 | 2,83E+12 | 2,42E+11 | 6,45E+08 | 12 | 375 |
| AAV2 RGD4C 453 & 587 | 1,34E+12 | 1,55E+11 | - | 9 | - |
| AAV2 RGD4C 453 & 587 A2 | 5,02E+11 | 2,25E+11 | - | 2 | - |

| | | | | | |
|---|---|---|---|---|---|
| (Cap = capsids; GenP = genomic particles; tP = transducing particles) | | | | | |

### 6.2. Binding of capsids to αᵥβ₃ integrin

The binding of AAV2 RGD-4C insertion mutants to their receptor molecule αᵥβ₃ integrin was analyzed as previously described (Shi and Bartlett, 2003) and normalized to the amount of AAV2 particles detected by A20. In brief an ELISA plate was coated with A20 (75 ng/well, PROGEN). After blocking (PBS, 1% milk powder, 1% Tween) 1.00 x10¹⁰ particles were given per well. For detection of a functional RGD purified αᵥβ₃ integrin (100 ng/well, CHEMICON) was added to the plate and detected with anti-integrin αV antibody (C-terminus/intracellular, Dil. 1:1.000, CHEMICON). For quantification of viral particles in each well biotinylated A20 (250 ng/ well) was used. The ratio "anti-integrin αᵥ" : "A20-biot" was used for normalization of the amount of αᵥβ₃ binding to total particles.

Both wild-type and the A2 mutant did not show any binding of αᵥβ₃ (Figure 6). The mutants with a single inserted targeting peptide either at I-453 or I-587 (RGD4C 453 and RGD4C 587) showed clearly detectable binding of αᵥβ₃. Once the two arginines R₅₈₅ and R₅₈₈ were replaced by alanine, these A2 mutants showed much better binding of αᵥβ₃ in the ELISA, whereas RGD4C 453 was even superior to RGD4C 587. The double insertion mutation RGD4C 453 & 587 also showed very good binding activity for αᵥβ₃. The double insertion double replacement mutant RGD4C 453 & 587 A2 did not show a further increase in binding compared to RGD4C A2. However, if compared to the RGD4C 587 A2 mutant the additional insertion in I-453 clearly increased its binding activity.

Consequently, the insertion site I-453 is well suited for the insertion of peptides, as the RGD4C peptide is displayed on the surface of the capsid and is accessible to antibodies and therefore most likely to the corresponding cellular receptors. Its combination with the R₅₈₅A and R₅₈₈A mutations increased its binding activities approximately 50 fold suggesting that R₅₈₅A and/or R₅₈₈A mutants enhance the accessibility of the insert to an antibody and/or receptor.

Considering the data from example 6.1, namely that the double insert mutants AAV2 RGD4C 453 & 587 and AAV2 RGD4C 453 & 587 A2 did not show any transducing activity on HeLa cells, whereas these double insert mutants very efficiently display the peptide on the surface and strongly bind αᵥβ₃, can be explained by the following hypothesis: (i) there might be a difference whether or not membrane bound receptor as in example 6.1 or the soluble receptor αᵥβ₃ in this ELISA is used. Whereas the inserted peptides are perfectly accessible to small, soluble receptors the high number of modifications on the surface of the capsid may sterically hinder the binding of a larger, membrane fixed receptor; (ii) although the double insert mutant binds to the membrane bound receptor, the uptake of the virus or the intracellular processing is hindered so that no transduction takes place. For example it is reasonable to believe that the known mechanism of endosomal acidification triggering conformational changes of the viral capsid might be impaired if epitopes are presented in a too dense fashion.

### 6.3. HSPG independent transduction

HSPG independent transduction was tested using a Chinese Hamster Ovarian (CHO) cell line with an HSPG knock-out phenotype (ATCC No.: CRL-2242) that likely expresses αᵥβ₃ integrin as can be concluded from Figure 8, where the addition of soluble RGD peptide inhibits transduction. Indeed, wild-type AAV2 had a very low transduction efficiency that was even reduced in the A2 mutant (Figure 7). The insertion of the RGD-4C peptide into I-453 alone does not lead to significant transduction rates, however, if combined with the A2 point mutations (RGD4C 453 A2 in Figure 7) transduction is even superior to I-587 insertion mutants, suggesting a conformational dependency for cell transduction.

This transduction could be inhibited by the addition of the soluble RGD peptide but only weakly by the unspecific RGE peptide (Figure 8), demonstrating that the transduction by this mutant became RGD-4C dependent. Transduction efficiency of these CHO HSPG KO cells was increased by a factor of about four in relation to wild-type (Figure 7).

The single insertion mutants at position I-587 (RGD4C 587 and RGD4C 587 A2) also showed an increased transduction rate compared to wild-type, but not as strong as for RGD4C 453 A2 (Figure 7). Transduction of RGD4C 587 was also inhibited by soluble RGD peptide indicating its dependence on the RGD-4C interaction with the αᵥβ₃ integrin.

The double insertion mutant (RGD4C 453 & 587 and RGD4C 453 & 587 A2) again were not able to efficiently transduce cells (Figure 7) as already shown for HeLa cells (Table 10) despite efficient binding to soluble αᵥβ₃ (Figure 6). Again this could be explained by either steric hindrance of the too dense targeting sequences or by an inefficient uptake and/or the intracellular processing of the virus.

### 7. Generation of further AAV Variants

### 7.1. Insertion of CETP Epitopes into the AAV2 capsid at position I-453

The following rabbit CETP derived epitopes were cloned into position I-453 of the AAV2 capsid using annealed oligonucleotides as described above. Each of the inserted epitope sequences in the AAV2 backbone at I-453 is flanked by the following alanine/glycine adaptors according to the following scheme (Xn represents the epitope sequence):
Type I Ala/Gly adaptor: (Ala)₂-(Gly)₃-Xn-(Gly)₄-Arg-(Ala)₂
Type II Ala/Gly adaptor: (Ala)₃-(Gly)₃-Xₙ-(Gly)₄-Arg-(Ala)₂

**Table 11: rabbit CETP derived epitopes in I-453**

| **Name / Peptide Seq.** | **Type** | **sense Oligonucleotide** | **anti-sense Oligonucleotide** | **Adaptor** |
|---|---|---|---|---|
| **CETP TP10** | | 5′ GGCCGGCGGTGGAGCCA AGGCCGTGAGCAACCTGAC CGAGAGCAGAAGCGAGAGC CTGCAGAGCGGTGGCGGTG GA 3′ SEQ ID NO: 128 | 5′CGCGTCCACCGCCACC GCTCTGCAGGCTCTCGCT TCTGCTCTCGGTCAGGTT GCTCACGGCCTTGGCTCC ACCGCC 3′ SEQ ID NO: 129 | Type I Ala/Gly |
| AKAVSNLTESRSESLQS | Epitope | | | |
| SEQ ID NO: 96 | | | | |
| **CETP TP11** | | 5′ GGCCGGCGGTGGAAGCC TGACCGGCGACGAATTCAA GAAGGTGCTGGAGACCGGT GGCGGTGGA 3′ SEQ ID NO: 130 | 5′ CGCGTCCACCGCCACC GGTCTCCAGCACCTTCTT GAATTCGTCGCCGGTCAG GCTTCCACCGCC 3′ SEQ ID NO: 131 | Type I Ala/Gly |
| SLTGDEFKKVLET | Epitope | | | |
| SEQ ID NO: 97 | | | | |

| **Name / Peptide Seq.** | **Type** | **sense Oligonucleotide** | **anti-sense Oligonucleotide** | **Adaptor** |
|---|---|---|---|---|
| **CETP TP12** | | 5′GGCCGGCGGTGGAAGAG AGGCCGTGGCCTACAGATT CGAAGAGGACGGTGGCGGT GGA 3′ SEQ ID NO: 132 | 5′CGCGTCCACCGCCACC GTCCTCTTCGAATCTGTA GGCCACGGCCTCTCTTCC ACCGCC 3′ SEQ ID NO: 133 | Type I Ala/Gly |
| REAVAYRFEED | Epitope | | | |
| SEQ ID NO: 98 | | | | |
| **CETP TP13** | | 5′GGCCGGCGGTGGAATCA ACCCCGAGATCATCACCCT GGACGGCGGTGGCGGTGGA 3′ SEQ ID NO: 134 | 5′CGCGTCCACCGCCACC GCCGTCCAGGGTGATGAT CTCGGGGTTGATTCCACC GCC 3′ SEQ ID NO: 135 | Type I Ala/Gly |
| INPEIITLDG | Epitope | | | |
| SEQ ID NO: 99 | | | | |
| **CETP TP18** | | 5′GGCCGGCGGTGGAGACA TCAGCGTGACCGGTGCACC CGTGATCACCGCCACCTAC CTGGGTGGCGGTGGA 3′ SEQ ID NO: 136 | 5′ CGCGTCCACCGCCACC CAGGTAGGTGGCGGTGAT CACGGGTGCACCGGTCAC GCTGATGTCTCCACCGCC 3′ SEQ ID NO: 137 | Type I Ala/Gly |
| DISVTGAPVITATYL | Epitope | | | |
| SEQ ID NO: 100 | | | | |
| **CETP TP20** | | 5′GGCCGGCGGTGGAGACA TCAGCGTGACCGGTGCACC CGTGATCACCGCCGGTGGC GGTGGA 3′ SEQ ID NO: 138 | 5′ CGCGTCCACCGCCACC GGCGGTGATCACGGGTGC ACCGGTCACGCTGATGTC TCCACCGCC 3′ SEQ ID NO: 139 | Type I Ala/Gly |
| DISVTGAPVITA | Epitope | | | |
| SEQ ID NO: 101 | | | | |
| **Ritsch-1** | | 5′GGCCGGCGGTGGAGACC AGAGCGTGGACTTCGAGAT CGACAGCGCCGGTGGCGGT GGA 3′ SEQ ID NO: 140 | 5′ CGCGTCCACCGCCACC GGCGCTGTCGATCTCGAA GTCCACGCTCTGGTCTCC ACCGCC 3′ SEQ ID NO: 141 | Type I Ala/Gly |
| DQSVDFEIDSA | Epitope | | | |
| SEQ ID NO: 127 | | | | |

### 7.2. Insertion of CETP Epitopes into the AAV2 capsid at position I-453 and I-587

Using the cloning strategy described above, the following AAV2 capsid variants carrying rabbit CETP epitopes at position I-453 and I-587 were produced:

**Table 12: CETP double insertion mutants**

| **Name** | **Epitope at I-453** | **Epitope at I-587** |
|---|---|---|
| **AAV-TP10-2x** | AKAVSNLTESRSESLQS SEQ ID NO: 96 | AKAVSNLTESRSESLQS SEQ ID NO: 96 |
| **AAV-TP11-2x** | SLTGDEFKKVLET SEQ ID NO: 97 | SLTGDEFKKVLET SEQ ID NO: 97 |
| **AAV-TP12/13** | REAVAYRFEED SEQ ID NO: 98 | INPEIITLDG SEQ ID NO: 99 |
| **AAV-TP12-2x** | REAVAYRFEED SEQ ID NO: 98 | REAVAYRFEED SEQ ID NO: 98 |
| **AAV-TP13-2x** | INPEIITLDG SEQ ID NO: 99 | INPEIITLDG SEQ ID NO: 99 |
| **AAV-TP18-2x** | DISVTGAPVITATYL SEQ ID NO: 100 | DISVTGAPVITATYL SEQ ID NO: 100 |
| **AAV-TP20-2x** | DISVTGAPVITA SEQ ID NO: 101 | DISVTGAPVITA SEQ ID NO: 101 |
| **AAV-Ritsch1-2x** | DQSVDFEIDSA SEQ ID NO: 127 | DQSVDFEIDSA SEQ ID NO: 127 |
| **AAV2-CETin-2x** | CDAGSVRTNAPD SEQ ID NO: 60 | CDAGSVRTNAPD SEQ ID NO: 60 |

### 7.3. Insertion of Cytokine Epitopes into the AAV2 capsid at position I-453

The following murine cytokine derived epitopes were cloned into position I-453 of the AAV2 capsid using annealed oligonucleotides as described above. Each of the inserted epitope sequences in the AAV2 backbone at I-453 is flanked by the alanine/glycine adaptors according this section 7 for I-453 above.

**Table 13: murine cytokine derived epitopes in I-453**

| **Name / Peptide Seq.** | **Type** | **sense Oligonucleotide** | **anti-sense Oligonucleotide** | **Adaptor** |
|---|---|---|---|---|
| **mTNFα-V1** | | 5′GGCCGCCGGTGGAGGCA GCAGCCAGAACAGCAGCGA CAAGCCCGTGGCCCACGTG GTGGCTAACCACCAGGTGG AGGGCGGTGGAGGG 3′ SEQ ID NO: 145 | 5′ CGCGCCCTCCACCGCC CTCCACCTGGTGGTTAGC CACCACGTGGGCCACGGG CTTGTCGCTGCTGTTCTG GCTGCTGCCTCCACCGGC 3′ SEQ ID NO: 148 | Type II Ala/Gly |
| SSQNSSDKPVAHVVANHQVE | Epitope | | | |
| SEQ ID NO: 142 | | | | |
| **mIL-17-V1** | | 5'GGCCGCCGGTGGAGGCA ACGCCGAGGGCAAGCTTGA CCACCACATGAACAGCGTG CTGGGCGGTGGAGGG 3' SEQ ID NO: 146 | 5′ CGCGCCCTCCACCGCC CAGCACGCTGTTCATGTG GTGGTCAAGCTTGCCCTC GGCGTTGCCTCCACCGGC 3′ SEQ ID NO: 149 | Type II Ala/Gly |
| NAEGKLDHHMNSVL | Epitope | | | |
| SEQ ID NO: 143 | | | | |
| **mIL-6-V2** | | 5′GGCCGCCGGTGGAGGCC TGGAGGAATTCCTGAAGGT GACCCTGAGAAGCGGCGGT GGAGGG 3′ SEQ ID NO: 147 | 5′CGCGCCCTCCACCGCC GCTTCTCAGGGTCACCTT CAGGAATTCCTCCAGGCC TCCACCGGC 3′ SEQ ID NO: 150 | Type II Ala/Gly |
| LEEFLKVTLRS | Epitope | | | |
| SEQ ID NO: 144 | | | | |

The following sequences, which are homologues to the corresponding murine cytokine sequences, can be integrated into the AAV2 capsid at position I-453 according to the methods described above:

**Table 14: human cytokine derived epitopes in I-453**

| **Cytokine** | **murine epitope** | **human epitope** |
|---|---|---|
| **TNF-α V1** | SSQNSSDKPVAHVVANHQVE SEQ ID NO: 142 | SSRTPSDKPVAHWANPQAE SEQ ID NO: 116 |
| **TNF-α V2** | SQNSSDKPVAHWANH SEQ ID NO: 151 | SRTPSDKPVAHWANP SEQ ID NO: 117 |
| **TNF-α V3** | SSQNSSDKP SEQ ID NO: 152 | SSRTPSDKP SEQ ID NO: 118 |
| **IL-17 V1** | NAEGKLDHHMNSVL SEQ ID NO: 143 | NADGNVDYHMNSVP SEQ ID NO: 119 |
| **IL-17 V2** | EGKLDHHMNSV SEQ ID NO: 153 | DGNVDYHMNSV SEQ ID NO: 120 |
| **IL-6 V1** | KSLEEFLKVTLRSTRQ SEQ ID NO: 154 | RSFKEFLQSSLRALRQ SEQ ID NO: 121 |
| **IL-6 V2** | LEEFLKVTLRS SEQ ID NO: 144 | FKEFLQSSLRA SEQ ID NO: 122 |

### 7.4. Insertion of Cytokine Epitopes into the AAV2 capsid at position I-453 and I-587

Using the cloning strategy described above, the following AAV variants carrying different cytokine epitopes at position I-453 and I-587 can be generated (bivalent vaccines):

**Table 15: double insertion variants for cytokine derived epitopes**

| **combination** | **Epitope at I-453** | **Epitope at I-587** |
|---|---|---|
| | **mTNFα-V1** | **mIL-17-V1** |
| **TNF-α / IL-17** | SSQNSSDKPVAHVVANHQVE | NAEGKLDHHMNSVL |
| | SEQ ID NO: 142 | SEQ ID NO: 143 |
| | **mTNFα-V1** | **mIL-6-V2** |
| **TNF-α / IL-6** | SSQNSSDKPVAHVVANHQVE | LEEFLKVTLRS |
| | SEQ ID NO: 142 | SEQ ID NO: 144 |
| | **mIL-17-V1** | **mTNFα-V1** |
| **IL-17 / TNF-α** | NAEGKLDHHMNSVL | SSQNSSDKPVAHVVANHQVE |
| | SEQ ID NO: 143 | SEQ ID NO: 142 |
| | **mIL-6-V2** | **mTNFα-V1** |
| **IL-6/ TNF-α** | LEEFLKVTLRS | SSQNSSDKPVAHVVANHQVE |
| | SEQ ID NO: 144 | SEQ ID NO: 142 |
| | **mIL-17-V1** | **mIL-6-V2** |
| **IL-17 / IL-6** | NAEGKLDHHMNSVL | LEEFLKVTLRS |
| | SEQ ID NO: 143 | SEQ ID NO: 144 |
| | **mIL-6-V2** | **mIL-17-V1** |
| **IL-6 / IL-17** | LEEFLKVTLRS | NAEGKLDHHMNSVL |
| | SEQ ID NO: 144 | SEQ ID NO: 143 |

### 8. Immunization of rabbits with AAV-based vaccines

### 8.1. Production and purification of AAV2-based vaccines for immunization experiments

For production of AAV particles HEK 293-T cells were co-transfected with the vector plasmid pUCAV2 containing the subcloned epitope (in I-453 and/or I-587) and the helper plasmid pUCAdV as described above. For large scale production 30 - 60 015 cm cell culture plates with 7.5x10⁶ 293-T cells were seeded and cultivated at 37°C, 5% CO₂ in a humidified atmosphere. Co-transfection of the cells with the vector plasmid pUCAV2 containing the epitope (in I-453 or I-587) and pUCAdV was performed as described above. 72h after transfection 293-T cells and medium were harvested and centrifuged at 3000 g at 4°C for 15 min. The cell pellet was resuspended in 15 - 30 ml lysis buffer (50 mM HEPES, 200 mM NaCl, 2.5 mM MHCl₂; pH 6.8) and objected to three rounds of freeze and thaw cycles. The cleared cell culture supernatant was concentrated by TFF (tangential flow filtration) using the SARTOFLOW^{®} Slice 200 Benchtop Cross-flow system using a SARTOCON^{®} Slice 200 cassette (Hdyrosart membrane). The TFF concentrate of the cell culture supernatant (about 35 ml) was pooled with the cleared crude lysate and subsequently treated with 1667 U/ml benzonase (MERCK) at 37°C for 2h - 4h. After benzonase treatment the pool of crude lysate and TFF concentrate was centrifuged at 3600 g for 5min at 4°C. The AAV-containing supernatant was separated through a size exclusion chromatography (SEC) column. SEC was performed using a XK50/20 column packed with SUPERDEX 200^{®} resin beads and SEC running buffer (50 mM HEPES, 400mM NaCl, 2.5 mM MgCl₂; pH 6.8). SEC fractions were analyzed by AAV2 ELISA. AAV-containing fractions were pooled and objected to iodixanol gradient centrifugation. Iodixanol solutions of different concentrations were layered beneath the pool of virus containing SEC fraction in QUICKSEAL^{®} centrifugation tubes (25 x 89 mm; BECKMAN). By this an Iodixanol gradient was created composed of 4.0 ml 60% on the bottom, 5.0 ml 40%, 4.0 ml 25% and 5.5 ml 15% Iodixanol with the virus solution on top. The gradient was centrifuged using a fixed angel rotor (Ti 70.1 rotor, BECKMAN) at 65000 rpm for 1 h at 18°C. The 40% phase containing the AAV particles was then extracted with a cannula by puncturing the tube underneath the 40% phase and allowing the solution to drip into collecting tubes. Fractions of about 0.5 ml were collected until the 25% phase was reached. The AAV capsid titer of the fractions was determined using a commercially available ELISA (AAV Titration ELISA, PROGEN). Purity of the AAV-containing fractions was determined by SDS-PAGE and subsequent colloidal Coomassie staining. Fractions with high purity of AAV particles were pooled and the capsid titer of the final pool was determined by AAV2 titration ELISA.

### 8.2. Breaking of self-tolerance by AAV-based vaccines

A panel of AAV-based vaccines carrying epitopes derived from rabbit CETP was generated as described above. AAV-based CETP vaccines were compared with the corresponding peptide vaccines containing the same epitope coupled to LPH (Limulus polyphemus hemocyanine) as a carrier protein. The peptides were chemically synthesized with a C- or N-terminal cysteine residue that was used for coupling of the peptides to LPH. Synthesis and coupling of the peptides was performed by BIOGENES (Berlin, Germany).

The vaccines described in table Table 16 were used for immunization of rabbits:

**Table 16: Vaccines used for immunization of rabbits**

| **Name of vaccine** | **Vaccine carrier** | **Insertion Site** | **Epitope** | **Dose (µg)** |
|---|---|---|---|---|
| **AAV-TP11** | AAV2 | I-587 | SLTGDEFKKVLET | 10.9 |
| | | | SEQ ID NO: 97 | |
| **AAV-TP12** | AAV2 | I-587 | REAVAYRFEED | 14.1 |
| | | | SEQ ID NO: 98 | |
| **AAV-TP13** | AAV2 | I-587 | INPEIITLDG | 13.3 |
| | | | SEQ ID NO: 99 | |
| **AAV-TP18** | AAV2 | I-587 | DISVTGAPVITATYL | 7.2 |
| | | | SEQ ID NO: 100 | |
| **LPH-TP11** | LPH | N/A | **C**SLTGDEFKKVLET | see text |
| | | | SEQ ID NO: 155 | |
| **LPH-TP12** | LPH | N/A | **C**REAVAYRFEED | see text |
| | | | SEQ ID NO: 156 | |
| **LPH-TP13** | LPH | N/A | **C**INPEIITLDG | see text |
| | | | SEQ ID NO: 157 | |
| **LPH-TP18** | LPH | N/A | **C**DISVTGAPVITATYL | see text |
| | | | SEQ ID NO: 158 | |

For each vaccination approach two rabbits were immunized s.c. with the vaccines shown in the table above four times (one prime and three boost immunizations). The first boost immunization was performed 2 weeks after an initial prime immunization. Rabbits were boosted another two times with the vaccines at intervals of 3 weeks. Serum of the immunized animals was prepared two weeks after each boost immunization.

The purified AAV-based vaccines were mixed an equal volume of formulation buffer (PBS with 1% sorbitol, 0.2% Tween-20, 25 % propylenglycol, 200 mM NaCl and 2.5 mM MgCl₂) for stabilization of the particles and stored at -80°C until administration. If necessary, the volume of the AAV-based vaccines was adjusted to 0.3 ml with formulation buffer directly before application. The vaccines were administered s.c. in the presence of 0.7 ml adjuvant (total volume 1 ml). The adjuvant was provided by BIOGENES and contained amongst others 0.01% lipopolysaccharide derived from Phormidium, 95% paraffin oil, 2.4% Tween-40 and 0.1% cholesterol.

The LPH-coupled peptides (in 0.3 ml TBS) were administered s.c. in the presence of 0.7 ml of the adjuvant provided by BIOGENES. 1 mg of the LPH-peptide conjugate was administered for the prime immunization. 0.5 mg of the conjugate was used for the 1^{st} boost immunization and 0.25 mg of the conjugate were used for the 2^{nd} and 3^{rd} boost immunization.

Induction of anti-CETP auto-antibodies in the vaccinated animals was determined by ELISA using recombinant rabbit CETP as antigen. For production of rabbit CETP, the CETP cDNA was amplified by RT-PCR using the primers
rCETP-uni 5'- GGG GAA TTC ATG TCC CAA AGG CGC CTC CTA CG-3'
   (SEQ ID NO: 159)
   and
rCETP-rev 5'- GGG GGA TCC CTA GCT CAG GCT CTG GAG GAA ATC C-3'
   (SEQ ID NO: 160)
and rabbit liver PolyA⁺ RNA (CLONTECH) as template. The CETP cDNA was cloned into the EcoRI / BamHI site of the vector p3XFLAG-CMV-8 (SIGMA). The resulting vector encodes the mature CETP sequence with a C-terminal FLAG^{®}-tag and an N-terminal preprotrypsin leader sequence for secretion of the recombinant protein. For expression of recombinant rabbit CETP 293T cells were transfected with the vector by calcium phosphate transfection as described above. CETP was purified from the cell culture supernatant by affinity chromatography using anti-FLAG^{®} M2 agarose beads (SIGMA). Purity of the recombinant rabbit CETP was analyzed by SDS-PAGE and subsequent colloidal Coomassie staining. CETP activity was determined using a commercially available CETP activity assay (ROAR).

For titration of rabbit CETP auto-antibodies in the immune sera, a 96-well MAXISORP plate (NUNC) was coated with purified recombinant rabbit CETP (100 ng/well) for 1 h at 37°C. After coating wells were washed with wash buffer (PBS / 0.1 % Tween-20) and subsequently incubated with blocking buffer (5% skim milk in wash buffer) for 1 h at 37°C. After blocking of the wells, immobilized CETP was incubated with serial dilutions of the immune sera in dilution buffer (wash buffer with 1% skim milk and 1% BSA) for 1 h at 37°C. Rabbit pre-immune sera or rabbit sera of unrelated vaccinations served as negative controls. After washing binding of rabbit IgG to the immobilized CETP was detected using a HRP-labelled anti-rabbit IgG antibody (H+L) (DAKO; 1:2500 in dilution buffer). Signals (OD) were detected using TMB (KEMENTEC) as substrate.

CETP auto-antibody titers were determined by end point dilution. The titer of the immune serum corresponds to the intersection point of the titration curve of the immune sera with the limit of detection of the assay.
The limit of detection (LOD) of the assay was calculated as follows:
Mean OD (unspecific sera) + 3.3 x standard deviation OD (unspecific sera)

In addition to the CETP auto-antibody titers, the anti-peptide titers of the immune sera were analyzed. The free peptides (corresponding to the epitopes integrated in the AAV capsid or coupled to LPH) were covalently immobilized in a 96-well plate (REACTI-BIND™ Amine-binding, Maleic Anhydride Activated Plates; PIERCE). For immobilization of the peptide, the 96-well plate was incubated with 1 µg peptide per well in a total volume of 50 µl PBS for at least 1 h at 37°C. After coating with the peptides wells were blocked with 200 µl / well blocking buffer (PBS / 5% skim milk / 0.1 % Tween-20) for 1 h at 37°C. After blocking of the wells, immobilized peptides were incubated with serial dilutions of the immune sera in dilution buffer (PBS with 1% skim milk, 1% BSA, 0.1% Tween-20) for 1 h at 37°C. Rabbit pre-immune sera or rabbit sera of unrelated vaccinations served as negative controls. After washing binding of rabbit IgG to the immobilized CETP was detected using a HRP-labelled anti-rabbit IgG antibody (DAKO; 1:2500 in dilution buffer). Signals (OD) were detected using TMB (KEMENTEC) as substrate. Antibody titers were determined as described above.

Except for one animal vaccinated with AAV-TP13 the data demonstrate that vaccination with AAV-based vaccines induces high titers of target-specific auto-antibodies that are not obtained using peptide-based vaccines. Accordingly AAV-based vaccines are able to break self-tolerance and induce high levels of auto-antibodies (Figure 9). The immunogenic properties of the peptide based vaccines are reflected by the high titers of peptide specific antibodies induced by the peptide vaccines (Figure 10). However, these antibodies show only weak reaction with native rabbit CETP (Figure 9) suggesting that peptide based vaccines - although immunogenic - have only a limited potential to break self-tolerance and induce low levels of auto-antibodies.

### 8.3. The AAV capsid structure is essential for breaking of self-tolerance and induction of auto-antibodies

To demonstrate that the capsid structure and the structured, repetitive presentation of epitopes within the AAV-capsid are essential for breaking of self-tolerance of the immune system and induction of auto-antibodies, rabbits were immunized with heat-denatured AAV-TP11-2x or AAV-TP18-2x particles. Results were compared with vaccinations using the corresponding native particles. The AAV-variant AAV-TP11-2x carries the CETP TP11 epitope (SLTGDEFKKVLET, SEQ ID NO: 97) at positions I-453 and I-587. The AAV-variant AAV-TP18-2x carries the CETP TP18 epitope (DISVTGAPVITATYL, SEQ ID NO: 100) at positions I-453 and I-587. For heat denaturation the particles were mixed with an equal volume of formulation buffer (PBS with 1% sorbitol, 0.2% Tween-20, 25% propylenglycol, 200 mM NaCl and 2.5 mM MgCl₂) and incubated at 90°C for 15 min. Destruction of the particle conformation was analyzed by AAV2 titration ELISA recognizing a conformational epitope within the native capsid. Protein concentration of the heat-denatured particles was determined by Micro BCA assay (Pierce) and analyzed by Western blotting using a polyclonal anti-AAV2 antibody generated by immunization of rabbits with purified VP3 protein of AAV2 (data not shown).

Rabbits were immunized with heat-denatured AAV-TP11-2x particles (5.7 µg per application) or AAV-TP18-2x particles (1.8 µg per application) s.c. in the presence of an adjuvant provided by BIOGENES as described above. 2 weeks after an initial prime immunization rabbits were boosted with the heat-denatured particles. Serum of the animals was analyzed 2 weeks after the boost immunization for levels of CETP auto-antibodies as described above. In a control group rabbits were vaccinated with native AAV-TP11-2x or AAV-TP18-2x particles using the same regimen as for the heat-denatured particles.

Analysis of the CETP auto-antibody titer in the sera of the immunized animals demonstrates that destruction of the native capsid conformation results in a strongly impaired induction of CETP antibodies compared with the native vaccine (Figure 11) showing that the native capsid structure and the structured presentation of the epitopes within the capsid are essential for breaking of self-tolerance.

### 8.4. Evaluation of the impact of anti-AAV2 antibodies on immunization with AAV2-based vaccines

The immunization experiments demonstrated that AAV-based vaccines induce high titers of anti-AAV capsid antibodies in addition to the target specific antibodies (data not shown). However, most humans are AAV2 positive meaning that these persons have anti-AAV2 antibody titers that potentially might affect vaccination results using AAV2-based particles. To evaluate the impact of anti-AAV2 antibodies on the immunization success of AAV2-based vaccines, rabbits were pre-immunized by two applications of wtAAV2 (4.5 µg per application), before immunization (prime and two boost immunizations) with an AAV2-based CETP vaccine (AAV-TP18) was started. wtAAV2 particles were administered s.c. or i.m. in the presence of an adjuvant provided by BIOGENES as described above. 2 weeks after an initial prime immunization with wtAAV2, rabbits were boosted once again with wtAAV2. Serum was analyzed two weeks after the prime and 1^{st} boost immunization for the level of anti-AAV2 antibodies. The anti-AAV2 antibody titer was determined by ELISA using immobilized wtAAV2 particles as described below. The data demonstrate that high levels of anti-wtAAV2 antibodies are detectable after two applications of wtAAV2 for both s.c. and i.m. administration (Figure 12A).

3 weeks after boost immunization with wtAAV2, rabbits received the first prime immunization with the AAV2-based vaccine AAV-TP18 (7.2 µg per application). The vaccine was administered s.c. or i.m. in the presence of adjuvant provided by BIOGENES as described above. Rabbits were boosted with the vaccines 2 weeks after the prime vaccination. Sera were analyzed 2 weeks after the boost vaccination for the level of CETP auto-antibodies (Figure 12B). CETP auto-antibody titers were determined as described above. Results were compared to vaccination (s.c.) of animals not pre-immunized with wtAAV2.

The data demonstrate that wtAAV2 pre-immunization results in high titers of anti-AAV2 capsid antibodies. However, these high anti-AAV2 capsid antibodies do not impair the immunization success of an AAV2-based vaccine, in this case regarding the induction of anti-CETP auto-antibodies. Accordingly, it is concluded that AAV2 sero-positive humans are equally eligible for vaccination with AAV2-particles as sero-negative humans and that sero-conversion of a vaccinated human during a vaccination protocol does not impair vaccination success.

Determination of anti-wtAAV2 antibody titers: The anti-AAV2 antibody titer was determined by ELISA using immobilized wtAAV2 particles. Briefly, 5x10⁰⁹ wtAAV2 particles were immobilized in each well of a 96-well MAXISORP plate (NUNC) in a total volume of 50 µl PBS per well. The plate was incubated at 37°C for 1 h. After blocking of the wells with PBS **/** 5% skim milk / 0.1% Tween-20, immobilized wtAAV2 particles were incubated with serial dilutions of the immune sera in dilution buffer (PBS with 1% skim milk, 1% BSA, 0.1 % Tween-20) for 1 h at 37°C. Rabbit pre-immune sera or rabbit sera of unrelated vaccinations served as negative controls. After washing binding of rabbit IgG to the immobilized AAV2 was detected using a HRP-labelled anti-rabbit IgG antibody and TMB as substrate. Antibody titers were determined as described above.

### 8.5. Prime / Boost regimen for AAV-based vaccines

16.4 µg AAV2 particles carrying the CETP-intern epitope (CDAGSVRTNAPD, SEQ ID NO: 60) at position I-453 and I-587 (AAV2-CETin-2x) were administered i.m. at each prime or boost immunization together with the adjuvant provided by BIOGENES as described above.

Three different regimens were evaluated. Group A received one prime and three boost applications of AAV2-CETin-2x (AAV2-based vaccination). Group B received one prime and one boost immunization with AAV2-CETin-2x followed by two boost immunizations with the LPH-coupled CETP-intern peptide (LPH-peptide boost). Group C received one prime and one boost immunization with AAV2-CETIn-2x followed by two boost immunizations with AAV1-CETin (AAV1 particle carrying the CETP-intern epitope at position I-588; 11.7 µg/application). In each group the first boost immunization was performed two weeks after the prime immunization. The 2^{nd} and 3^{rd} boost immunization was performed three weeks after the preceding boost vaccination.

Immune sera were analyzed for anti-CETP-reactivity (CETP auto-antibody titer) two weeks after the 1st, 2nd and 3rd boost immunization as described above (Figure 13).

Resulting data demonstrate that high levels of CETP auto-antibodies are detectable in animals vaccinated with AAV2-CETin-2x only (group A). There is no increase of CETP auto-antibodies observed in the group of animals boosted with LPH-coupled CETP peptide (group B). Furthermore, data demonstrate that switching of the serotype of the AAV-backbone (group C) has the potential to increase the immune response to a self-antigen compared to boost vaccinations with an individual AAV serotype.

### 8.6. Immunization against human IgE using AAV-based vaccines

A panel of AAV-based vaccines carrying epitopes derived from human IgE was generated as described above. AAV-based IgE vaccines were compared to the corresponding peptide vaccines containing the same epitope coupled to LPH as carrier protein. The peptides were chemically synthesized with a C- or N-terminal cysteine residue that was used for coupling of the peptides to LPH.
The following vaccines were used for immunization of rabbits:

**Table 17: AAV- and LPH-based vaccines used for immunization against human IgE**

| **Name of vaccine** | **Vaccine carrier** | **Insertion Site** | **Epitope** | **Dose (µg)** | **Appl.** |
|---|---|---|---|---|---|
| **AAV-Kricek** | AAV2 | I-587 | Kricek | 3.1 | s.c. |
| **AAV-3DEpi3** | AAV2 | I-587 | 3DEpi3 | 4.4 | s.c. |
| **AAV-Flex** | AAV2 | I-587 | Flex | 16.3 | i.m. |
| **AAV-Bind2** | AAV2 | I-587 | Bind2 | 5.1 | i.m. |
| **LPH-Kricek** | LPH | N/A | VNLTWSRASGC SEQ ID NO: 161 | see text | i.m. |
| **LPH-3DEpi3** | LPH | N/A | CDSNPRGVSAYLSR SEQ ID NO: 162 | see text | i.m. |
| **LPH-Flex** | LPH | N/A | CEDGQVMDVDLS SEQ ID NO: 163 | see text | i.m. |
| **LPH-Bind2** | LPH | N/A | CEKQRNGTLT SEQ ID NO: 164 | see text | i.m. |

For each vaccination approach two rabbits were immunized with the vaccines shown in the table above four times (one prime and three boost immunizations). The first boost immunization was performed 2 weeks after an initial prime immunization. Rabbits were boosted another two times with the vaccines at intervals of 3 weeks.

The purified AAV-based vaccines were mixed with an equal volume of formulation buffer (PBS with 1% sorbitol, 0.2% Tween-20, 25% propylenglycol, 200 mM NaCl and 2.5 mM MgCl₂) for stabilization of the particles and stored at -80°C until administration. If necessary, the volume of the vaccine was adjusted to 0.3 ml - 0.5 ml with formulation buffer directly before application. The AAV-based vaccines were administered s.c. or i.m. together with the BIOGENES adjuvant (total volume 1 ml).

The LPH-coupled peptides (in 0.3 ml TBS) were administered i.m. in the presence of 0.7 ml of the adiuvant provided bv BIOGENES. 1 mg of the LPH-peptide conjugate was administered for the prime immunization. 0.5 mg of the conjugate was used for the 1^{st} boost immunization and 0.25 mg of the conjugate were used for the 2^{nd} and 3^{rd} boost immunization.

Induction of anti-human IgE antibodies in the vaccinated animals was determined by ELISA using human IgE (DIATEC, Oslo, Norway) as antigen. A 96-well MAXISORP plate (NUNC) was coated with human IgE (1 µg/well) for 1 h at 37°C. After coating wells were washed with wash buffer (PBS / 0.1% Tween-20) and subsequently incubated with blocking buffer (5% skim milk in wash buffer) for 1h at 37°C. After blocking of the wells, immobilized human IgE was incubated with serial dilutions of the immune sera in dilution buffer (wash buffer with 1% skim milk and 1% BSA) for 1 h at 37°C. Rabbit pre-immune sera or rabbit sera of unrelated vaccinations served as negative controls. After washing binding of rabbit IgG to the immobilized IgE was detected using a HRP-labelled anti-rabbit IgG antibody (DAKO; 1:2500 in dilution buffer). Signals (OD) were detected using TMB (KEMENTEC) as substrate.

In addition to the IgE titers, the anti-peptide titers of the immune sera were analyzed. The free peptides (corresponding to the epitopes integrated in the AAV capsid or coupled to LPH) were covalently immobilized in a 96-well plate (REACTI-BIND™ Amine-binding, Maleic Anhydride Activated Plates; PIERCE) as described above. After blocking of the wells, immobilized peptides were incubated with serial dilutions of the immune sera in dilution buffer (PBS with 1% skim milk, 1% BSA, 0.1% Tween-20) for 1 h at 37°C. Rabbit pre-immune sera or rabbit sera of unrelated vaccinations served as negative controls. After washing binding of rabbit IgG to the immobilized CETP was detected using a HRP-labelled anti-rabbit IgG antibody (DAKO; 1:2500 in dilution buffer). Signals (OD) were detected using TMB (KEMENTEC) as substrate. Antibody titers were determined as described above

The anti-IgE titers of the immune sera are summarized in Table 18 below:

**Table 18: Mean anti-IgE titer of immunizations with AAV- vs. LPH-based IgE vaccines**

| **Vaccine** | **anti-IgE Titer 1^{st} Boost** | **anti-IgE Titer 2^{nd} Boost** | **anti-IgE Titer 3^{rd} Boost** |
|---|---|---|---|
| **AAV-Kricek** | 4750 | 20150 | 25460 |
| **AAV-Kricek*** | n.d. | 7950 | 27000 |
| **AAV-3DEpi3*** | 5000 | 18200 | 30140 |
| **AAV-Bind2** | 575 | 3075 | 7750 |
| **AAV-Flex** | 17200 | 40300 | 38100 |
| **LPH-Kricek** | n.d. | 1300 | 400 |
| **LPH-3DEpi3** | 705 | 1400 | 1600 |
| **LPH-Flex** | 15000 | 14000 | 23250 |
| **LPH-Bind2** | 0 | 0 | 0 |

| | | | |
|---|---|---|---|
| * AAV-based vaccines were used for the prime and 1^{st} boost immunization; 2^{nd} and 3^{rd} boost immunization were performed with the corresponding LPH-coupled peptide | | | |

Interestingly, vaccination of rabbits with LPH-Kricek, LPH-3DEpi3 or LPH-Bind2 failed to induce significant levels of antibodies against human IgE. The immunogenic properties of the peptide based vaccines are reflected by the high titers of peptide specific antibodies induced by the peptide vaccines (data not shown). However, these antibodies show no or only weak reaction with native human IgE. Only LPH-Flex induced reasonably high titers of antibodies specific for native human IgE. This is in clear contrast to the results obtained with the corresponding AAV-based vaccines like AAV-Kricek (Figure 14) which generate considerably higher human IgE specific antibody titers compared to the corresponding LPH-fusion constructs. This indicates that the fixed conformation of the corresponding IgE epitopes in the AAV2 capsid resembles the structure of the sequence within the IgE molecule in a better way than the LPH-coupled peptides. It should be noted that the generation of anti-human IgE antibodies in this animal model with rabbits does not overcome tolerance of the immune system to self-antigens.

### Literature

Arnold, G. S., Sasser, A. K., Stachler, M. D. and Bartlett, J. S. (2006) Mol Ther, 14, 97-106.
Asokan, A. and Samulski, R. J. (2006) Nat Biotechnol, 24, 158-60.
Asquith, D. L. and I. B. McInnes (2007). "Emerging cytokine targets in rheumatoid arthritis." Curr Opin Rheumatol 19(3): 246-51.
Aumailley, M., Gerl, M., Sonnenberg, A., Deutzmann, R. and Timpl, R. (1990) FEBS Lett, 262, 82-6.
Barassi, C., E. Soprana, et al. (2005). J Virol 79(11): 6848-58.
Bousquet, J., Cabrera, P., Berkman, N., Buhl, R., Holgate, S., Wenzel, S., Fox, H., Hedgecock, S., Blogg, M. and Cioppa, G. D. (2005) Allergy, 60, 302-8.
Chackerian, B., Lowy, D. R. et al. (1999). Proc Natl Acad Sci U S A 96(5): 2373-8.
Chackerian, B., Lowy, D. R. and Schiller, J. T. (2001) J Clin Invest, 108, 415-23.
Chatterjee, M. B., Foon, K. A. and Kohler, H. (1994) Cancer Immunology Immunotherapy, 38, 75-82.
Cook, J. P., Henry, A. J., McDonnell, J. M., Owens, R. J., Sutton, B. J. and Gould, H. J. (1997) Biochemistry, 36, 15579-88.
Corpet, F. (1988) Nucleic Acids Res, 16, 10881-90.
Dean, D. A., Strong, D. D. and Zimmer, W. E. (2005) Gene Ther, 12, 881-90.
Gamsjaeger, R., C. K. Liew, et al. (2007). Trends Biochem Sci 32(2): 63-70.
Garman, S. C., Wurzburg, B. A., Tarchevskaya, S. S., Kinet, J. P. and Jardetzky, T. S. (2000) Nature, 406, 259-66.
Girod, A., Ried, M., Wobus, C., Lahm, H., Leike, K., Kleinschmidt, J., Deleage, G. and Hallek, M. (1999) Nat Med, 5, 1438.
Grifman, M., Trepel, M., Speece, P., Gilbert, L. B., Arap, W., Pasqualini, R. and Weitzman, M. D. (2001) Mol Ther, 3, 964-75.
Helm, B., Kebo, D., Vercelli, D., Glovsky, M. M., Gould, H., Ishizaka, K., Geha, R. and Ishizaka, T. (1989) Proc Natl Acad Sci USA, 86, 9465-9.
Helm, B., Marsh, P., Vercelli, D., Padlan, E., Gould, H. and Geha, R. (1988) Nature, 331, 180-3.
Huttner, N. A., Girod, A., Perabo, L., Edbauer, D., Kleinschmidt, J. A., Buning, H. and Hallek, M. (2003) Gene Ther, 10, 2139-47.
Jefferis, R. (1993) Immunol Today, 14, 119-21.
Jerne, N. K. (1974) Ann Immunol (Paris), 125C, 373-89.
Jerne, N. K., Roland, J. and Cazenave, P. A. (1982) Embo J, 1, 243-7.
Kay, M. A., Glorioso, J. C. and Naldini, L. (2001) Nat Med, 7, 33-40.
Kern, A., Schmidt, K., Leder, C., Muller, O. J., Wobus, C. E., Bettinger, K., Von der Lieth, C. W., King, J. A. and Kleinschmidt, J. A. (2003) J Virol, 77, 11072-81.
Klenerman, P., Tolfvenstam, T., Price, D. A., Nixon, D. F., Broliden, K. and Oxenius, A. (2002) Pathol Biol (Paris), 50, 317-25.
Kricek, F., Ruf, C., Rudolf, M. P., Effenberger, F., Mayer, P. and Stadler, B. M. (1999) Int Arch Allergy Immunol, 118, 222-3.
Laity, J. H., B. M. Lee, et al. (2001). Curr Opin Struct Biol 11(1): 39-46.
Laughlin, C. A., Tratschin, J. D., Coon, H. and Carter, B. J. (1983) Gene, 23, 65-73.
Levy, D. A. and Chen, J. (1970) N Engl J Med, 283, 541-2.
Li, Q., Cao, C., Chackerian, B., Schiller, J., Gordon, M., Ugen, K. E. and Morgan, D. (2004) BMC Neurosci, 5, 21.
Lieber, A. (2003) Nat Biotechnol, 21, 1011-3.
Lux, K., Goerlitz, N., Schlemminger, S., Perabo, L., Goldnau, D., Endell, J., Leike, K., Kofler, D. M., Finke, S., Hallek, M. and Buning, H. (2005) J Virol, 79, 11776-87.
Maheshri, N., Koerber, J. T., Kaspar, B. K. and Schaffer, D. V. (2006) Nat Biotechnol, 24, 198-204.
Misumi, S., D. Nakayama, et al. (2006). J Immunol 176(1): 463-71.
Moskalenko, M., Chen, L., van Roey, M., Donahue, B. A., Snyder, R. O., McArthur, J. G. and Patel, S. D. (2000) J Virol, 74, 1761-6.
Muller, O. J., Kaul, F., Weitzman, M. D., Pasqualini, R., Arap, W., Kleinschmidt, J. A. and Trepel, M. (2003) Nat Biotechnol, 21, 1040-6.
Nicklin, S. A., Buening, H., Dishart, K. L., de Alwis, M., Girod, A., Hacker, U., Thrasher, A. J., Ali, R. R., Hallek, M. and Baker, A. H. (2001) Mol Ther, 4, 174-81.
Nygren, P. A. and Skerra, A. (2004) J Immunol Methods, 290, 3-28.
Opie, S. R., Warrington, K. H., Jr., Agbandje-McKenna, M., Zolotukhin, S. and Muzyczka, N. (2003) J Virol, 77, 6995-7006.
Parker, K. C., M. A. Bednarek, et al. (1994). J Immunol 152(1): 163-75.
Perabo, L. (2003) In Institut für BiochemieLMU, München, pp. 1-121.
Perabo, L., Buning, H., Kofler, D. M., Ried, M. U., Girod, A., Wendtner, C. M., Enssle, J. and Hallek, M. (2003) Mol Ther, 8, 151-7.
Perabo, L., Endell, J., King, S., Lux, K., Goldnau, D., Hallek, M. and Buning, H. (2006a) J Gene Med, 8, 155-62.
Perabo, L., Goldnau, D., White, K., Endell, J., Boucas, J., Humme, S., Work, L. M., Janicki, H., Hallek, M., Baker, A. H. and Buning, H. (2006b) J Virol, 80, 7265-9.
Pfeifer, A. and Verma, I. M. (2001) Annu Rev Genomics Hum Genet, 2, 177-211.
Presta, L., Shields, R., O'Connell, L., Lahr, S., Porter, J., Gorman, C. and Jardieu, P. (1994) J Biol Chem, 269, 26368-73.
Ried, M. U., Girod, A., Leike, K., Buning, H. and Hallek, M. (2002) J Virol, 76, 4559-66.
Riemer, A. B., Untersmayr, E., Knittelfelder, R., Duschl, A., Pehamberger, H., Zielinski, C. C., Scheiner, O. and Jensen-Jarolim, E. (2007) Cancer Res, 67, 3406-11.
Rittershaus, C. W., Miller, D. P., Thomas, L. J., Picard, M. D., Honan, C. M., Emmett, C. D., Pettey, C. L., Adari, H., Hammond, R. A., Beattie, D. T., Callow, A. D., Marsh, H. C. and Ryan, U. S. (2000) Arterioscler Thromb Vasc Biol, 20, 2106-12.
Rudolf, M. P., Vogel, M., Kricek, F., Ruf, C., Zurcher, A. W., Reuschel, R., Auer, M., Miescher, S. and Stadler, B. M. (1998) J Immunol, 160, 3315-21.
Rudolf, M. P., Zuercher, A. W., Nechansky, A., Ruf, C., Vogel, M., Miescher, S. M., Stadler, B. M. and Kricek, F. (2000) J Immunol, 165, 813-9.
Ruffing, M., Heid, H. and Kleinschmidt, J. A. (1994) J Gen Virol, 75 (Pt 12), 3385-92.
Shi, W., Arnold, G. S. and Bartlett, J. S. (2001) Hum Gene Ther, 12, 1697-711.
Shi, W. and Bartlett, J. S. (2003) Mol Ther, 7, 515-25.
Shi, X., Fang, G., Shi, W. and Bartlett, J. S. (2006) Hum Gene Ther, 17, 353-61.
Smolen, J. S. and Steiner, G. (2003) Nat Rev Drug Discov, 2, 473-88.
Stachler, M. D. and Bartlett, J. S. (2006) Gene Ther, 13, 926-31.
Stadler, B. M., Zurcher, A. W., Miescher, S., Kricek, F. and Vogel, M. (1999) Int Arch Allergy Immunol, 118, 119-21.
Summerford, C., Bartlett, J. S. and Samulski, R. J. (1999) Nat Med, 5, 78-82.
Theiss, H. D., Kofler, D. M., Buning, H., Aldenhoff, A. L., Kaess, B., Decker, T., Baumert, J., Hallek, M. and Wendtner, C. M. (2003) Exp Hematol, 31, 1223-9.
Uversky V.N., Fernández A. and Fink A. L. (2006) chapter 1, 1-20 in: Protein Reviews Volume 4, editor: M. Zouhair Atassi: Protein Misfolding, Aggregation, and Conformational Disease, Part A: Protein Aggregation and Conformational Disease; Springer.
Varela, F. J. and Coutinho, A. (1991) Immunol Today, 12, 159-66.
Vogel, M., Miescher, S., Kuhn, S., Zurcher, A. W., Stadler, M. B., Ruf, C., Effenberger, F., Kricek, F. and Stadler, B. M. (2000) J Mol Biol, 298, 729-35.
Vogel, M., Tschopp, C., Bobrzynski, T., Fux, M., Stadler, M. B., Miescher, S. M. and Stadler, B. M. (2004) J Mol Biol, 341, 477-89.
Warrington, K. H., Jr., Gorbatyuk, O. S., Harrison, J. K., Opie, S. R., Zolotukhin, S. and Muzyczka, N. (2004) J Virol, 78, 6595-609.
Waterkamp, D. A., Muller, O. J., Ying, Y., Trepel, M. and Kleinschmidt, J. A. (2006) J Gene Med, 8, 1307-19.
White, S. J., Nicklin, S. A., Buning, H., Brosnan, M. J., Leike, K., Papadakis, E. D., Hallek, M. and Baker, A. H. (2004) Circulation, 109, 513-9.
Work, L. M., Buning, H., Hunt, E., Nicklin, S. A., Denby, L., Britton, N., Leike, K., Odenthal, M., Drebber, U., Hallek, M. and Baker, A. H. (2006) Mol Ther, 13, 683-93.
Work, L. M., Nicklin, S. A., Brain, N. J., Dishart, K. L., Von Seggern, D. J., Hallek, M., Buning, H. and Baker, A. H. (2004) Mol Ther, 9, 198-208.
Wu, P., Xiao, W., Conlon, T., Hughes, J., Agbandje-McKenna, M., Ferkol, T., Flotte, T. and Muzyczka, N. (2000) J Virol, 74, 8635-47.
Wu, Z., Asokan, A., Grieger, J. C., Govindasamy, L., Agbandje-McKenna, M. and Samulski, R. J. (2006) J Virol, 80, 11393-7.
Xiao, X., Li, J. and Samulski, R. J. (1998) J Virol, 72, 2224-32.

## Claims

1. A multimeric structure comprising a parvovirus structural protein which comprises an amino acid insertion
wherein the amino acid insertion is directly C-terminally of amino acid G₄₅₃ in the sequence af AAV-2 or the corresponding amino acid of any other parvovirus, and
wherein the structural protein with the insertion is capable of particle formation, and
wherein the insertion
(a) has a length of 4 to 30 amino acids,
and/or
(b) is an epitope

2. The multimeric structure of claim 1,
a) wherein the insertion is located on the surface of the capsid formed by the structural proteins;
b) wherein the parvovirus is an adeno-associated virus, preferably selected from the group consisting of AAV-1, AAV-2, AAV-3b, AAV-4, AAV-5, AAV-6, AAV-7, AAV-8, AAV-9, AAV-10, AAV-11, AAV-12 and b-AAV, especially selected from the group consisting of AAV-1, AAV-2, AAV-5 and AAV-8;
c) wherein the parvovirus is selected from the group consisting of feline panleukopenia virus (FPV), canine parvovirus (CPV), B19, goose parvovirus (GPV) and minute virus of mice (MVM), especially FPV, CPV and B19;
d) wherein the amino acid insertion has a length of 5 to 20, most preferably 5 to 15 amino acids; and/or
e) wherein the amino acid insertion is an epitope.

3. The multimeric structure of claim 2,
a) wherein the epitope is a B-cell epitope;
b) wherein the insertion is a tolerogen-derived epitope;
c) wherein the insertion is a part of a protein selected from the group consisting of a tumor antigen, a misfolded protein, a serum protein, a membrane protein, a viral receptor, a TNF-family member and an interleukin; and/or
d) wherein the tolerogen-derived epitope is derived from a protein from the group consisting of CETP, CD20, acetylcholine receptors, IL13R, EGFR, IgE, Melan A, HMW MAA, CA125, Her2/NEU, CCR5, L1 cell adhesion molecule, VEGF, EGFR, CD20, TNF-α, IL-6, IL9, IL-13, IL-17, and β-amyloid, particularly wherein the tolerogen-derived epitope is selected from the group consisting of VNLTWSRASG, EFCINHRGYWVCGD, EDGQVMDVDLS, EKQRNGTLT, TYQCRVTHPHLPRALMR, RHSTTQPRKTKGSG, DSNPRGVSAYLSR, TITCLVVDLAPSK, KTKGSGFFVF, THPHLPRALMRS, GETYQCRVTHPHLPRALMRSTTK, LPRALMRS, INHRGYWV, CDAGSVRTNAPD AKAVSNLTESRSESLQS, SLTGDEFKKVLET, REAVAYRFEED, INPEIITLDG, DISVTGAPVITATYL, DISVTGAPVITA, PKTVSNLTESSSESVQS, SLMGDEFKAVLET, QHSVAYTFEED, INPEIITRDG, DISLTGDPVITASYL, DISLTGDPVITA, DQSIDFEIDSA, KNVSEDLPLPTFSPTLLGDS, KNVSEDLPLPT, CDSGRVRTDAPD, FPEHLLVDFLQSLS, DAEFRHDSG, HYAAAQWDFGNTMCQL, YAAQWDFGNTMCQ, RSQKEGLHYT, SSRTPSDKPVAHVVANPQAE, SRTPSDKPVAHVVANP, SSRTPSDKP, NADGNVDYHMNSVP, DGNVDYHMNSV, RSFKEFLQSSLRALRQ; FKEFLQSSLRA, and QMWAPQWGPD.

4. The multimeric structure of claim 1 or 2,
a) wherein the amino acid insertion brings about an increase in the transducing activity of the parvovirus, particularly
i) wherein the amino acid insertion mediates binding of the structural protein to a cell membrane receptor,
ii) wherein the amino acid insertion is a ligand for a given receptor, and/or
iii) wherein the amino acid insertion contains an RGD motif, especially wherein the amino acid insertion is ACDCRGDCFCA;
b) wherein the amino acid insertion brings about an alteration in a chromatographic property of the structural protein, particularly wherein the amino acid insertion is a tag useful for binding to a ligand; and/or
c) wherein the insertion has aC-terminal linker, preferably wherein linker sequence is selected to form a Zinc-finger (Zn-finger) such as C₂H₂, C₄, and C₂HC, particularly wherein the linker comprises at least one Cys N-terminal and at least one Cys C-terminal of the insertion.

5. The multimeric structure of claim 1 to 4, wherein the parvovirus structural protein comprises one or more further mutation(s) at a site different from the site directly C-terminally of amino acid G₄₅₃ in the sequence of AAV-2 or the corresponding amino acid of any other parvovirus, independently selected from a point mutation, an internal deletion, an N-terminal deletion, an insertion and a substitution, preferably one further insertion.

6. The multimeric structure of claim 5,
a) wherein the further mutation is an insertion defined in analogy to the first insertion of claim 2 a), 2 d), 3a) to 3d) and/or 4a), and/or wherein the structural protein with the further insertion is capable of particle formation and/or wherein the insertion has a length of 4 to about 30 amino acids and/or
b) wherein the further mutation is an insertion of a B-cell epitope or a cytotoxic T-cell epitope.

7. The multimeric structure of claim 5, wherein the further mutation reduces the transducing activity of the particle for a given target cell by at least 50%, preferably at least 80%, especially at least 95%, particularly wherein the further mutation is an mutation inactivating the HSPG binding site located in the proximity of the sequence LQRGN₅₈₇ RQAAT (site I-587), in particular wherein the further mutation is a deletion or substitution of R₅₈₅ and/or R₅₈₈ of AAV-2 or the corresponding amino acids of other parvoviruses, preferably amino acid substitutions selected from the group consisting of R₄₈₄A, R₄₈₇A, R₄₈₇G, K₅₃₂A, K₅₃₂A, K₅₃₂D, R₅₈₅A, R₅₈₅S, R₅₈₅Q, R₅₈₈A and R₅₈₈T, especially R₅₈₅A and R₅₈₈A for AAV-2, and K₅₃₁A and K₅₃₁E for AAV-6.

8. The multimeric structure of claim 5, wherein the further mutation reduces the ability to induce a B-cell response against a parvovirus-specific epitope and/or mimotope.

9. A library of structural proteins, wherein the library comprises a set of different parvoviral structural proteins according to any of claims 1 to 8, particularly wherein the library is coupled and/or particularly wherein the library of the present invention has a multiplicity of parvoviral mutants of greater than 10³, preferably greater than 10⁵, more preferably greater than 10⁶, especially greater than 10⁷.

10. The multimeric structure of any of claims 1 to 8, wherein the multimeric structure is a capsomere, a virus like particle or a virus and/or particularly wherein the structure is an aggregate of at least about 5, preferably at least about 10, more preferably at least about 30, most preferably at least about 60 structural proteins.

11. A nucleic acid encoding a structural protein of any of claims 1 to 8.

12. A vector comprising a nucleic acid of claim 11.

13. A library of vectors, wherein the library comprises a set of different vectors according to claim 12, particularly wherein genotype and phenotype of virion particles are coupled.

14. A virus comprising a nucleic acid of claim 11 or a vector of claim 12.

15. An isolated cell comprising a nucleic acid of claim 11 or a vector of claim 12.

16. A method for altering the tropism of a parvovirus, the method comprising the steps of:
a) co-expressing parvoviral helper and vector functions, wherein the helper function expresses a parvoviral structural protein according to any of claims 1 to 8 under conditions that enable parvovirus formation, and
b) isolating the parvovirus

17. A method for displaying an epitope on the surface of a parvovirus, the method comprising the steps of:
a) expressing the nucleic acid according to claim 11 under suitable conditions, and
b) isolating the expressed structural protein of step a).

18. Medicament comprising at least one parvovirus structural protein according to any of claims 1 to 8 and/or a nucleic acid according to claim 11, preferably at least one multimeric structure according to claim 10.

19. Medicament of claim 18 for use as a vaccine or as a gene transfer vector.

20. Use of at least one parvovirus structural protein according to any of claims 1 to 8 and/or a nucleic acid according to claim 11, preferably at least one multimeric structure according to claim 10 for the manufacture of a vaccine or a gene transfer vector.

21. The medicament of claim 18 or 19 for the treatment and/or prevention of
a) an allergic disease and/or asthma wherein at the amino acid insertion directly C-terminally of amino acid G₄₅₃ in the sequence of AAV-2 or the corresponding amino acid of any other parvovirus, and preferably at at least one further insertion site, more preferably at insertion site directly N- or C-terminal of one amino acid in the sequence YKQIS₂₆₁ SQSGA (I-261), directly N- or C-terminal of one amino acid in the sequence EEKFF₅₃₄ PQSGV (I-534), directly N- or C-terminal of one amino acid in the sequence RTTNP₅₇₀ VATEQ (I-570), directly N- or C-terminal of one amino acid in the sequence NPVAT₅₇₃ EQYGS (I-573) or directly N- or C-terminal of one amino acid in the sequence LQRGN₅₈₇ RQAAT (I-587), especially at the amino acid insertion directly C-terminally of amino acid G₄₅₃ in the sequence of AAV-2 or the corresponding amino acid of any other parvovirus and directly N- or C-terminal of one amino acid in the sequence LQRGN₅₈₇ RQAAT (I-587), preferably of AAV1, AAV2 or AAV-6, the structural protein of a parvovirus comprises an anti-idiotypic epi-/mimotope of an anti-IgE antibody, and/or an IgE epi-/mimotope, particularly a mimotope of sequence of EFCINHRGYWVCGD, with the first G, W and V being conserved and cysteine residues C mediating a circular form of the peptide via disulfide bridging, VNLTWSRASG or INHRGYWV or particularly an epitope selected from the group consisting of EKQRNGTLT, EDGQVMDVDLS, TYQCRVTHPHLPRALMR, RHSTTQPRKTKGSG, DSNPRGVSAYLSR, TITCLWDLAPSK, KTKGSGFFVF, THPHLPRALMRS, GETYQCRVTHPHLPRALMRSTTK, LPRALMRS and a functionally active variant thereof;
b) Alzheimer's disease wherein at the amino acid insertion directly C-terminally of amino acid G₄₅₃ in the sequence of AAV-2 or the corresponding amino acid of any other parvovirus, and preferably at at least one further insertion site, more preferably at insertion site directly N- or C-terminal of one amino acid in the sequence YKQIS₂₆₁ SQSGA (I-261), directly N- or C-terminal of one amino acid in the sequence EEKFF₅₃₄ PQSGV (I-534), directly N- or C-terminal of one amino acid in the sequence RTTNP₅₇₀ VATEQ (I-570), directly N- or C-terminal of one amino acid in the sequence NPVATF₅₇₃ EQYGS (I-573) or directly N- or C-terminal of one amino acid in the sequence LQRGN₅₈₇ RQAAT (I-587), especially at the amino acid insertion directly C-terminally of amino acid G₄₅₃ in the sequence of AAV-2 or the corresponding amino acid of any other parvovirus and directly N- or C-terminal of one amino acid in the sequence LQRGN₅₈₇ RQAAT (I-587), preferably of AAV1, AAV2 or AAV-6, the structural protein of a parvovirus comprises a β-amyloid epitope or mimotope, particularly comprising or having the sequence DAEFRHDSG or a functionally active variant thereof;
c) atherosclerosis wherein at the amino acid insertion directly C-terminally of amino acid G₄₅₃ in the sequence of AAV-2 or the corresponding amino acid of any other parvovirus, and preferably at at least one further insertion site, more preferably at insertion site directly N- or C-terminal of one amino acid in the sequence YKQIS₂₆₁ SQSGA (I-261), directly N- or C-terminal of one amino acid in the sequence EEKFF₅₃₄ PQSGV (I-534), directly N- or C-terminal of one amino acid in the sequence RTTNP₅₇₀ VATEQ (I-570), directly N- or C-terminal of one amino acid in the sequence NPVAT₅₇₃ EQYGS (I-573) or directly N- or C-terminal of one amino acid in the sequence LQRGN₅₈₇ RQAAT (I-587), especially at the amino acid insertion directly C-terminally of amino acid G₄₅₃ in the sequence of AAV-2 or the corresponding amino acid of any other parvovirus and directly N- or C-terminal of one amino acid in the sequence LQRGN₅₈₇ RQAAT (I-587), especially I-453 and I-587, preferably of AAV1, AAV2 or AAV-6, the structural protein of a parvovirus comprises a CETP epitope or mimotope, particularly an epitope selected from the group consisting of PKTVSNLTESSSESVQS, SLMGDEFKAVLET, QHSVAYTFEED, INPEIITRDG, DISLTGDPVITASYL, DISLTGDPVITA, DG2SIDFEIDSA, KNVSEDLPLPTFSPTLLGDS, KNVSEDLPLPT, CDSGRVRTDAPD, FPEHLLVDFLQSLS and a functionally active variant thereof;
d) a tumor disease whereas wherein at the amino acid insertion directly C-terminally of amino acid G₄₅₃ in the sequence of AAV-2 or the corresponding amino acid of any other parvovirus, and preferably at at least one further insertion site, more preferably at insertion site directly N- or C-terminal of one amino acid in the sequence YKQIS₂₆₁ SQSGA (I-261), directly N- or C-terminal of one amino acid in the sequence EEKFF₅₃₄ PQSGV (I-534), directly N- or C-terminal of one amino acid in the sequence RTTNP₅₇₀ VATEQ (I-570), directly N- or C-terminal of one amino acid in the sequence NPVAT₅₇₃ EQYGS (I-573) or directly N- or C-terminal of one amino acid in the sequence LQRGN₅₈₇ RQAAT (I-587), especially at the amino acid insertion directly C-terminally of amino acid G₄₅₃ in the sequence of AAV-2 or the corresponding amino acid of any other parvovirus and directly N- or C-terminal of one amino acid in the sequence LQRGN₅₈₇ RQAAT (I-587), preferably of AAV1, AAV2 or AAV-6, the structural protein of a parvovirus comprises a tumor antigen, particularly a HER2/neu epitope or mimotope, especially the epitope QMWAPQWGPD or a functionally active variant thereof;
e) an autoimmune disease and/or a chronic inflammatory disease, preferably rheumatoid arthritis and/or Crohn's disease, wherein at the amino acid insertion directly C-terminally of amino acid G₄₅₃ in the sequence of AAV-2 or the corresponding amino acid of any other parvovirus, and preferably at at least one further insertion site, more preferably at insertion site directly N- or C-terminal of one amino acid in the sequence YKQIS₂₆₁ SQSGA (I-261), directly N- or C-terminal of one amino acid in the sequence EEKFF₅₃₄ PQSGV (I-534), directly N- or C-terminal of one amino acid in the sequence RTTNP₅₇₀ VATEQ (I-570), directly N- or C-terminal of one amino acid in the sequence NPVAT₅₇₃ EQYGS (I-573) or directly N- or C-terminal of one amino acid in the sequence LQRGN₅₈₇ RQAAT (I-587), especially at the amino acid insertion directly C-terminally of amino acid G₄₅₃ in the sequence of AAV-2 or the corresponding amino acid of any other parvovirus and directly N- or C-terminal of one amino acid in the sequence LQRGN₅₈₇ RQAAT (I-587), preferably of AAV1, AAV2 or AAV-6, the structural protein of a parvovirus comprises an epitope or mimotope of a cytokine, preferably of TNF-α, IL-6 and/or IL-17, especially an epitope selected from the group consisting of SSRTPSDKPVAHVVANPQAE, SRTPSDKPVAHVVANP, SSRTPSDKP, NADGNVDYHMNSVP, DGNVDYHMNSV, RSFKEFLQSSLRALRQ, FKEFLQSSLRA or or a functionally active variant thereof, or
f) an infectious disease, preferably an HIV infection, wherein at the amino acid insertion directly C-terminally of amino acid G₄₅₃ in the sequence of AAV-2 or the corresponding amino acid of any other parvovirus, and preferably at at least one further insertion site, more preferably at insertion site directly N- or C-terminal of one amino acid in the sequence YKQIS₂₆₁ SQSGA (I-261), directly N- or C-terminal of one amino acid in the sequence EEKFF₅₃₄ PQSGV (I-534), directly N- or C-terminal of one amino acid in the sequence RTTNP₅₇₀ VATEQ (I-570), directly N- or C-terminal of one amino acid in the sequence NPVAT₅₇₃ EQYGS (I-573) or directly N- or C-terminal of one amino acid in the sequence LQRGN₅₈₇ RQAAT (I-587), especially at the amino acid insertion directly C-terminally of amino acid G₄₅₃ in the sequence of AAV-2 or the corresponding amino acid of any other parvovirus and directly N- or C-terminal of one amino acid in the sequence LQRGN₅₈₇ RQAAT (I-587), preferably of AAV1, AAV2 or AAV-6, the structural protein of a parvovirus comprises an epitope or mimotope of a viral receptor, preferably of CCR5, especially an epitope selected from the group consisting of HYAAAQWDFGNTMCQL, YAAQWDFGNTMCQ, RSQKEGLHYT or a functionally active variant thereof.

## Patentansprüche

1. Multimere Struktur umfassend ein Parvovirus-Strukturprotein, das eine Aminosäureinsertion umfasst,
wobei die Aminosäureinsertion direkt C-terminal der Aminosäure G₄₅₃ in der Sequenz von AAV-2 oder der entsprechenden Aminosäure eines beliebigen anderen Parvovirus ist und
wobei das Strukturprotein mit der Insertion zur Partikelbildung fähig ist und wobei die Insertion
(a) eine Länge von 4 bis 30 Aminosäuren aufweist,
und/oder
(b) ein Epitop ist.

2. Multimere Struktur nach Anspruch 1,
a) wobei die Insertion auf der Oberfläche des Kapsids lokalisiert ist, das von den Strukturproteinen gebildet wird;
b) wobei das Parvovirus ein Adeno-assoziertes Virus ist, vorzugsweise ausgewählt aus der Gruppe bestehend aus AAV-1, AAV-2, AAV-3b, AAV-4, AAV-5, AAV-6, AAV-7, AAV-8, AAV-9, AAV-10, AAV-11, AAV-12 und b-AAV, besonders ausgewählt aus der Gruppe bestehend aus AAV-1, AAV-2, AAV-5 und AAV-8;
c) wobei das Parvovirus ausgewählt ist aus der Gruppe bestehend aus felinem Panleukopenie-Virus (FPV), caninem Parvovirus (CPV), B19, Gänse-Parvovirus (GPV) und Minute Virus von Mäusen (MVM), besonders FPV, CPV und B19;
d) wobei die Aminosäureinsertion eine Länge von 5 bis 20 Aminosäuren, am meisten bevorzugt 5 bis 15 Aminosäuren aufweist; und/oder
e) wobei die Aminosäureinsertion ein Epitop ist.

3. Multimere Struktur nach Anspruch 2,
a) wobei das Epitop ein B-Zell-Epitop ist;
b) wobei die Insertion ein Epitop ist, das von einem Tolerogen stammt;
c) wobei die Insertion ein Teil eines Proteins ist ausgewählt aus der Gruppe bestehend aus einem Tumorantigen, einem fehlgefalteten Protein, einem Serumprotein, einem Membranprotein, einem viralen Rezeptor, einem Mitglied der TNF-Familie und einem Interleukin; und/oder
d) wobei das Epitop, das von einem Tolerogen stammt, von einem Protein stammt aus der Gruppe bestehend aus CETP, CD20, Acetylcholinrezeptoren, IL13R, EGFR, IgE, Melan A, HMW MAA, CA125, Her2/NEU, CCR5, L1-Zelladhäsionsmolekül, VEGF, EGFR, CD20, TNF-α, IL-6, IL9, IL-13, IL-17, und β-Amyloid, insbesondere wobei das Epitop, das von einem Tolerogen stammt, ausgewählt ist aus der Gruppe bestehend aus VNLTWSRASG, EFCINHRGYWVCGD, EDGQVMDVDLS, EKQRNGTLT, TYQCRVTHPHLPRALMR, RHSTTQPRKTKGSG, DSNPRGVSAYLSR, TITCLVVDLAPSK, KTKGSGFFVF, THPHLPRALMRS, GETYQCRVTHPHLPRALMRSTTK, LPRALMRS, INHRGYWV, CDAGSVRTNAPD AKAVSNLTESRSESLQS, SLTGDEFKKVLET, REAVAYRFEED, INPEIITLDG, DISVTGAPVITATYL, DISVTGAPVITA, PKTVSNLTESSSESVQS, SLMGDEFKAVLET, QHSVAYTFEED, INPEIITRDG, DISLTGDPVITASYL, DISLTGDPVITA, DQSIDFEIDSA, KNVSEDLPLPTFSPTLLGDS, KNVSEDLPLPT, CDSGRVRTDAPD, FPEHLLVDFLQSLS, DAEFRHDSG, HYAAAQWDFGNTMCQL, YAAQWDFGNTMCQ,
RSQKEGLHYT, SSRTPSDKPVAHVVANPQAE, SRTPSDKPVAHVVANP, SSRTPSDKP, NADGNVDYHMNSVP, DGNVDYHMNSV, RSFKEFLQSSLRALRQ; FKEFLQSSLRA und QMWAPQWGPD.

4. Multimere Struktur nach Anspruch 1 oder 2,
a) wobei die Aminosäureinsertion eine Erhöhung der Transduktionsaktivität des Parvovirus bewirkt, insbesondere
i) wobei die Aminosäureinsertion die Bindung des Strukturproteins an einen Zellmembranrezeptor vermittelt,
ii) wobei die Aminosäureinsertion ein Ligand für einen gegebenen Rezeptor ist und/oder
iii) wobei die Aminosäureinsertion ein RGD-Motiv enthält, besonders wobei die Aminosäureinsertion ACDCRGDCFCA ist;
b) wobei die Aminosäureinsertion eine Veränderung einer chromatographischen Eigenschaft des Strukturproteins bewirkt, insbesondere wobei die Aminosäureinsertion ein Tag ist, das zur Bindung an einen Liganden verwendet werden kann, und/oder
c) wobei die Insertion einen C-terminalen Linker aufweist, vorzugsweise wobei die Linkersequenz ausgewählt ist aus einem Zinkfinger (Zn-Finger) wie zum Beispiel C₂H₂, C₄ und C₂HC, insbesondere wobei der Linker mindestens ein Cys N-terminal und mindestens ein Cys C-terminal der Insertion umfasst.

5. Multimere Struktur nach Anspruch 1 bis 4, wobei das Parvovirus-Strukturprotein eine oder mehrere weitere Mutation(en) an einer anderen Stelle als der Stelle direkt C-terminal der Aminosäure G₄₅₃ in der Sequenz von AAV-2 oder der entsprechenden Aminosäure eines beliebigen anderen Parvovirus, unabhängig voneinander ausgewählt aus der Gruppe von einer Punktmutation, einer internen Deletion, einer N-terminalen Deletion, einer Insertion und einer Substitution, vorzugweise eine weitere Insertion, umfasst.

6. Multimere Struktur nach Anspruch 5,
a) wobei die weitere Mutation eine Insertion ist, definiert in Analogie zur ersten Insertion von Anspruch 2 a), 2 d), 3 a) bis 3d) und/oder 4a), und/oder wobei das Strukturprotein mit der weiteren Insertion zur Partikelbildung fähig ist und/oder wobei die Insertion eine Länge von 4 bis ungefähr 30 Aminosäuren aufweist und/oder
b) wobei die weitere Mutation eine Insertion eines B-Zell-Epitops oder eines Epitops einer zytotoxischen T-Zelle ist.

7. Multimere Struktur nach Anspruch 5, wobei die weitere Mutation die Transduktionsaktivität des Partikels für eine gegebene Zielzelle um mindestens 50%, vorzugsweise mindestens 80%, besonders mindestens 95% reduziert, insbesondere wobei die weitere Mutation eine Mutation ist, die die HSPG-Bindungsstelle, die in der Nähe der Sequenz LQRGN₅₈₇RQAAT (Stelle I-587) lokalisiert ist, inaktiviert, insbesondere wobei die weitere Mutation eine Deletion oder Substitution von R₅₈₅und/oder R₅₈₈ von AAV-2 oder der entsprechenden Aminosäuren eines beliebigen anderen Parvovirus ist, vorzugsweise Aminosäuresubstitutionen ausgewählt aus der Gruppe bestehend aus R₄₈₄A, R₄₈₇A, R₄₈₇G, K₅₃₂A, K₅₃₂A, K₅₃₂D, R₅₈₅A, R₅₈₅S, R₅₈₅Q, R₅₈₈A und R₅₈₈T, besonders R₅₈₅A und R₅₈₈A für AAV-2 sowie K₅₃₁A und K₅₃₁ E für AAV-6.

8. Multimere Struktur nach Anspruch 5, wobei die weitere Mutation die Fähigkeit zur Induktion einer B-Zell-Antwort gegen ein Parvovirusspezifisches Epitop und/oder Mimotop reduziert.

9. Bibliothek von Strukturproteinen, wobei die Bibliothek einen Satz von verschiedenen parvoviralen Strukturproteinen gemäß einen beliebigen der Ansprüche 1 bis 8 umfasst, insbesondere wobei die Bibliothek gekoppelt ist und/oder insbesondere wobei die Bibliothek der vorliegenden Erfindung eine Multiplizität der parvoviralen Mutanten von mehr als 10³, vorzugsweise mehr als 10⁵, weiter vorzugsweise mehr als 10⁶, besonders mehr als 10⁷ aufweist.

10. Multimere Struktur nach einem beliebigen der Ansprüche 1 bis 8, wobei die multimere Struktur ein Kapsomer, ein Virus-ähnliches Partikel oder ein Virus ist und/oder vorzugsweise wobei die Struktur ein Aggregat von mindestens ungefähr 5, vorzugsweise mindestens ungefähr 10, weiter vorzugsweise mindestens ungefähr 30, am meisten vorzugsweise mindestens ungefähr 60 Strukturproteinen ist.

11. Nukleinsäure kodierend für ein Strukturprotein nach einem beliebigen der Ansprüche 1 bis 8.

12. Vektor umfassend die Nukleinsäure von Anspruch 11.

13. Bibliothek von Vektoren, wobei die Bibliothek einen Satz verschiedener Vektoren gemäß Anspruch 12 umfasst, insbesondere wobei Genotyp und Phänotyp der Virionpartikel gekoppelt sind.

14. Virus umfassend eine Nukleinsäure nach Anspruch 11 oder einen Vektor nach Anspruch 12.

15. Isolierte Zelle umfassend eine Nukleinsäure nach Anspruch 11 oder einen Vektor nach Anspruch 12.

16. Verfahren zum Verändern des Tropismus eines Parvovirus, wobei das Verfahren die Schritte umfasst:
a) Co-Exprimieren von parvoviralen Helfer- und Vektorfunktionen, wobei die Helferfunktion ein parvovirales Strukturprotein gemäß einem beliebigen der Ansprüche 1 bis 8 unter Bedingungen, die die Parvovirusbildung ermöglichen, exprimiert und
b) Isolieren des Parvovirus.

17. Verfahren zum Zeigen (display) eines Epitops auf der Oberfläche eines Parvovirus, wobei das Verfahren die Schritte umfasst:
a) Exprimieren des Nukleinsäure gemäß Anspruch 11 unter geeigneten Bedingungen und
b) Isolieren des exprimierten Strukturproteins von Schritt a).

18. Arzneimittel umfassend mindestens ein Parvovirus-Strukturprotein gemäß einem beliebigen der Ansprüche 1 bis 8 und/oder eine Nukleinsäure gemäß Anspruch 11, vorzugsweise mindestens eine multimere Struktur gemäß Anspruch 10.

19. Arzneimittel nach Anspruch 18 zur Verwendung als ein Vakzin oder als ein Gentransfervektor.

20. Verwendung mindestens eines Parvovirus-Strukturproteins gemäß einem beliebigen der Ansprüche 1 bis 8 und/oder einer Nukleinsäure gemäß Anspruch 11, vorzugsweise einer multimeren Struktur gemäß Anspruch 10 zur Herstellung eines Vakzins oder eines Gentransfervektors.

21. Arzneimittel nach Anspruch 18 oder 19 zur Behandlung von und/oder Vorbeugung vor
a) einer allergischen Erkrankung und/oder Asthma, wobei das Strukturprotein eines Parvovirus an der Aminosäureinsertion direkt C-terminal zu Aminosäure G₄₅₃ in der Sequenz von AAV-2 oder der entsprechenden Aminosäure eines beliebigen anderen Parvovirus und vorzugsweise an mindestens einer weiteren Insertionsstelle, weiter vorzugsweise an der Insertionsstelle direkt N- oder C-terminal zu einer Aminosäure in der Sequenz YKQIS₂₆₁ SQSGA (I-261), direkt N- oder C-terminal zu einer Aminosäure in der Sequenz EEKFF₅₃₄ PQSGV (I-534), direkt N- oder C-terminal zu einer Aminosäure in der Sequenz RTTNP₅₇₀ VATEQ (I-570), direkt N- oder C-terminal zu einer Aminosäure in der Sequenz NPVAT₅₇₃ EQYGS (I-573) oder direkt N- oder C-terminal zu einer Aminosäure in der Sequenz LQRGN₅₈₇ RQAAT (I-587), besonders an der Aminosäureinsertion direkt C-terminal zu Aminosäure G₄₅₃ in der Sequenz von AAV-2 oder der entsprechenden Aminosäure eines beliebigen anderen Parvovirus und direkt N- oder C-terminal zu einer Aminosäure in der Sequenz LQRGN₅₈₇ RQAAT (I-587), vorzugsweise von AAV1, AAV2 oder AAV-6, ein anti-idiotypisches Epi-/Mimotop eines anti-IgE Antikörpers und/oder ein IgE Epi-/Mimotop, insbesondere ein Mimotop der Sequenz EFCINHRGYWVCGD, wobei die ersten G, W und V konserviert sind und Cysteinreste C eine zirkuläre Form des Peptids über Disulfidbrückenbindung vermitteln, VNLTWSRASG oder INHRGYWV oder insbesondere ein Epitop ausgewählt aus der Gruppe bestehend aus EKQRNGTLT, EDGQVMDVDLS, TYQCRVTHPHLPRALMR, RHSTTQPRKTKGSG, DSNPRGVSAYLSR, TITCLVVDLAPSK, KTKGSGFFVF, THPHLPRALMRS, GETYQCRVTHPHLPRALMRSTTK, LPRALMRS und einer funktionell aktiven Variante hiervon, umfasst;
b) Alzheimer-Erkrankung, wobei das Strukturprotein eines Parvovirus an der Aminosäureinsertion direkt C-terminal zu Aminosäure G₄₅₃ in der Sequenz von AAV-2 oder der entsprechenden Aminosäure eines beliebigen anderen Parvovirus, und vorzugsweise an mindestens einer weiteren Insertionsstelle, weiter vorzugsweise an der Insertionsstelle direkt N- oder C-terminal zu einer Aminosäure in der Sequenz YKQIS₂₆₁ SQSGA (I-261), direkt N- oder C-terminal zu einer Aminosäure in der Sequenz EEKFF₅₃₄ PQSGV (I-534), direkt N- oder C-terminal zu einer Aminosäure in der Sequenz RTTNP₅₇₀ VAIEQ (I-570), direkt N-oder C-terminal zu einer Aminosäure in der Sequenz NPVAT₅₇₃ EQYGS (I-573) oder direkt N- oder C-terminal zu einer Aminosäure in der Sequenz LQRGN₅₈₇ RQAAT (I-587), besonders an der Aminosäureinsertion direkt C-terminal zu Aminosäure G₄₅₃ in der Sequenz von AAV-2 oder der entsprechenden Aminosäure eines beliebigen anderen Parvovirus und direkt N- oder C-terminal zu einer Aminosäure in der Sequenz LQRGN₅₈₇ RQAAT (I-587), vorzugsweise von AAV1, AAV2 oder AAV-6, ein β-Amyloid-Epitop oder -Mimotop umfasst, insbesondere umfassend oder aufweisend die Sequenz DAEFRHDSG oder eine funktionell aktive Variante hiervon;
c) Arteriosklerose, wobei das Strukturprotein eines Parvovirus an der Aminosäureinsertion direkt C-terminal zu Aminosäure G₄₅₃ in der Sequenz von AAV-2 oder der entsprechenden Aminosäure eines beliebigen anderen Parvovirus, und vorzugsweise an mindestens einer weiteren Insertionsstelle, weiter vorzugsweise an der Insertionsstelle direkt N- oder C-terminal einer Aminosäure in der Sequenz YKQIS₂₆₁ SQSGA (I-261), direkt N- oder C-terminal zu einer Aminosäure in der Sequenz EEKFF₅₃₄ PQSGV (I-534), direkt N- oder C-terminal zu einer Aminosäure in der Sequenz RTTNP₅₇₀ VAIEQ (I-570), direkt N-oder C-terminal zu einer Aminosäure in der Sequenz NPVAT₅₇₃ EQYGS (I-573) oder direkt N- oder C-terminal zu einer Aminosäure in der Sequenz LQRGN₅₈₇ RQAAT (I-587), besonders an der Aminosäureinsertion direkt C-terminal zu Aminosäure G₄₅₃ in der Sequenz von AAV-2 oder der entsprechenden Aminosäure eines beliebigen anderen Parvovirus und direkt N- oder C-terminal zu einer Aminosäure in der Sequenz LQRGN₅₈₇ RQAAT (I-587), besonders I-453 und I-587, vorzugsweise von AAV1, AAV2 oder AAV-6, ein CETP-Epitop oder -Mimotop, insbesondere ein Epitop ausgewählt aus der Gruppe bestehend aus PKTVSNLTESSSESVQS, SLMGDEFKAVLET, QHSVAYTFEED, INPEIITRDG, DISLTGDPVITASYL, DISLTGDPVITA, DQSIDFEIDSA, KNVSEDLPLPTFSPTLLGDS, KNVSEDLPLPT, CDSGRVRTDAPD, FPEHLLVDFLQSLS und einer funktionell aktiven Variante hiervon, umfasst;
d) einer Tumorerkrankung, wobei das Strukturprotein eines Parvovirus an der Aminosäureinsertion direkt C-terminal zu Aminosäure G₄₅₃ in der Sequenz von AAV-2 oder der entsprechenden Aminosäure eines beliebigen anderen Parvovirus, und vorzugsweise an mindestens einer weiteren Insertionsstelle, weiter vorzugsweise an der Insertionsstelle direkt N- oder C-terminal zu einer Aminosäure in der Sequenz YKQIS₂₆₁ SQSGA (I-261), direkt N- oder C-terminal zu einer Aminosäure in der Sequenz EEKFF₅₃₄ PQSGV (I-534), direkt N- oder C-terminal zu einer Aminosäure in der Sequenz RTTNP₅₇₀ VATEQ (I-570), direkt N-oder C-terminal zu einer Aminosäure in der Sequenz NPVAT₅₇₃ EQYGS (I-573) oder direkt N- oder C-terminal zu einer Aminosäure in der Sequenz LQRGN₅₈₇ RQAAT (I-587), besonders an der Aminosäureinsertion direkt C-terminal zu Aminosäure G₄₅₃ in der Sequenz von AAV-2 oder der entsprechenden Aminosäure eines beliebigen anderen Parvovirus und direkt N- oder C-terminal zu einer Aminosäure in der Sequenz LQRGN₅₈₇ RQAAT (I-587), vorzugsweise von AAV1, AAV2 oder AAV-6, ein Tumorantigen, insbesondere ein HER2/neu-Epitop oder -Mimotop, besonders das Epitop QMWAPQWGPD oder eine funktionell aktive Variante hiervon, umfasst;
e) einer Autoimmunerkrankung und/oder einer chronischen inflammatorischen Erkrankung, vorzugsweise rheumatoider Arthritis und/oder Morbus Crohn, wobei das Strukturprotein eines Parvovirus an der Aminosäureinsertion direkt C-terminal zu Aminosäure G₄₅₃ in der Sequenz von AAV-2 oder der entsprechenden Aminosäure eines beliebigen anderen Parvovirus, und vorzugsweise an mindestens einer weiteren Insertionsstelle, weiter vorzugsweise an der Insertionsstelle direkt N- oder C-terminal zu einer Aminosäure in der Sequenz YKQIS₂₆₁ SQSGA (I-261), direkt N- oder C-terminal zu einer Aminosäure in der Sequenz EEKFF₅₃₄ PQSGV (I-534), direkt N- oder C-terminal zu einer Aminosäure in der Sequenz RTTNP₅₇₀ VATEQ (I-570), direkt N-oder C-terminal zu einer Aminosäure in der Sequenz NPVAT₅₇₃ EQYGS (I-573) oder direkt N- oder C-terminal zu einer Aminosäure in der Sequenz LQRGN₅₈₇ RQAAT (I-587), besonders an der Aminosäureinsertion direkt C-terminal zu Aminosäure G₄₅₃ in der Sequenz von AAV-2 oder der entsprechenden Aminosäure eines beliebigen anderen Parvovirus und direkt N- oder C-terminal zu einer Aminosäure in der Sequenz LQRGN₅₈₇ RQAAT (I-587), vorzugsweise von AAV1, AAV2 oder AAV-6, ein Epitop oder Mimotop eines Cytokins, vorzugsweise von TNF-α, IL-6 und/oder IL-17, besonders ein Epitop ausgewählt aus der Gruppe bestehend aus SSRTPSDKPVAHVVANPQAE,SRTPSDKPVAHVVANP,SSRTPSDKP, NADGNVDYHMNSVP, DGNVDYHMNSV, RSFKEFLQSSLRALRQ, FKEFLQSSLRA oder einer funktionell aktiven Variante hiervon, umfasst, oder
f) eine Infektionskrankheit, vorzugweise eine HIV-Infektion, wobei das Strukturprotein eines Parvovirus an der Aminosäureinsertion direkt C-terminal zu Aminosäure G₄₅₃ in der Sequenz von AAV-2 oder der entsprechenden Aminosäure eines beliebigen anderen Parvovirus, und vorzugsweise an mindestens einer weiteren Insertionsstelle, weiter vorzugsweise an der Insertionsstelle direkt N- oder C-terminal zu einer Aminosäure in der Sequenz YKQIS₂₆₁ SQSGA (I-261), direkt N- oder C-terminal zu einer Aminosäure in der Sequenz EEKFF₅₃₄ PQSGV (I-534), direkt N- oder C-terminal zu einer Aminosäure in der Sequenz RTTNP₅₇₀ VATEQ (I-570), direkt N- oder C-terminal zu einer Aminosäure in der Sequenz NPVAT₅₇₃ EQYGS (I-573) oder direkt N- oder C-terminal zu einer Aminosäure in der Sequenz LQRGN₅₈₇ RQAAT (I-587), besonders an der Aminosäureinsertion direkt C-terminal zu Aminosäure G₄₅₃ in der Sequenz von AAV-2 oder der entsprechenden Aminosäure eines beliebigen anderen Parvovirus und direkt N- oder C-terminal zu einer Aminosäure in der Sequenz LQRGN₅₈₇ RQAAT (I-587), vorzugsweise von AAV1, AAV2 oder AAV-6, ein Epitop oder Mimotop eines viralen Rezeptors, vorzugsweise von CCR5, besonders ein Epitop ausgewählt aus der Gruppe bestehend aus HYAAAQWDFGNTMCQL, YAAQWDFGNTMCQ, RSQKEGLHYT oder einer funktionell aktiven Variante hiervon, umfasst.

## Revendications

1. Structure multimérique comprenant une protéine structurale de parvovirus qui comprend une insertion d'acide aminé
dans laquelle l'insertion d'acide aminé est directement en position C-terminale de l'acide aminé G₄₅₃ dans la séquence de l'AAV-2 ou de l'acide aminé correspondant d'un quelconque autre parvovirus, et
dans laquelle la protéine structurale comportant l'insertion est capable de former une particule, et
dans laquelle l'insertion
(a) possède une longueur de 4 à 30 acides aminés,
et/ou
(b) est un épitope.

2. Structure multimérique selon la revendication 1,
a) dans laquelle l'insertion est localisée sur la surface de la capside formée par les protéines structurales ;
b) dans laquelle le parvovirus est un virus adéno-associé, de préférence sélectionné dans le groupe consistant en l'AAV-1, l'AAV-2, l'AAV-3b, l'AAV-4, l'AAV-5, l'AAV-6, l'AAV-7, l'AAV-8, l'AAV-9, l'AAV-10, l'AAV-11, l'AAV-12 et le b-AAV, particulièrement sélectionné dans le groupe consistant en l'AAV-1, l'AAV-2, l'AAV-5 et l'AAV-8 ;
c) dans laquelle le parvovirus est sélectionné dans le groupe consistant en le virus de la panleucopénie féline (FPV), le parvovirus canin (CPV), B19, le parvovirus de l'oie (GPV) et le virus minute de la souris (MVM), particulièrement le FPV, le CPV et B19 ;
d) dans laquelle l'insertion d'acide aminé possède une longueur de 5 à 20, de la manière la plus préférée de 5 à 15 acides aminés ; et/ou
e) dans laquelle l'insertion d'acide aminé est un épitope.

3. Structure multimérique selon la revendication 2,
a) dans laquelle l'épitope est un épitope de cellules B ;
b) dans laquelle l'insertion est un épitope dérivé d'un tolérogène ;
c) dans laquelle l'insertion est une partie d'une protéine sélectionnée dans le groupe consistant en un antigène tumoral, une protéine mal repliée, une protéine sérique, une protéine membranaire, un récepteur viral, un membre de la famille du TNF et une interleukine ; et/ou
d) dans laquelle l'épitope dérivé d'un tolérogène est dérivé d'une protéine du groupe consistant en la CETP, CD20, des récepteurs de l'acétylcholine, l'IL13R, l'EGFR, une IgE, Melan A, HMW MAA, CA125, Her2/NEU, CCR5, la molécule d'adhésion cellulaire L1, le VEGF, l'EGFR, CD20, le TNF-α, l'IL-6, l'IL-9, l'IL-13, l'IL-17, et la β-amyloïde, particulièrement dans laquelle l'épitope dérivé d'un tolérogène est sélectionné dans le groupe consistant en VNLTWSRASG, EFCINHRGYWVCGD, EDGQVMDVDLS, EKQRNGTLT, TYQCRVTHPHLPRALMR, RHSTTQPRKTKGSG, DSNPRGVSAYLSR, TITCLVVDLAPSK, KTKGSGFFVF, THPHLPRALMRS, GETYQCRVTHPHLPRALMRSTTK, LPRALMRS, INHRGYWV, CDAGSVRTNAPD AKAVSNLTESRSESLQS, SLTGDEFKKVLET, REAVAYRFEED, INPEIITLDG, DISVTGAPVITATYL, DISVTGAPVITA, PKTVSNLTESSSESVQS, SLMGDEFKAVLET, QHSVAYTFEED, INPEIITRDG, DISLTGDPVITASYL, DISLTGDPVITA, DQSIDFEIDSA, KNVSEDLPLPTFSPTLLGDS, KNVSEDLPLPT, CDSGRVRTDAPD, FPEHLLVDFLQSLS, DAEFRHDSG, HYAAAQWDFGNTMCQL, YAAQWDFGNTMCQ, RSQKEGLHYT, SSRTPSDKPVAHVVANPQAE, SRTPSDKPVAHVVANP, SSRTPSDKP, NADGNVDYHMNSVP, DGNVDYHMNSV, RSFKEFLQSSLRALRQ, FKEFLQSSLRA, et QMWAPQWGPD.

4. Structure multimérique selon la revendication 1 ou 2,
a) dans laquelle l'insertion d'acide aminé entraîne une augmentation de l'activité de transduction du parvovirus, particulièrement
i) dans laquelle l'insertion d'acide aminé médie la liaison de la protéine structurale à un récepteur membranaire cellulaire,
ii) dans laquelle l'insertion d'acide aminé est un ligand pour un récepteur donné, et/ou
iii) dans laquelle l'insertion d'acide aminé contient un motif RGD, particulièrement dans laquelle l'insertion d'acide aminé est ACDCRGDCFCA ;
b) dans laquelle l'insertion d'acide aminé entraîne une modification d'une propriété chromatographique de la protéine structurale, particulièrement dans laquelle l'insertion d'acide aminé est un marqueur utile pour une liaison à un ligand ; et/ou
c) dans laquelle l'insertion comporte un segment de liaison C-terminal, de préférence dans laquelle la séquence du segment de liaison est sélectionnée pour former un doigt de zinc (doigt Zn) tel que C₂H₂, C₄, et C₂HC, particulièrement dans laquelle le segment de liaison comprend au moins une Cys en position N-terminale et au moins une Cys en position C-terminale de l'insertion.

5. Structure multimérique selon la revendication 1 à 4, dans laquelle la protéine structurale de parvovirus comprend une ou plusieurs mutation(s) supplémentaire(s) au niveau d'un site différent du site directement en position C-terminale de l'acide aminé G₄₅₃ dans la séquence de l'AAV-2 ou de l'acide aminé correspondant d'un quelconque autre parvovirus, indépendamment sélectionnée(s) parmi une mutation ponctuelle, une délétion interne, une délétion N-terminale, une insertion et une substitution, de préférence une insertion supplémentaire.

6. Structure multimérique selon la revendication 5,
a) dans laquelle la mutation supplémentaire est une insertion définie par analogie avec la première insertion de la revendication 2a), 2d), 3a) à 3d) et/ou 4a), et/ou dans laquelle la protéine structurale comportant l'insertion supplémentaire est capable de former une particule et/ou dans laquelle l'insertion possède une longueur de 4 à environ 30 acides aminés et/ou
b) dans laquelle la mutation supplémentaire est une insertion d'un épitope de cellule B ou d'un épitope de cellule T cytotoxique.

7. Structure multimérique selon la revendication 5, dans laquelle la mutation supplémentaire réduit l'activité de transduction de la particule pour une cellule cible donnée d'au moins 50 %, de préférence d'au moins 80 %, particulièrement d'au moins 95 %, particulièrement dans laquelle la mutation supplémentaire est une mutation qui inactive le site de liaison HSPG localisé à proximité de la séquence LQRGN₅₈₇ RQAAT (site 1-587), en particulier dans laquelle la mutation supplémentaire est une délétion ou une substitution de R₅₈₅ et/ou R₅₈₈ de l'AAV-2 ou de l'acide aminé correspondant d'autres parvovirus, de préférence des substitutions d'acides aminés sélectionnées dans le groupe consistant en R₄₈₄A, R₄₈₇A, R₄₈₇G, K₅₃₂A, K₅₃₂A, K₅₃₂D, R₅₈₅A, R₅₈₅S, R₅₈₅Q, R₅₈₈A et R₅₈₈T, particulièrement R₅₈₅A et R₅₈₈A pour l'AAV-2, et K₅₃₁A et K₅₃₁E pour l'AAV-6.

8. Structure multimérique selon la revendication 5, dans laquelle la mutation supplémentaire réduit l'aptitude à induire une réponse de cellule B contre un épitope et/ou mimotope spécifique d'un parvovirus.

9. Banque de protéines structurales, dans laquelle la banque comprend un ensemble de protéines structurales de parvovirus différentes selon l'une quelconque des revendications 1 à 8, particulièrement dans laquelle la banque est couplée et/ou particulièrement dans laquelle la banque de la présente invention comporte une multiplicité de mutants de parvovirus de plus de 10³, de préférence de plus de 10⁵, de manière davantage préférée de plus de 10⁶, particulièrement de plus de 10⁷.

10. Structure multimérique selon l'une quelconque des revendications 1 à 8, dans laquelle la structure multimérique est un capsomère, une pseudo-particule virale ou un virus et/ou particulièrement dans laquelle la structure est un agrégat d'au moins environ 5, de préférence au moins environ 10, de manière davantage préférée d'au moins environ 30, de la manière la plus préférée entre toutes d'au moins environ 60 protéines structurales.

11. Acide nucléique codant pour une protéine structurale selon l'une quelconque des revendications 1 à 8.

12. Vecteur comprenant un acide nucléique selon la revendication 11.

13. Banque de vecteurs, dans laquelle la banque comprend un ensemble de vecteurs différents selon la revendication 12, particulièrement dans laquelle le génotype et le phénotype des particules de virion sont couplés.

14. Virus comprenant un acide nucléique selon la revendication 11 ou un vecteur selon la revendication 12.

15. Cellule isolée comprenant un acide nucléique selon la revendication 11 ou un vecteur selon la revendication 12.

16. Procédé pour modifier le tropisme d'un parvovirus, le procédé comprenant les étapes consistant à :
a) coexprimer des fonctions de vecteur et auxiliaire de parvovirus, où la fonction auxiliaire exprime une protéine structurale de parvovirus selon l'une quelconque des revendications 1 à 8 dans des conditions qui permettent la formation d'un parvovirus, et
b) isoler le parvovirus.

17. Procédé pour présenter un épitope sur la surface d'un parvovirus, le procédé comprenant les étapes consistant à :
a) exprimer l'acide nucléique selon la revendication 11 dans des conditions appropriées, et
b) isoler la protéine structurale exprimée de l'étape a).

18. Médicament comprenant au moins une protéine structurale de parvovirus selon l'une quelconque des revendications 1 à 8 et/ou un acide nucléique selon la revendication 11, de préférence au moins une structure multimérique selon la revendication 10.

19. Médicament selon la revendication 18 destiné à être utilisé en tant que vaccin ou en tant que vecteur de transfert de gènes.

20. Utilisation d'au moins une protéine structurale de parvovirus selon l'une quelconque des revendications 1 à 8 et/ou d'un acide nucléique selon la revendication 11, de préférence d'au moins une structure multimérique selon la revendication 10 pour la fabrication d'un vaccin ou d'un vecteur de transfert de gènes.

21. Médicament selon la revendication 18 ou 19 pour le traitement et/ou la prévention de
a) une maladie allergique et/ou l'asthme, dans lequel au niveau de l'insertion d'acide aminé directement en position C-terminale de l'acide aminé G₄₅₃ dans la séquence de l'AAV-2 ou de l'acide aminé correspondant d'un quelconque autre parvovirus, et de préférence au niveau d'au moins un site d'insertion supplémentaire, de manière davantage préférée au niveau d'un site d'insertion directement en position N- ou C-terminale d'un acide aminé dans la séquence YKQIS₂₆₁ SQSGA (I-261), directement en position N- ou C-terminale d'un acide aminé dans la séquence EEKFF₅₃₄ PQSGV (I-534), directement en position N- ou C-terminale d'un acide aminé dans la séquence RTTNP₅₇₀ VATEQ (1-570), directement en position N- ou C-terminale d'un acide aminé dans la séquence NPVAT₅₇₃ EQYGS (I-573) ou directement en position N- ou C-terminale d'un acide aminé dans la séquence LQRGN₅₈₇ RQAAT (1-587), particulièrement au niveau de l'insertion d'acide aminé directement en position C-terminale de l'acide aminé G₄₅₃ dans la séquence de l'AAV-2 ou de l'acide aminé correspondant d'un quelconque autre parvovirus et directement en position N- ou C-terminale d'un acide aminé dans la séquence LQRGN₅₈₇ RQAAT (1-587), de préférence de l'AAV-1, l'AAV-2 ou l'AAV-6, la protéine structurale d'un parvovirus comprend un épitope/mimotope anti-idiotypique d'un anticorps anti-IgE, et/ou un épitope/mimotope d'IgE, particulièrement un mimotope ayant la séquence EFCINHRGYWVCGD, le premier G, W et V étant conservés et les résidus cystéine C permettant de médier une forme circulaire du peptide via des ponts disulfure, VNLTWSRASG ou INHRGYWV, ou particulièrement un épitope sélectionné dans le groupe consistant en EKQRNGTLT, EDGQVMDVDLS, TYQCRVTHPHLPRALMR, RHSTTQPRKTKGSG, DSNPRGVSAYLSR, TITCLVVDLAPSK, KTKGSGFFVF, THPHLPRALMRS, GETYQCRVTHPHLPRALMRSTTK, LPRALMRS et un variant fonctionnellement actif de celui-ci ;
b) la maladie d'Alzheimer, dans lequel au niveau de l'insertion d'acide aminé directement en position C-terminale de l'acide aminé G₄₅₃ dans la séquence de l'AAV-2 ou de l'acide aminé correspondant d'un quelconque autre parvovirus, et de préférence au niveau d'au moins un site d'insertion supplémentaire, de manière davantage préférée au niveau d'un site d'insertion directement en position N- ou C-terminale d'un acide aminé dans la séquence YKQIS₂₆₁ SQSGA (1-261), directement en position N- ou C-terminale d'un acide aminé dans la séquence EEKFF₅₃₄ PQSGV (I-534), directement en position N- ou C-terminale d'un acide aminé dans la séquence RTTNP₅₇₀ VATEQ (1-570), directement en position N- ou C-terminale d'un acide aminé dans la séquence NPVAT₅₇₃ EQYGS (I-573) ou directement en position N- ou C-terminale d'un acide aminé dans la séquence LQRGN₅₈₇ RQAAT (1-587), particulièrement au niveau de l'insertion d'acide aminé directement en position C-terminale de l'acide aminé G₄₅₃ dans la séquence de l'AAV-2 ou de l'acide aminé correspondant d'un quelconque autre parvovirus et directement en position N- ou C-terminale d'un acide aminé dans la séquence LQRGN₅₈₇ RQAAT (1-587), de préférence de l'AAV-1, l'AAV-2 ou l'AAV-6, la protéine structurale d'un parvovirus comprend un épitope ou mimotope de la β-amyloïde, particulièrement comprenant ou possédant la séquence de DAEFRHDSG ou un variant fonctionnellement actif de celui-ci ;
c) l'athérosclérose, dans lequel au niveau de l'insertion d'acide aminé directement en position C-terminale de l'acide aminé G₄₅₃ dans la séquence de l'AAV-2 ou de l'acide aminé correspondant d'un quelconque autre parvovirus, et de préférence au niveau d'au moins un site d'insertion supplémentaire, de manière davantage préférée au niveau d'un site d'insertion directement en position N- ou C-terminale d'un acide aminé dans la séquence YKQIS₂₆₁ SQSGA (1-261), directement en position N- ou C-terminale d'un acide aminé dans la séquence EEKFF₅₃₄ PQSGV (I-534), directement en position N- ou C-terminale d'un acide aminé dans la séquence RTTNP₅₇₀ VATEQ (1-570), directement en position N- ou C-terminale d'un acide aminé dans la séquence NPVAT₅₇₃ EQYGS (I-573) ou directement en position N- ou C-terminale d'un acide aminé dans la séquence LQRGN₅₈₇ RQAAT (1-587), particulièrement au niveau de l'insertion d'acide aminé directement en position C-terminale de l'acide aminé G₄₅₃ dans la séquence de l'AAV-2 ou de l'acide aminé correspondant d'un quelconque autre parvovirus et directement en position N- ou C-terminale d'un acide aminé dans la séquence LQRGN₅₈₇ RQAAT (I-587), particulièrement 1-453 et 1-587, de préférence de l'AAV-1, l'AAV-2 ou l'AAV-6, la protéine structurale d'un parvovirus comprend un épitope ou mimotope de la CETP, particulièrement un épitope sélectionné dans le groupe consistant en PKTVSNLTESSSESVQS, SLMGDEFKAVLET, QHSVAYTFEED, INPEIITRDG, DISLTGDPVITASYL, DISLTGDPVITA, DQSIDFEIDSA, KNVSEDLPLPTFSPTLLGDS, KNVSEDLPLPT, CDSGRVRTDAPD, FPEHLLVDFLQSLS et un variant fonctionnellement actif de celui-ci ;
d) une maladie tumorale, dans lequel au niveau de l'insertion d'acide aminé directement en position C-terminale de l'acide aminé G₄₅₃ dans la séquence de l'AAV-2 ou de l'acide aminé correspondant d'un quelconque autre parvovirus, et de préférence au niveau d'au moins un site d'insertion supplémentaire, de manière davantage préférée au niveau d'un site d'insertion directement en position N- ou C-terminale d'un acide aminé dans la séquence YKQIS₂₆₁ SQSGA (1-261), directement en position N- ou C-terminale d'un acide aminé dans la séquence EEKFF₅₃₄ PQSGV (I-534), directement en position N- ou C-terminale d'un acide aminé dans la séquence RTTNP₅₇₀ VATEQ (1-570), directement en position N- ou C-terminale d'un acide aminé dans la séquence NPVAT₅₇₃ EQYGS (I-573) ou directement en position N- ou C-terminale d'un acide aminé dans la séquence LQRGN₅₈₇ RQAAT (1-587), particulièrement au niveau de l'insertion d'acide aminé directement en position C-terminale de l'acide aminé G₄₅₃ dans la séquence de l'AAV-2 ou de l'acide aminé correspondant d'un quelconque autre parvovirus et directement en position N- ou C-terminale d'un acide aminé dans la séquence LQRGN₅₈₇ RQAAT (1-587), de préférence de l'AAV-1, l'AAV-2 ou l'AAV-6, la protéine structurale d'un parvovirus comprend un antigène tumoral, particulièrement un épitope ou mimotope de HER2/neu, particulièrement l'épitope QMWAPQWGPD ou un variant fonctionnellement actif de celui-ci ;
e) une maladie auto-immune et/ou une maladie inflammatoire chronique, de préférence la polyarthrite rhumatoïde et/ou la maladie de Crohn, dans lequel au niveau de l'insertion d'acide aminé directement en position C-terminale de l'acide aminé G₄₅₃ dans la séquence de l'AAV-2 ou de l'acide aminé correspondant d'un quelconque autre parvovirus, et de préférence au niveau d'au moins un site d'insertion supplémentaire, de manière davantage préférée au niveau d'un site d'insertion directement en position N- ou C-terminale d'un acide aminé dans la séquence YKQIS₂₆₁ SQSGA (1-261), directement en position N- ou C-terminale d'un acide aminé dans la séquence EEKFF₅₃₄ PQSGV (1-534), directement en position N- ou C-terminale d'un acide aminé dans la séquence RTTNP₅₇₀ VATEQ (1-570), directement en position N- ou C-terminale d'un acide aminé dans la séquence NPVAT₅₇₃ EQYGS (1-573) ou directement en position N- ou C-terminale d'un acide aminé dans la séquence LQRGN₅₈₇ RQAAT (I-587), particulièrement au niveau de l'insertion d'acide aminé directement en position C-terminale de l'acide aminé G₄₅₃ dans la séquence de l'AAV-2 ou de l'acide aminé correspondant d'un quelconque autre parvovirus et directement en position N- ou C-terminale d'un acide aminé dans la séquence LQRGN₅₈₇ RQAAT (1-587), de préférence de l'AAV-1, l'AAV-2 ou l'AAV-6, la protéine structurale d'un parvovirus comprend un épitope ou mimotope d'une cytokine, de préférence du TNF-α, l'IL-6 et/ou l'IL-17, particulièrement un épitope sélectionné dans le groupe consistant en SSRTPSDKPVAHVVANPQAE, SRTPSDKPVAHVVANP, SSRTPSDKP, NADGNVDYHMNSVP, DGNVDYHMNSV, RSFKEFLQSSLRALRQ, FKEFLQSSLRA ou un variant fonctionnellement actif de celui-ci, ou
f) une maladie infectieuse, de préférence une infection par le VIH, dans lequel au niveau de l'insertion d'acide aminé directement en position C-terminale de l'acide aminé G₄₅₃ dans la séquence de l'AAV-2 ou de l'acide aminé correspondant d'un quelconque autre parvovirus, et de préférence au niveau d'au moins un site d'insertion supplémentaire, de manière davantage préférée au niveau d'un site d'insertion directement en position N- ou C-terminale d'un acide aminé dans la séquence YKQIS₂₆₁ SQSGA (1-261), directement en position N- ou C-terminale d'un acide aminé dans la séquence EEKFF₅₃₄ PQSGV (I-534), directement en position N- ou C-terminale d'un acide aminé dans la séquence RTTNP₅₇₀ VATEQ (1-570), directement en position N- ou C-terminale d'un acide aminé dans la séquence NPVAT₅₇₃ EQYGS (1-573) ou directement en position N- ou C-terminale d'un acide aminé dans la séquence LQRGN₅₈₇ RQAAT (I-587), particulièrement au niveau de l'insertion d'acide aminé directement en position C-terminale de l'acide aminé G₄₅₃ dans la séquence de l'AAV-2 ou de l'acide aminé correspondant d'un quelconque autre parvovirus et directement en position N- ou C-terminale d'un acide aminé dans la séquence LQRGN₅₈₇ RQAAT (1-587), de préférence de l'AAV-1, l'AAV-2 ou l'AAV-6, la protéine structurale d'un parvovirus comprend un épitope ou mimotope d'un récepteur viral, de préférence de CCR5, particulièrement un épitope sélectionné dans le groupe consistant en HYAAAQWDFGNTMCQL, YAAQWDFGNTMCQ, RSQKEGLHYT ou un variant fonctionnellement actif de celui-ci.
